(19) Europäisches Patentamt
European Patent Office
Office européen des brevets

(11) EP 3 899 535 B1

(12) EUROPEAN PATENT SPECIFICATION

(45) Date of publication and mention of the grant of the patent:
**19.06.2024 Bulletin 2024/25**

(21) Application number: **19832117.6**

(22) Date of filing: **20.12.2019**

(51) International Patent Classification (IPC):
***G01N 33/50*** *(2006.01)* ***C12Q 1/6897*** *(2018.01)*

(52) Cooperative Patent Classification (CPC):
(C-Sets available)
**G01N 33/5008; C12Q 1/6897;** G01N 2500/04; G01N 2500/10; G01N 2500/20 (Cont.)

(86) International application number:
**PCT/EP2019/086750**

(87) International publication number:
**WO 2020/128015 (25.06.2020 Gazette 2020/26)**

(54) **SCREENING ASSAY**

SCREENING-ASSAY

ESSAI DE CRIBLAGE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.12.2018 GB 201820863**

(43) Date of publication of application:
**27.10.2021 Bulletin 2021/43**

(73) Proprietor: **The University of Bath**
**Bath Somerset BA2 7AY (GB)**

(72) Inventors:
- **MASON, Jody Michael**
  **Bath Somerset BA2 7AY (GB)**
- **KAD, Neil Mark**
  **Canterbury Kent CT2 7NH (GB)**

(74) Representative: **Mewburn Ellis LLP**
**Aurora Building**
**Counterslip**
**Bristol BS1 6BX (GB)**

(56) References cited:
**WO-A1-91/16456** **WO-A1-99/13069**
**US-A1- 2007 141 672**

- **J. M Mason ET AL: "Peptides tailored to interfere with protein interaction and function", , 1 January 2009 (2009-01-01), XP055678865, Retrieved from the Internet: URL:http://www.syntbio.net/ka/pdf-files/Mason%20Arndt%20Chemistry%20Today%202009%202 7%2047.pdf [retrieved on 2020-03-23]**
- **Stanley Sabrina Y R: "An Investigation of Bacteriophage Anti-CRISPR and Anti-CRISPR Associated Proteins", Thesis University of Toronto, 1 November 2018 (2018-11-01), pages 1-120, XP055800251, Retrieved from the Internet: URL:https://tspace.library.utoronto.ca/handle/1807/97883 [retrieved on 2021-04-30]**
- **Pavco P A ET AL: "Elongation by Escherichia coli RNA polymerase is blocked in vitro by a site-specific DNA binding protein.", Journal of Biological Chemistry, vol. 265, no. 17, 1 June 1990 (1990-06-01) , pages 9960-9969, XP93090314, US ISSN: 0021-9258, DOI: 10.1016/S0021-9258(19)38764-2**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **MERULLA DAVIDE ET AL: "Regulatable and Modulable Background Expression Control in Prokaryotic Synthetic Circuits by Auxiliary Repressor Binding Sites", ACS SYNTHETIC BIOLOGY, [Online] vol. 5, no. 1, 14 September 2015 (2015-09-14), pages 36-45, XP093090326, Washington DC ,USA ISSN: 2161-5063, DOI: 10.1021/acssynbio.5b00111 Retrieved from the Internet: URL:http://pubs.acs.org/doi/pdf/10.1021/acssynbio.5b00111> [retrieved on 2023-10-10]**
- **QI LEI S ET AL: "Repurposing CRISPR as an RNA-Guided Platform for Sequence-Specific Control of Gene Expression", CELL, ELSEVIER, AMSTERDAM NL, vol. 152, no. 5, 28 February 2013 (2013-02-28), pages 1173-1183, XP028987304, ISSN: 0092-8674, DOI: 10.1016/J.CELL.2013.02.022**
- **BRENNAN ANDREW ET AL: "An Approach to Derive Functional Peptide Inhibitors of Transcription Factor Activity", JACS AU, [Online] vol. 2, no. 4, 6 April 2022 (2022-04-06), pages 996-1006, XP093090334, ISSN: 2691-3704, DOI: 10.1021/jacsau.2c00105 Retrieved from the Internet: URL:https://pubs.acs.org/doi/pdf/10.1021/jacsau.2c00105> [retrieved on 2023-10-10]**


(52) Cooperative Patent Classification (CPC): (Cont.)

C-Sets
**C12Q 1/6897, C12Q 2522/10, C12Q 2525/143**

## Description

### *Field of the Invention*

The present invention relates to a method for screening for an antagonist of a DNA-binding protein, such as a transcription factor, as defined in the claims. The methods of the invention find application, for example, in the identification of antagonists that may be useful in the treatment of cancers.

### *Background*

Transcription factors are commonly deregulated in human cancers and targeting them can be highly effective in treating particular malignancies e.g. nuclear hormone receptors (Soucek et al., 2013). The diversity and potency of transcription factors as drivers of cell transformation justifies a continued pursuit as therapeutic targets for drug discovery.

An example of a transcription factor that is dysregulated in numerous human cancers is Activator Protein-1 (AP-1). AP-1 consists of a heterodimer made up of a Fos family member and a Jun family member and binds to variations of the consensus DNA binding site known as the TPA Response Element (TRE): TGA[G/C]TCA (Ozanne et al. 2007). The Fos family contains four proteins (c-Fos, FosB, Fra1 and Fra2), while the Jun family contains three proteins (c-Jun, JunB, and JunD). AP-1 is a member of the basic leucine zipper (bZIP) family of transcriptional regulators. Dimerisation, typically by members of the Jun and Fos families, is driven by the leucine zipper / coiled coil motif and is required for functional activity of the protein. Key residues within the basic region are then able to interact with specific bases within the TRE and is therefore able to regulate the expression of a number of genes involved in differentiation, proliferation, and apoptosis. Increased AP-1 levels lead to increased transactivation of target gene expression leading to growth and malignant transformation in many cell types.

Transcription factors such as AP-1 have typically remained very challenging drug targets using small-molecule led approaches due to the lack of tractable pockets and targeting protein-protein interactions. Teams have typically tried to target transcription factors indirectly e.g. promoting targeting them for proteolysis or inhibiting up stream signalling factors (McCormick, 2016).

Another approach to target transcription factors is to identity small peptide antagonists. These peptides are larger than a typical small molecule and have a larger surface area capable of forming many more points of contact with high specificity to better disrupt protein-protein interactions (PPIs). Many rational design approaches, randomised screening approaches, and selection systems result in the successful identification of compounds capable of binding to given protein targets.

An example of a method that aims to identify inhibitors that disrupt PPIs is described in Miranda et al. (2011). This method makes use of a bacterial reverse two-hybrid system that enables the identification of interacting protein partners. Compounds that disrupt the interaction between two targeted proteins, expressed as hybrid fusions of a chimeric repressor complex (434 and P22), prevent expression of reporter genes. In this way, inhibitors that disrupt interaction of the two targeted proteins are reported to be identifiable.

Another example of a method that looks to identify inhibitors of PPIs is described in Mason et al. 2006. This method identified potential inhibitors of c-Jun using a Protein-fragment Complementation Assay (PCA) involving the cFos sequence as a design scaffold and with semi-randomisation of key residues involved in heterodimerisation with cJun. Using this method, the PPI inhibitor FosW was identified. FosW is a 37 residue peptide based on the cFos leucine zipper domain that was shown to bind to c-Jun with a high affinity (Mason et al. 2006). Use of the PCA approach to identify PPIs is also described in Pelletier et al. (1998).

However, what is much more difficult to ensure is that binding to said target will result in ablating target protein function. Methods that identify inhibitors that disrupt PPIs, such as that described in Miranda et al. (2011) and Mason et al. (2006) are designed to identifier binders of a target. They do not reveal whether the inhibitor identified is also able to ablate protein function. In other words, they do not reveal whether the inhibitor is a functionally active antagonist of the protein. Until the inhibitor is tested at the protein level, there is no way of knowing if the inhibitors identified will translate into functional antagonists of protein function. There are many instances where formation of a PPI has not ensured loss of function. This is also even more relevant for targeting transcription factors bound to DNA as these complexes are typically very stable. For example, Olive et al. (1997) describe the generation of an inhibitor construct termed 4H-Fos that was able to inhibit binding of the protein Fos to Jun, but a subsequent gel shift experiment showed that this construct was unable to inhibit the DNA-binding activity of the protein. This demonstrates that potential inhibitors identified using these methods that screen for PPI inhibitors do not necessarily translate into functional antagonists.

Various prior art documents describe methods for analysing PPIs and/or the activity of specific DNA-binding proteins and involve the use of a reporter gene operably linked to a promoter, where the promoter contains a binding site for the DNA-binding protein. For example:
WO 2018/075486 A1 discloses the use of a fusion protein comprising a ligand-binding protein and a DNA-binding protein

in a method for sensing a ligand in a cell or a reaction mixture, where the DNA-binding protein is able to bind to a promoter operably linked to a reporter gene.

FR 2861742 discloses methods that select for compounds that bind to the EthR mycobacterial repressor and prevent EthR from repressing expression of the *ethA* gene. This document discloses a method involving a construct where the promoter and operator region of the *ethA* gene are cloned upstream of a reporter gene.

WO 99/13069 A1 discloses a system for assaying PPIs using "bait" and "prey" fusion proteins. The bait protein includes a DNA-binding domain and the prey protein includes a transcriptional repression domain and the system is described as assaying for compounds that interfere with the interaction of the bait and prey fusion proteins. This document discloses the use of reporter genes having promoters with binding sites for the DNA-binding domain of the bait fusion protein.

WO 01/59450 A2 describes a method of screening a compound for its effect on a cellular process by providing a first polynucleotide encoding a zinc finger protein operably linked to a transcriptional control element and a second polynucleotide encoding a reporter. The reporter can be operably linked to transcriptional control elements that are modulated by the zinc finger protein.

WO 99/57535 A2 discloses recombinant cell lines and screening methods useful for identifying agents that induce apoptosis in target cells. These cell lines and methods may comprise plasmids expressing promoters operably linked to reporter genes.

WO 2008/110784 A1 discloses a method that involves determining whether a test agent modulates at least one activity or function of *M. tuberculosis* Rv3574, a member of the TetR family of transcriptional repressors. This method may involve measuring expression of a reporter gene operably linked to a DNA sequence that is bound to by Rv3574.

Stanley Sabrina Y R: "An Investigation of Bacteriophage Anti-CRISPR and Anti-CRISPR Associated Proteins", Thesis University of Toronto, 1 November 2018 (2018-11-01), discloses a transcriptional reporter assay in which a DNA binding protein (Cascade) is targeted to a reporter expression cassette (*phzM* gene) in the presence of an anti-CRISPR of interest. Anti-CRISPR binds to the Cascade complex, prevents DNA binding such that RNA polymerase access to the phzM promoter is unimpeded and cultures appear green in colour.

Thus, there remains a need for methods that are reliably able to identify functionally active antagonists of transcription factors, in particular those transcription factors that are known to be dysregulated in human cancers.

The present invention has been devised in light of the above considerations.

### *Disclosure of the Invention*

The present inventors have recognised that there is a need for a screening method that is capable of identifying functionally active antagonists of DNA-binding proteins, such as transcription factors. Functionally active antagonists are compounds that are able to both bind to the DNA-binding protein and antagonise its DNA-binding activity. As described in the background section above, transcription factors are commonly deregulated in human cancers and therefore the use of this screening method to identify functionally active antagonists is expected to be useful in identifying inhibitors that may have therapeutic use in the treatment of diseases such as cancer.

The screening method of the present invention makes use of a reporter expression cassette that encodes a reporter expression product, such as a protein that provides a phenotypic readout (also termed a "reporter protein"). The present inventors recognised that by engineering the reporter expression cassette to contain a binding site for a DNA-binding protein, it is possible to make use of the interaction between the DNA-binding protein and the binding site to inhibit expression of the reporter expression product. Without wishing to be bound by theory, it is believed that binding of the DNA-binding protein to the binding site inhibits transcription of the reporter expression cassette, thereby inhibiting expression of the reporter expression product. Inhibition of reporter expression product expression is also believed to be entirely independent on whether the DNA-binding protein is classed as a transcriptional activator or a transcriptional repressor (e.g. whether it normally functions to activate or repress transcription when it binds to promoter or enhancer sequences containing its binding sites in the genome of a cell).

The present inventors recognised that these components could be used in a method to identify 'functionally active' antagonists of the DNA-binding protein. If a test compound is able to both bind and inhibit DNA-binding activity of the DNA-binding protein then it will reduce the ability of the DNA-binding protein to inhibit expression of the reporter expression product. Thus, a method was devised where the DNA-binding protein, test compound and reporter expression cassette were all present. If the test compound is a functionally active antagonist, there will be an increased expression of reporter expression product in the presence of the test compound relative to the expression of the expression product in the absence of the test compound. An increase in expression in the presence of the test compound can be determined by comparing (i) expression of the reporter expression product in situations where the DNA-binding protein and the reporter expression cassette are present without the test compound, against (ii) expression of the reporter expression product in situations where the cell comprises the DNA-binding protein, the expression cassette and the test compound. If expression of the reporter expression product is observed to be greater in (ii) than in (i), then expression of the reporter

expression product is increased in the presence of the test compound. In this way, it can be determined whether a given test compound is a functionally active antagonist of the DNA-binding protein.

Thus, in one aspect the present invention provides a method for screening for an antagonist of a DNA-binding protein, the method comprising:

i) providing a cell, wherein the cell comprises a test compound, a DNA-binding protein, and a reporter expression cassette that encodes a reporter expression product,
wherein the reporter expression cassette comprises at least one binding site for the DNA-binding protein such that binding of the DNA-binding protein to the binding site inhibits expression of the reporter expression product; and

ii) determining expression of the reporter expression product in the presence of the test compound;

wherein an increase in expression of the reporter expression product in the presence of the test compound indicates that the test compound is capable of inhibiting DNA-binding activity of the DNA-binding protein, and

wherein some or all of the binding site(s) are located in the transcribed sequence of the reporter expression cassette.

Importantly, the present inventors have demonstrated that this screening method can distinguish between 1) test compounds that are able to bind the DNA-binding protein but do not disrupt DNA-binding activity of the DNA-binding protein, and 2) those inhibitors that are able to both bind and disrupt DNA-binding activity of the DNA-binding protein. Only those in the second category are functionally active antagonists. As described herein, only those test compounds that are able to both bind and dissociate the DNA-bound complex, or both bind and prevent DNA-binding of the DNA-binding protein, result in the increase in expression of the reporter expression product of the current method. For the avoidance of doubt, reference herein to a test compound that is capable of inhibiting DNA-binding activity of the DNA-binding protein (a "functionally active antagonist") is intended to encompass both inhibitors that bind and disassociate the DNA-bound complex as well as inhibitors that bind and prevent DNA-binding of the DNA-binding protein.

Thus, advantageously, the screening method allows the identification of functionally active antagonists of DNA-binding proteins. It is expected that such functionally active antagonists are more likely to represent compounds that will be useful for inhibiting the DNA-binding protein in a therapeutic setting, e.g. in the treatment of cancer, than those test compounds that bind but do not disrupt function of the DNA-binding protein. The ability to screen for and only take forward those test compounds that inhibit DNA-binding activity has the potential to save considerable investment of time and money.

Furthermore, the method indicates that the antagonist identified is specific to the target DNA-binding protein, as only dissociation of the target DNA-binding protein is expected to result in the increased expression. For example, test compounds that inhibit other DNA-binding proteins that are generally involved in expression of the reporter expression product (e.g. components of the initiation complex) will not result in increased expression and therefore will not be identified by the present screening method.

A further advantage of the method is that the readout is an increase in expression. Having an increase in expression as a readout is advantageous, as it is less likely to result in false positives that may occur when the readout is a decrease in expression. The screening method described herein therefore produces results with a high degree of confidence, reducing the likelihood of needing additional screening to confirm the result.

In another aspect, the present invention provides a cell-free method for screening for an antagonist of a DNA-binding protein, the method comprising:

i) contacting a test compound with a DNA-binding protein and a reporter expression cassette that encodes a reporter expression product,
wherein the reporter expression cassette comprises at least one binding site for the DNA-binding protein such that binding of the DNA-binding protein to the binding site inhibits expression of the reporter expression product; and
ii) determining expression of the reporter expression product;

wherein an increase in expression of the reporter expression product in the presence of the test compound indicates that the test compound is capable of inhibiting DNA-binding activity of the DNA-binding protein, and
wherein the method is carried out outside a cell in an *in vitro* system that comprises the components required for expression of the reporter expression product, and
wherein some or all of the binding site(s) are located in the transcribed sequence of the reporter expression cassette.

Another aspect of the present disclosure relates to antagonists identified by the methods for screening of the present invention.

Some particular aspects of the invention will now be discussed in more detail.

*Reporter expression product*

The reporter expression product used herein can be a peptidic compound or an RNA molecule (such as microRNA, siRNA, or a ribozyme). Methods of measuring expression of protein are well known in the art and include western blot, immunohistochemistry, luciferase gene reporter assays, colorimetric assays such as the BCA assay or Bradford assay, UV spectroscopy, as well as methods that involve observing the phenotypic readout of the protein, as described in more detail below. Methods of measuring the expression of an RNA molecule are also well known in the art and include quantitative PCR (qPCR), transcriptomic analyses, UV spectroscopy and microfluidic analysis. Preferably, the expression product is a protein.

In preferred embodiments, the reporter expression product is a protein that provides a phenotypic readout (also termed a "reporter protein"). A reporter protein that provides a phenotypic readout advantageously allows for a simple and rapid screening of test compounds.

Examples of reporter proteins include cell survival proteins, cell reproduction proteins, fluorescence proteins, bioluminescence proteins, enzymes that act on a substrate to produce a colorimetric signal, protein kinases, proteases, transcription factors, and regulatory proteins such as ubiquitin. The use of suitable reporter proteins in assays for determining PPIs is described, for example, in Wehr and Rossner (2016).

In some embodiments, the reporter protein is a cell survival protein or a cell reproduction protein. A cell survival protein is a protein that is essential for cell survival, such that survival is dependent upon the presence or activity of the cell survival protein. A cell reproduction protein is a protein that is essential for reproduction of the cell, such that cell proliferation (division) is dependent upon the activity of the cell reproduction protein. The essentiality of the cell survival or cell reproduction protein may depend on certain conditions, e.g. the presence of certain factors, such as a cytotoxic compound, in the cell medium.

If the reporter protein is a cell survival protein, then inhibition of expression of the cell survival protein will result in cell death. Thus, in methods described herein where the reporter protein is a cell survival protein, binding of the DNA-binding protein to the binding site in the absence of a test compound will result in cell death. Cell death can be determined by one of a number of techniques known to the person skilled in the art, e.g. the observing of morphological changes such as cytoplasmic blebbing, cell shrinkage, internucleosomal fragmentation and chromatin condensation. DNA cleavage typical of the apoptotic process may be demonstrated using TUNEL and DNA ladder assays. In these situations, when a test compound is added that is a functionally active antagonist of the DNA-binding protein, this will result in cell survival and therefore such a method uses cell survival as an indicator that the test compound is a functionally active antagonist. Use of a cell survival protein as a reporter protein can be advantageous as it gives a simple binary readout, i.e. the cell is either dead or alive.

If the reporter protein is a cell reproduction protein, then inhibition of expression of the cell reproduction protein will result in the cell being unable to proliferate and therefore unable to form progeny. Thus, in methods described herein where the reporter protein is a cell reproduction protein, binding of the DNA-binding protein to the binding site in the absence of a test compound will inhibit cell proliferation. Cell proliferation can be determined by one of a number of techniques known to the person skilled in the art, e.g. by counting of individual cells, foci or colonies, measuring metabolic activity using dyes such as MTT and WST-1, using nucleoside analogues such as bromodeoxyuridine (BrdU)and measuring incorporation of this analogue in the cells, staining dividing cells using reagents such as succinimidyl ester of carboxyfluorescein diacetate, and detecting proliferation markers such as PCNA, poisomerase IIB or phosphohistone H3. Inhibition of cell proliferation may also result in cell death, which can be measured as described above. In these situations, when a test compound is added that is a functionally active antagonist of the DNA-binding protein, this will restore cell proliferation and therefore such a method uses cell proliferation as an indicator that the test compound is a functionally active antagonist.

Examples of cell survival proteins include enzymes that are involved in synthesising compounds that are required for cell survival and proteins that are capable of inhibiting action of a toxic agent, such as an antibiotic. Examples of cell reproduction proteins include enzymes that are required for cell reproduction.

Examples of enzymes that are involved in synthesising compounds required for cell survival or reproduction are set out in **Table 1.** Thus, in some embodiments, the cell survival protein or cell reproduction protein is an enzyme selected from the first column **Table 1.**

**Table 1 - Example enzymes involved in synthesising compounds required for cell survival or reproduction**

| Enzyme | Compounds / conditions able to inhibit enzyme function |
|---|---|
| Dihydrofolate reductase (DHFR) | methotrexate or trimethoprim, cultured without nucleosides |
| Thymidine kinase | ganciclovir, hypoxanthine/aminopterin/thymidine (HAT) |
| thymidylate synthase | 2 fluorodeoxyuridine |
| Xanthine-guanine phosphoribosyl | mycophenolic acid with limiting xanthine |
| Asparagine synthetase | B-aspartyl hydroxamate or albizin puromycin |
| Cytosine methyltransferase | 5-Azacytidine (5-aza-CR) and 5-aza-2'-deoxycytidine |
| O6-alkylguanine alkyltransferase | N-methyl-N-nitro-sourea |
| Glycinamide ribonucleotide transformylase | dideazatetrahydrofolate, cultured without purine |
| Glycinamide ribonucleotide synthetase | cultured without purine |
| Phosphoribosyl-aminoimidazole synthetase | cultured without purine |
| Formylglycinamide ribotide amidotransferase | L-azaserine, 6-diazo-5-oxo-L-nor-leucine, cultured without purine |
| Phosphoribosyl-aminoimidazole carboxylase | cultured without purine |
| Phosphoribosyl-aminoimidazole carboxamide formyltransferase | cultured without purine |
| Fatty acid synthase | cerulenin |
| IMP dehydrogenase | mycophenolic acid |
| histidinol dehydrogenase | cultured without histidine |

For example, dihydrofolate reductase (DHFR) catalyses the reduction of dihydrofolate to tetrahydrofolate, for use in transfer of one-carbon units required for biosynthesis of serine, methionine, purines, pantothenate and thymidylate. In the absence of DHFR function, *de novo* synthesis of nucleoside precursors (hypoxanthine and thymidine) is inhibited. Thus, if cells are grown in the absence of a functioning DHFR and in the absence of nucleosides (e.g. all nucleosides, or at least the purine nucleosides), the cells will die. Reconstitution of enzyme activity can be monitored *in vivo* by cell survival in DHFR-negative cells grown in the absence of nucleosides.

Examples of proteins that are capable of inhibiting action of a toxic agent include enzymes that are capable of metabolising a toxic agent, e.g. to a less toxic agent, and antibiotic resistance proteins, e.g. proteins that bind and inhibit antibiotics. Examples of these are set out in **Table 2.** Thus, in some embodiments, the cell survival protein is a protein selected from the first column in **Table 2.**

**Table 2 - Examples of proteins that are capable of inhibiting action of a toxic agent**

| Cell survival protein | Toxic agent / antibiotic |
|---|---|
| beta-lactamase | β-lactam antibiotics such as penicillins, cephalosporins, cephamycins |
| chloramphenicol acetyl transferase | chloramphenicol |
| Puromycin N-acetyltransferase | puromycin |
| Aminoglycoside phosphotransferase | neomycin, G418, gentamycin |
| Hygromycin B phosphotransferase | hygromycin B |
| Blebomycin binding protein | Blebomycin |
| Adenosine deaminase | Xyl-A or adenosine, alanosine, and 2'-deoxycoformycin |

For example, Hygromycin-B is an aminocyclitol that inhibits protein synthesis by disrupting translocation and promoting misreading. The *E.coli* enzyme hygromycin-B-phosphotransferase detoxifies the cells by phosphorylating

hygromycin-B. When expressed in mammalian cells, hygromycin-B-phosphotransferase can confer resistance to hygromycin-B (Gritz and Davies, 1983).

As a further example, adenosine deaminase (ADA) catalyses the irreversible conversion of cytotoxic adenine nucleosides to their respective conversion of cytotoxic adenine nucleosides to their respective nontoxic inosine analogues. ADA only becomes a cell survival protein when cytotoxic concentrations of adenosine are added. By adding cytotoxic concentrations of adenosine or cytotoxic adenosine analogues such as 9-b-D-xylofuranosyladenine to the cells, ADA is required for cell growth to detoxify the cytotoxic agent. An exemplary method that uses ADA as a reporter protein is described in Kaufman et al. 1986.

Bleomycin, a member of the leomycin/phyleomycin family of antibiotics, is toxic to bacteria, fungi, plants, and mammalian cells. The expression of the bleomycin binding protein confers resistance by binding to and sequestering the drug and thus preventing its association and hydrolysis of DNA.

Methods using cell survival proteins as reporter proteins in screening for inhibitors that disrupt PPIs are known. See, for example, Park et al. (2007), which describes methods involving beta-lactamase in a fragmentation complementation strategy.

In some embodiments, the cell survival protein is a dihydrofolate reductase (DHFR). The DHFR may be murine DHFR, which may be the protein identified by UniProt accession number P00375-1 (version 3, last modified 23 January 2007). For example, the murine DHFR may have an amino acid sequence that is at least 80%, at least 85%, or at least 90% identical to the sequence set forth in **SEQ ID NO: 1.** In particularly preferred embodiments, the murine DHFR has an amino acid sequence that is at least 90%, at least 91%, at least 92%, at least 93%, at least 94%, at least 95%, at least 96%, at least 97%, at least 98%, at least 99%, or 100% identical to the sequence set forth in **SEQ ID NO: 2.**

Sequence identity may be measured by any suitable alignment algorithm, including but not limited to the BLAST algorithm (see e.g. the BLAST alignment tool available at blast.ncbi.nlm.nih.gov/Blast.cgi, optionally with default settings), the Needleman-Wunsch algorithm (see e.g. the EMBOSS Needle aligner available at https://www.ebi.ac.uk/Tools/psa/emboss_needle/, optionally with default settings), or the Smith-Waterman algorithm (see e.g. the EMBOSS Water aligner available at https://www.ebi.ac.uk/Tools/psa/emboss_water/, optionally with default settings). Optimal alignment may be assessed using any suitable parameters of a chosen algorithm, including default parameters.

The DHFR may be human DHFR, which may be the protein identified by UniProt accession number P00374-1 (version 2, last modified 23 January 2007). For example, the human DHFR may have an amino acid sequence that is at least 80%, at least 85%, or at least 90% identical to the sequence set forth in **SEQ ID NO: 3.**

In some embodiments, the cell may be conditionally dependent upon the activity of the cell survival protein or cell reproduction protein for its survival or reproduction, respectively. In some cases, the cell may not contain an endogenous cell survival protein or cell reproduction protein and therefore requires the addition of an exogenous protein to proliferate. In other cases, the cell may contain an endogenous cell survival protein or endogenous cell reproduction protein that is necessary for cell survival or proliferation, respectively, and the function of this endogenous protein can be inhibited or removed in certain conditions (also termed "selection conditions"). Thus, the cell can make use of the endogenous protein for its survival until the selection conditions are activated, at which point the activity of the cell survival protein (also termed an "exogenous cell survival protein") becomes essential for the cell's survival. This is advantageous as it allows the cells to survive until the screening method is ready to be run. It may be possible to elicit these selection conditions using, for example, a selection agent, where the selection agent is a compound that inhibits the activity of endogenous protein but does not inhibit the activity of the cell survival protein. For example, the endogenous cell survival protein or cell reproduction protein may one of the proteins set out in the first column **Table 1,** above and may be inhibited using the selection conditions set out in the second column.

The term "endogenous" in the context of cell survival proteins and cell reproduction proteins is intended to mean a protein that originates from the cell in which the screening method is being performed. The term "exogenous" in the context of cell survival proteins and cell reproduction proteins is intended to mean a protein that has equivalent activity to the endogenous protein such that it can compensate for a deficiency in the function of the endogenous cell survival protein, but is resistant to selection conditions, e.g. the presence of a particular compound that inhibits the function of the endogenous protein, such that survival or proliferation of the cell is dependent upon the activity of the exogenous protein under these selection conditions. The exogenous and endogenous protein will normally have similar, but not identical amino acid sequences. For example, the exogenous protein may be at least 80%, at least 85%, at least 90%, or at least 95% identical to the endogenous protein and the exogenous protein may contain one or more modifications in its amino acid sequence compared to the amino acid sequence of the endogenous cell survival protein. The exogenous protein and endogenous protein may be orthologues, i.e. genes from different species that descended from a common ancestral sequence. For example, the endogenous cell survival protein or cell reproduction protein may be a bacterial version of the cell survival protein or cell reproduction protein set out above, e.g. in **Table 1** or **Table 2,** and the exogenous protein may be an orthologous protein from a mammalian species, e.g. murine or human. Alternatively or additionally, the exogenous protein may contain one or more mutations in its amino acid sequence that render it resistant to the

selection conditions that inhibits the function of the endogenous protein.

For example, where the cell is a bacterial cell, the endogenous protein may be a bacterial cell survival protein and the exogenous cell survival protein may be an orthologous eukaryotic cell survival protein, such as a mammalian cell survival protein, e.g. mouse or human cell survival protein. A bacterial specific inhibitor can then be used as the selection agent to inhibit the bacterial cell survival protein without affecting the function of the eukaryotic cell survival protein.

In a more specific example, the bacterial cell survival protein may be DHFR from *E. coli,* which may be the protein identified by UniProt accession number P0ABQ4-1 (version 1, last modified 21 July 1986) and the eukaryotic cell survival protein may be mouse or human DHFR, as set out above. Bacterial DHFR, can be specifically inhibited using compounds such as trimethoprim (TMP), rendering cells dependent upon the activity of exogenous DHFR, e.g. murine or human DHFR, for their survival.

Thus, the bacterial cells may be grown in a medium, such as a rich liquid broth medium, until the screening method is ready to be performed. At this point the cells can make use of the endogenous protein in order to survive and/or proliferate. When the screening method is ready to be performed, the cells may be grown in a medium that lacks nucleosides such a purines and a selection agent, such as trimethoprim (TMP) that inhibits bacterial DHFR, added. Once the selection agent is added, the cells are conditionally dependent on the activity of the exogenous cell survival protein, such as mammalian DHFR, for its survival. Cell survival will therefore be dependent on the activity of the cell reporter protein and an increase in cell survival will indicate that the test compound is a functionally active antagonist. A person of ordinary skill in the art would be able to select an appropriate type and amount of selection agent to use such that cell survival is dependent on the activity of the cell reporter protein. For example, where TMP is used to inhibit bacterial DHFR, the concentration may be between 4 - 20 $\mu$M. If the cells are able to survive in presence of TMP, bacterial colonies are typically observed between 2 days and 2 weeks of incubation at 37 °C.

In another example, where the cell is a mammalian cell, the endogenous cell survival protein may be a mammalian DHFR. Methods of using detecting PPIs using a mammalian DHFR as a cell survival protein are described in Remy et al. (2007). Briefly, the principle of the DHFR survival assay in mammalian cells is that cells lacking endogenous DHFR activity, can be rescued by the simultaneous expression of complementary DHFR in media depleted of nucleosides. The assay could be performed in DHFR-negative cells, or selection can be achieved in DHFR-positive cells using an exogenous DHFR as the cell survival protein, where the exogenous DHFR contains one or more mutations that render the DHFR resistant to a selection agent, such as the anti-folate drug methotrexate (MTX). When the cells are grown in the absence of nucleotides with selection for MTX resistance, only those cells that can make use of the exogenous DHFR will survive.

An example of a mutation in a mammalian DHFR that renders the mammalian DHFR resistant to MTX is the F31S mutation, wherein residue numbering is according to the murine DHFR set forth in **SEQ ID NO: 1.** Thus, the cell survival protein may be a murine DHFR that has an amino acid sequence that is at least 80%, at least 85%, at least 90%, or at least 95% identical to the sequence set forth in **SEQ ID NO: 1,** wherein the murine DHFR further comprises a serine (S) at position 31, and wherein residue numbering is according to the murine DHFR set forth in **SEQ ID NO: 1.** The cell survival protein may be a murine DHFR that has an amino acid sequence that is at least 90%, at least 91%, at least 92%, at least 93%, at least 94%, at least 95%, at least 96%, at least 97%, at least 98%, at least 99%, or 100% identical to the sequence set forth in **SEQ ID NO: 2,** wherein the murine DHFR further comprises a serine (S) at position 31.

Such a method may comprise growing the mammalian cells comprising the exogenous cell survival protein under conditions where the cell is dependent on the activity of the exogenous cell reporter protein for survival. For example, the exogenous cell survival protein may be murine DHFR that has been modified to be resistant to the anti-folate drug methotrexate (MTX), and the mammalian cell may be grown in the absence of nucleosides and in the presence of MTX. Cell survival will therefore be dependent on the activity of the cell reporter protein and an increase in cell survival will indicate that the test compound is a functionally active antagonist.

As a further example of a reporter protein that provides an observable phenotype, the reporter protein can be a fluorescent reporter protein. In these cases, binding of the DNA-binding protein to the binding site in the absence of a test compound will inhibit the fluorescent signal. When a test compound is added that is a functionally active antagonist of the DNA-binding protein, this will result in an increase in fluorescent signal and therefore such a method uses fluorescence as an indicator that the test compound is a functionally active antagonist. The cells expressing fluorescence could be sorted (e.g. by fluorescence-activated cell sorting, FACS) in order to rank cells by fluorescence and therefore the most effective test compound(s), where the cell with the highest level of fluorescence indicates the most effective test compound.

Thus, in some embodiments, the reporter protein is a fluorescent reporter protein, such as green fluorescent protein (GFP), yellow fluorescent protein (YFP), mNeonGreen, mCherry or Kusabira-Green fluorescent protein (mKG).

In some embodiments, the reporter protein is a bioluminescence protein, such as a luciferase enzyme. Such proteins work in a similar manner to fluorescent proteins, except that instead of requiring an external light source, they require the addition of luciferin. Cells expressing bioluminescence can be sorted, e.g. by FACS, to rank cells in a similar

manner as described above for fluorescence.

In another example of a reporter protein that provides an observable phenotype, the reporter protein can be an enzyme that acts on a substrate to produce a colorimetric signal. In these cases, binding of the DNA-binding protein to the binding site in the absence of a test compound will inhibit the colorimetric signal. When a test compound is added that is a functionally active antagonist of the DNA-binding protein, this will result in an increase in the colorimetric signal and therefore such a method uses colorimetric signal as an indicator that the test compound is a functionally active antagonist.

Thus, in some embodiments, the reporter protein is an enzyme that acts on a substrate to produce a colorimetric signal. For example, the enzyme may be horseradish peroxidase or beta-galactosidase.

A further example of a reporter protein is a protein kinase, such as the focal adhesion kinase (FAK). FAK is a tyrosine kinase that is made up of distinct domains that are phosphorylated. Phosphorylation can be detected by, for example, lysing and immunoblotting the cell lysate. A method of probing protein-protein interactions using FAK is described, for example, by Ma et al. (2014).

Thus, in some embodiments, the reporter protein is a protein kinase, such as FAK.

Another example of a reporter protein is a protease, such as tobacco etch virus protease (TEV). TEV is a highly specific viral cysteine protease and can be applied to analyse PPIs using a modular approach of various reporters, including ’silent’ fluorescent and luminescent reporter proteins that require proteolysis in order to become active.

Thus, in some embodiments, the reporter protein is a protease, such as TEV, used in combination with a silent fluorescent or luminescent reporter protein that requires proteolysis in order to become active. A method of monitoring PPIs using TEV is described, for example, by Wehr et al. (2006).

In some embodiments, the reporter protein is a transcription factor, such as a transcriptional activator. Examples of transcriptional activators include GAL4, which is well known for its use in "two hybrid" systems for studying PPIs. See, for example, Young, 1998. A transcriptional activator binds a DNA sequence causing activation of a downstream reporter gene. For example, GAL4 binds the UAS and drives transcription of the downstream reporter gene. The downstream reporter gene may encode any of the reporter proteins described above, for example, it may encode a cell survival protein or may encode a fluorescent protein. Expression of the transcriptional activator can therefore be measured indirectly by measuring expression of the protein encoded by the downstream reporter gene.

In some embodiments, the reporter protein is not a split reporter protein. Split reporter proteins are made up of a functional reporter protein that has been split into two or more inactive fragments, i.e. the inactive fragments do not provide a phenotypic readout unless they are reassembled. Thus, in some embodiments the reporter protein is capable of providing a phenotypic readout without requiring reassembly with another protein or peptide.

*Expression cassettes*

In this specification, the term "expression cassette" is intended to mean a DNA polynucleotide sequence that is capable of effecting transcription of an expression product. The expression cassette and may be derived from a eukaryotic gene or a prokaryotic gene. A eukaryotic gene typically comprises, from 5’ to 3’, a promoter, a 5’ untranslated region (UTR), an open reading frame made up of exons and introns, a 3’ UTR and may further comprise one or more enhancers and/or silencers. Promoters are well known to be regions of DNA that are responsible for the initiation of transcription. Enhancers, are well known to be regions of DNA that can be bound by activator proteins to increase the likelihood that transcription will progress. Silencers are well known to be regions of DNA that can be bound by repressor proteins to decrease the likelihood that transcription will progress.

During transcription in eukaryotic cells, the eukaryotic gene is typically first transcribed into pre-mRNA in the nucleus of the cells, which contains the 5’ and 3’ UTRs and the exons and introns that make up the open reading frame. Following this, the pre-mRNA is processed into mRNA, which involves the addition of a 5’ cap to the beginning of the RNA, the addition of a poly-A tail to the end of the RNA and the removal of introns. The final mature mRNA is then able to travel out of the nucleus and be translated into a protein.

A prokaryotic gene has a similar structure, except it does not contain introns within the open reading frame. This means that the RNA transcript of a prokaryotic gene is ready to act as a mature mRNA and does not require the processing that features in eukaryotic cells. The transcription of an operon’s mRNA is often controlled by a repressor that binds to a segment of DNA known as an operator. For example, the Lac operon encodes a repressor protein, which is under allosteric regulation. In the prokaryotic cell, the repressor protein is normally bound to the operator, which prevents transcription of the open reading frame. However, when the repressor is bound to the effector molecule lactose, or the structural analogue isopropyl β-D-1-thiogalactopyranoside (IPTG), the repressor will not bind to the operator, which allows transcription to occur. In this way, the initiation of transcription is dependent upon the availability of lactose or IPTG within the prokaryotic cell.

A "coding sequence" is intended to mean a portion of a gene’s DNA sequence that encodes the expression product. Where the expression product is a protein, this sequence may be referred to as a "protein coding sequence".

The protein coding sequence typically begins at the 5' end by a start codon and ends at the 3' end with a stop codon. Furthermore, the protein coding sequence is typically the sequence of the gene exon(s) that in a gene is flanked by 5' and 3' UTRs. An example of a protein coding sequence is set forth in **SEQ ID NO: 4.**

Typically, the expression cassette comprises a promoter operably linked to a protein coding sequence. The term "operably linked" includes the situation where a selected coding sequence and promoter are covalently linked in such a way as to place the expression of the protein coding sequence under the influence or control of the promoter. Thus a promoter is operably linked to the protein coding sequence if the promoter is capable of effecting transcription of the protein coding sequence. Where appropriate, the resulting transcript may then be translated into a desired protein. In some embodiments, the expression cassette may further comprise further components of a eukaryotic or prokaryotic gene, such as one or more selected from the a list consisting of: an intron, an enhancer, a silencer, a 5' UTR, a 3' UTR, and a regulator.

Any suitable promoter known in the art may be used in the expression cassette providing it functions in the cell type being used. For example, where the cell is a bacterial cell, expression may be under control of the lac operon. In such cases, the cell may also contain a lac repressor protein, whereby expression can be controlled by the introduction of isopropyl β-D-1-thiogalactopyranoside (IPTG). The promoter may be endogenous to the cell in which the method is being carried out. Where multiple expression cassettes are used, each coding sequence may be independently operably linked to its own promoter. Alternatively, the coding sequence for one or more of the expression cassettes may be operably linked to the same promoter.

As already described here, the reporter expression product is encoded by a reporter expression cassette. The DNA-binding protein may also be encoded by an expression cassette, termed herein a "DNA-binding protein expression cassette". Where the DNA-binding protein is a protein complex, such as a heterodimer, each component of the complex may be encoded on the same expression cassette or on separate expression cassettes. The test compound may also be a peptide or polypeptide that is expressed intracellularly from an expression cassette, termed herein a "test compound expression cassette".

The expression cassettes described herein may be part of one or more expression vector(s). An "expression vector" as used herein is a DNA molecule used for expression of foreign genetic material in a cell. Any suitable vectors known in the art may be used. Suitable vectors include plasmids, binary vectors, viral vectors and artificial chromosomes (e.g. yeast artificial chromosomes). Alternatively, the expression cassettes described herein may be incorporated into the genome of the cell.

The methods described herein may comprise administering one or more expression cassettes described herein to the cell. For example, the method may comprise administering a reporter expression cassette, DNA-binding protein expression cassette, and/or a test compound expression cassette to the cell, optionally where the expression cassette(s) are part of one or more expression vector(s). Molecular biology techniques suitable for administering expression cassettes and producing proteins such as the DNA-binding protein and reporter protein described herein in cells are well known in the art, such as those set out in Sambrook et al., Molecular Cloning: A Laboratory Manual, New York: Cold Spring Harbor Press, 1989.

*Reporter expression cassette and binding site(s)*

As described above, the reporter expression cassette comprises at least one binding site such that binding of the DNA-binding protein to the binding site is capable of inhibiting expression of the reporter expression product. Preferably, the expression product comprises a plurality of such binding sites.

The at least one binding site may be located anywhere in the reporter expression cassette, providing binding of the DNA-binding protein to the at least one binding site is capable of inhibiting expression of the reporter expression product. For example, the binding site(s) may be located in a promoter, protein coding sequence, enhancer, silencer, 5' UTR, 3' UTR, regulator, exon, and/or intron. Binding of the DNA-binding protein to the binding site(s) may inhibit expression of the reporter expression product to less than 50%, less than 40%, less than 30%, less than 20%, less than 10%, or less than 5% of the expression of the reporter expression product when the cell comprises the reporter expression cassette without the DNA-binding protein.

Thus, in some embodiments the reporter expression cassette comprises at least 2, at least 3, at least 4, at least 5, at least 6, at least 7, at least 8, at least 9, at least 10, at least 11, at least 12, at least 13, at least 14, or at least 15 binding sites. Preferably, the reporter expression cassette comprises at least 2, more preferably at least 5, even more preferably at least 10, still more preferably at least 12, still further preferably at least 15 binding sites. In some embodiments, the reporter expression cassette comprises: between 1 and 20, between 1 and 18, between 1 and 15, between 1 and 10, between 1 and 5, between 2 and 20, between 2 and 18, between 2 and 15, between 2 and 10, between 2 and 5, between 5 and 18, between 5 and 10, between 10 and 18, or between 12 and 16 binding sites. In some embodiments, the reporter expression cassette comprises up to 5, up to 10, up to 15, up to 18, up to 20 binding sites. In an exemplified embodiment, the reporter expression cassette comprises 15 binding sites.

Some or all of the binding site(s) are located in the transcribed sequence of the reporter expression cassette, e.g. in the coding sequence of the reporter expression cassette. Preferably, the reporter expression cassette comprises a plurality of binding sites that are located in the transcribed sequence or coding sequence. Without wishing to be bound by theory, it is believed that a plurality of binding sites located in the transcribed region or coding sequence will increase the likelihood that binding of the DNA-binding protein to the binding sites will efficiently inhibit expression of the reporter expression product.

In embodiments where the reporter expression product is a reporter protein, it is preferable that the expression product is functional in order to determine whether the expression of the reporter protein is increased in the presence of the test compound of the screening method. In preferred embodiments, the presence of the binding site(s) in the reporter expression cassette does not substantially affect the function of the reporter protein. For example, the reporter protein may retain at least 50%, at least 70%, at least 90%, or at least 95% of the function of a parent reporter protein, wherein the parent reporter protein is encoded by a parent reporter expression cassette that corresponds to the reporter expression cassette but does not comprise the binding site(s).

In order to preserve the activity of the reporter protein, at least some of the binding site(s), preferably the majority of the binding site(s) may be introduced into the protein coding sequence of the reporter expression cassette as silent, semi-conservative and/or conservative mutations. The protein coding sequence is made up of a series of codons, each of which encodes a specific amino acid or stop signal when the protein coding sequence is transcribed and translated. Silent mutations are mutations in a codon of the protein coding sequence that do not affect the resulting amino acid residue of the codon. For example, the codon GCA encodes the amino acid Alanine (A). Mutating the GCA codon to GCG would be considered a silent mutation as the GCG codon still encodes the amino acid Alanine (A).

A conservative or semi-conservative mutation is a change to a given codon that leads to the replacement of one amino acid with a biochemically similar one, e.g. as set out according to the following table.

| | | |
|---|---|---|
| Hydrophobic (non-polar) | Alkyl | G A V L I M P |
| | Aromatic | F Y W |
| Hydrophilic (polar) | Neutral | S T C Q N |
| | Acidic | E D |
| | Basic | K H R |

For example, a change to a given codon that replaces a hydrophobic amino acid for another hydrophobic amino acid, or a hydrophilic amino acid for another hydrophilic amino acid, may be considered a semi-conservative mutation. For example, a change to a given codon that replaces a serine (S) to aspartic acid (D) may be considered a semi-conservative mutation. A change to a given codon that replaces an alkyl amino acid for another alkyl amino acid, or an aromatic amino acid for another aromatic amino acid, or a neutral amino acid for another neutral amino acid, or an acidic amino acid for another acidic amino acid, or a basic amino acid for another basic amino acid, may be considered a conservative mutation. For example, a change to a given codon that replaces a neutral, hydrophilic amino acid for another neutral, hydrophilic amino acid (e.g. threonine (T) to glutamine (Q)) may be considered a conservative mutation.

Thus, the reporter protein may have an amino acid sequence that is at least 80%, at least 85%, at least 90%, or at least 95% identical to a parent reporter protein, wherein the parent reporter protein is encoded by a parent reporter expression cassette that corresponds to the reporter expression cassette but does not comprise the binding site(s).

In some embodiments, the majority of the differences in the amino acid sequence of the reporter protein and the amino acid sequence of the parent reporter protein are conservative and/or semi-conservative substitutions. In these cases, it is expected that the reporter protein will have substantially the same function as the parent reporter protein.

The location of the binding site(s) in the reporter expression cassette may be selected so as to avoid affecting the function of the reporter protein. For example, the binding site(s) may be located at a position in the protein coding sequence that does not encode a residue that forms part, or is in close proximity to the catalytic centre (active site) of the reporter protein, or forms part, or is in close proximity to a residue involved in cofactor binding (e.g. NADH, NDDPH). Close proximity can mean that the residue is less than 15 Å, more preferably less than 10 Å, even more preferably less than 5 Å away from a residue that forms part of the catalytic centre and/or is involved in cofactor binding. Alternatively or additionally, close proximity can mean that the residue is less than 5 residues, more preferably less than 4 residues, even more preferably less than 3 residues, still more preferably less than 2 residues away from a residue that forms part of the catalytic centre and/or is involved in cofactor binding, when assessed in a linear sequence of amino acids.

Changes outside the catalytic centre of the reporter protein are expected to minimise functional alterations. Alternatively or additionally, the binding site(s) may be located at a position in the protein coding sequence that encodes a solvent exposed residue in the reporter protein. Changes made at solvent exposed regions of the reporter protein are

expected to minimise the structural perturbations and therefore minimise perturbation to the overall function.

Methods of identifying the solvent exposed regions of the reporter protein are known. For example, it is possible to take the coordinate files for the reporter protein, e.g. a protein databank (PDB) file and use a program that calculates the accessible surface area (ASA) which informs the user how exposed / buried residues are within a structure. An exemplary ASA program can be found at http://cib.cf.ocha.ac.jp/bitool/ASA/. An exemplary cut-off value of 20 $Å^2$ can be used, such that residues that are lower than this are considered to be buried and greater than this are considered exposed. In this way, the locations of solvent exposed residues can be identified and codons modified accordingly.

In some embodiments, the reporter expression cassette encodes a reporter protein that is a fusion protein, where the fusion protein comprises two or more of the cell survival proteins, cell reproduction proteins, fluorescence proteins, bioluminescence proteins, enzymes that act on a substrate to produce a colorimetric signal, protein kinases, proteases, transcription factors, and regulatory proteins that are described herein. For example, the reporter expression cassette may encode a fusion protein comprising a cell survival protein as described herein and a fluorescence protein as described herein. In such an example, the binding site(s) may be located in the part of the reporter expression cassette (e.g. the coding sequence) that encodes the cell survival protein. This exemplary reporter expression cassette would therefore provide a two readouts of efficacy, namely cell survival and fluorescence. In a particular example, the fusion protein may comprise a DHFR as a cell survival protein and mNeonGreen as a fluorescence protein.

*DNA-binding protein*

The DNA-binding protein used in the methods of the invention may be or may contain a transcription factor that binds to a binding site (also known as a recognition site) in a sequence specific manner, or a DNA-binding fragment thereof. It is expected that any transcription factor, or DNA-binding fragment thereof, that is capable of binding to a binding site in a sequence specific manner can be used with the methods described herein. In particular embodiments, the transcription factor is a eukaryotic transcription factor, such as a human transcription factor. As described in the background section, numerous transcription factors are known to be deregulated in diseases such as cancers. In some embodiments, the human transcription factor is known to be, or suspected of being, deregulated in diseases such as cancers. Accordingly, the identification of functionally active antagonists of transcription factors, or DNA-binding fragments thereof, is expected to be useful in the identification of antagonists that may be useful in the treatment of cancers.

The DNA-binding protein may be a protein complex comprising one or more polypeptide chains. For example, it may be a dimeric protein complex, which may be a homodimer or a heterodimer.

The DNA-binding protein may be any of the human transcription factors described in Vaquerizas et al. (2009) (e.g. any of those listed in Supplementary information S3), or a DNA-binding fragment thereof. For example, the DNA-binding protein may be a member of the C2H2 zinc-finger family, the homeodomain family or the helix-loop-helix family or a DNA-binding fragment thereof.

In some embodiments, the DNA-binding protein is a basic leucine zipper (bZIP), basic helix-loop helix (bHLH) or bHLH leucine zipper (bHLH-Zip) transcription factor, or a DNA-binding fragment thereof. bHLH and bHLH-Zip transcription factors are exclusively eukaryotic proteins that bind to sequence-specific double-stranded DNA as homodimers or heterodimers to either activate or repress gene transcription. bZIP transcription factors form one of the largest families of transcription factors in eukaryotic cells contain a basic region that contacts DNA bases in order to bind to its DNA-binding site. bZIP transcription factors also contain leucine repeats through which the proteins dimerize to form a coiled coil, which is required for functional activity of the protein. As well as human proteins, certain viral proteins such as BZLF1 form part of the bZIP family. Details of bZIP, bHLH and bHLH-ZIP transcription factors and their consensus sequences are provided in Vinson et al. (2002), Newman & Keating (2003) and Rodriguez-Martinez et al. (2017). Typically, the DNA-binding fragment of these transcription factors will be (or will comprise) the basic portions of the transcription factors which physically interact with DNA.

Exemplary bHLH transcription factor include ATOH1, AhR, AHRR, ARNT, ASCL1, BHLH2, BHLH3, BHLH9, ARNTL, ARNTL2, CLOCK, EPAS1, FIGLA, HAND1, HAND2, HES5, HES6, HEY1, HEY2, HEYL, HES1, HIF1A, HIF3A, ID1, ID2, ID3, ID4, LYL1, MESP2, MXD4, MYCL1, MYCN, MyoD, Myogenin, MYF5, MYF6, Neurogenin1, Neurogenin2, Neurogenin3, NeuroD1, NeuoD2, NPAS1, NPAS2, NPAS3, OLIG1, OLIG2, Pho4, Scleraxis, SIM1, SIM2, TAL1, TAL2, Twist and USF1. Exemplary bHLH-ZIP transcription factors include AP-4, Max, MXD1, MXD3, MITF, MNT, MLX, MLXIPL, MXI1, Myc, SREBP1 and SREBP2. In particular embodiments, the bHLH-ZIP transcription factor used may be c-Myc or Max, or a heterodimer between c-Myc and Max (c-Myc-Max). bHLH and bHLH-ZIP transcription factors typically bind to a consensus sequence called an E-box, which can have the sequence CANNTG ('N' being any nucleotide) and in particular cases has the sequence CACGTG. The DNA-binding protein may be or may comprise any of these bHLH or bHLH-Zip transcription factors and the reporter expression cassette comprise at least one E-box as a binding site, where the E-box may have the sequence CANNTG, e.g. CACGTG.

Exemplary human bZIP transcription factor subfamilies, the nucleotide sequences of their binding sites and examples of proteins of these subfamilies are set forth in the following table. The DNA-binding protein may be, or may

comprise, any of these human bZIP proteins or a DNA-binding fragment thereof, and the at least one binding site may be the nucleotide sequence of the binding site set forth in the same row as the bZIP protein in the table below. For example, the DNA-binding protein may be a protein of the Fos/Jun subfamily (e.g. cJun) and the at least one binding site may have the nucleotide sequence TGACTCA or TGAGTCA.

| Human bZIP subfamily | Exemplary bZIP protein | Nucleotide sequence(s) of binding site | Name of binding site |
|---|---|---|---|
| PAP | PAP1, YAP1, YAP2, YAP3, YAP4, YAP5, YAP6, YAP7, Cap1 | TTACGTAA | PAP/CREB-2/PAR |
| CREB-2 | AFT4, mATFP4, ApCREB-2, hCREB2, acr1 | | |
| PAR | DBP, VBP/TEF, HLF, CES2, TEF | | |
| C/EBP | C/EBPα, C/EBPβ, C/EBPδ, C/EBPε, C/EBPγ, CRP1, CRP2, CRP3, Ig/EBP, lap, DDIT3 | ATTGCGCAAT | CCAAT |
| Fos/Jun | cFos, FRA1 (FosL1), FRA2 (FosL2), cJun, JUNB, JUND, GCN4, BATF, BATF2, BATF3 | TGACTCA or TGAGTCA | TPA response element (TRE) |
| CREB | CREB1, ATF1, ATF2, ATF3, ARF5, ATFa, BBF-2, CREB3L1 | TGACGTCA | cAMP response element (CRE) |
| Maf | MafA, MafB, BACH1, BACH2 | TGCTGA(G/C)TCAGCA and TG CTGAG (C/C) GTCAG CA | Maf recognition element (MARE) |

AP-1 is a dimer, typically a heterodimer, that is composed of proteins belonging to the Fos/Jun subfamily (e.g. cFos, FRA1, FRA2, cJun, JUNB, JUND, GCN4, BATF, BATF2, BATF3).

In addition to the human bZIP transcription factors, certain viral proteins that bind DNA also belong to the bZIP family. This includes the bZIP transactivator of Epstein-Barr virus, BZLF1. BZLF1 can bind to either the TRE binding site (TGACTCA or TGAGTCA) or the CCAAT binding site (ATTGCGCAAT). The DNA-binding protein may be, or may comprise, BZLF1 or a DNA-binding fragment thereof, and the at least one binding site may be a TRE binding site or CCAAT binding site.

The DNA-binding protein may alternatively be a transcription factor or DNA-binding fragment thereof that does not form part of the bZIP, bHLH or bHLH-ZIP families. Examples of additional suitable eukaryotic transcription factors which the DNA-binding protein may be, comprise or may be derived from are set forth in the following table, along with the nucleotide sequences of their DNA binding sites and the names of these binding sites. The DNA-binding protein may be, or may comprise, any of these eukaryotic (e.g. human) transcription factors or a DNA-binding fragment thereof, and the at least one binding site may be the nucleotide sequence of the binding site set forth in the same row as the transcription factor in the table below.

| Eukaryotic transcription factor(s) | Name of binding site | Nucleotide sequence(s) of binding site |
|---|---|---|
| CAAT-box binding factor * | CAAT box | GGCCAATCT |
| Serum response factor * | CArG box | $CC(A/T)_6GG$ |
| Snail proteins (e.g. SNAI1) * | E2 box | CAGGTG and CACCTG |
| Runx2 * | HY box | TG(A/T)GGG |
| T box transcription factors * | T box | TCACACCT |
| RNA polymerase in eukaryotes * | TATA box | TATAAA |
| RFX proteins (e.g. RFX1) * | X box | GTTGGCATGGCAAC |
| Y box binding protein * | Y box | (A/G)CTAACC(A/G)(A/G)(C/T) |
| Ethylene-responsive element binding proteins | ATA box | AAATAT |
| AtSR1 (*Arabidopsis thaliana* signal-responsive genes) | CGCG box | (A/C/G)CGCG(C/G/T) |

(continued)

| Eukaryotic transcription factor(s) | Name of binding site | Nucleotide sequence(s) of binding site |
|---|---|---|
| Dehydration-responsive element-binding (DREB)-like proteins | DREB box | TACCGACAT |
| Fur protein | Fur box | GATAATGATAATCATTATC |
| EmBP1 | G box | GCCACGTGGC |
| EREBP-like proteins | GCC box | AGCCGCC |
| KAP-2 protein | H box | ACACCA |
| barley prolamin-box (P-box) binding factor | Prolamin box | TGTAAAG |
| Aleurone proteins | Pyrimidine box | CCTTTT |
| U2 snRNP | TACTAAC box | ATTTACTAAC |
| * Eukaryotic transcription factors that are also human transcription factors. | | |

Thus, in some embodiments,

a) the at least one binding site is a TPA response element (TRE) having the nucleotide sequence **TGACTCA (SEQ ID NO: 5)** or **TGAGTCA (SEQ ID NO: 6);**

b) the at least one binding site is an Ebox response element having the nucleotide sequence **CACGTG (SEQ ID NO: 7)** or **CACATG (SEQ ID NO: 8);**

c) the at least one binding site is a CCAAT binding site having the nucleotide sequence **ATTGCGCAAT (SEQ ID NO: 9);**

d) the at least one binding site is a cAMP response element (CRE) having the nucleotide sequence **TGACGTCA (SEQ ID NO: 10);**

e) the at least one binding site is a Maf recognition element (MARE) having the nucleotide sequence **TGCTGA$^{G}/_{C}$TCAGCA (SEQ ID NO: 32)** or **TGCTGA$^{GC}/_{CG}$TCAGCA (SEQ ID NO: 33);** or

f) the at least one binding site is a PAP/CREB-2/PAR binding site having the nucleotide sequence **TTACGTAA (SEQ ID NO: 34).**

In more specific embodiments,

a) the at least one binding site is a TPA response element (TRE) having the nucleotide sequence **TGACTCA (SEQ ID NO: 5)** or **TGAGTCA (SEQ ID NO: 6);**

b) the at least one binding site is an Ebox response element having the nucleotide sequence **CACGTG (SEQ ID NO: 7)** or **CACATG (SEQ ID NO: 8);** or

c) the at least one binding site is a CCAAT binding site having the nucleotide sequence **ATTGCGCAAT (SEQ ID NO: 9).**

In even more specific embodiments, the at least one binding site is a TPA response element (TRE) having the nucleotide sequence **TGACTCA (SEQ ID NO: 5)** or **TGAGTCA (SEQ ID NO: 6).**

In particular embodiments,

a) the DNA-binding protein is AP-1 or a member of the Fos/Jun subfamily of transcription factors (such as c-Jun),

or a DNA-binding fragment thereof, and the at least one binding site is a TPA response element (TRE) having the nucleotide sequence **TGACTCA (SEQ ID NO: 5)** or **TGAGTCA (SEQ ID NO: 6);**

b) the DNA-binding protein is a bHLH transcription factor, such as c-Myc or Max, or a DNA-binding fragment thereof, and the at least one binding site is an Ebox response element having the nucleotide sequence **CACGTG (SEQ ID NO: 7)** or **CACATG (SEQ ID NO: 8);**

c) the DNA-binding protein is a member of the C/EBP subfamily of transcription factors (such as C/EBP alpha), or a DNA-binding fragment thereof, and the at least one binding site is a CCAAT binding site having the nucleotide sequence **ATTGCGCAAT (SEQ ID NO: 9);**

d) the DNA-binding protein is BZLF1, or a DNA-binding fragment thereof, and the at least one binding site is a TPA response element (TRE) having the nucleotide sequence **TGACTCA** (**SEQ ID NO: 5)** or **TGAGTCA (SEQ ID NO: 6),** or a CCAAT binding site having the nucleotide sequence **ATTGCGCAAT (SEQ ID NO: 9);**

e) the DNA-binding protein is a member of the CREB subfamily of transcription factors (such as CRE), or a DNA-binding fragment thereof, and the at least one binding site is a cAMP response element (CRE) having the nucleotide sequence **TGACGTCA (SEQ ID NO: 10);**

f) the DNA-binding protein is a Maf transcription factor, or a DNA-binding fragment thereof, and the at least one binding site is a Maf recognition element (MARE) having the nucleotide sequence **TGCTGA$^{G}/_{C}$TCAGCA (SEQ ID NO: 32)** or **TGCTGA$^{GC}/_{CG}$TCAGCA (SEQ ID NO: 33);**

g) the DNA-binding protein is a member of the poly(ADP-ribose) (PAR) subfamily of transcription factors, or a DNA-binding fragment thereof, and the at least one binding site is a PAP/CREB-2/PAR binding site having the nucleotide sequence **TTACGTAA** (**SEQ ID NO: 34**) ; or

h) the DNA-binding protein is a member of the CREB-2 subfamily of transcription factors, or a DNA-binding fragment thereof, and the at least one binding site is a PAP/CREB-2/PAR binding site having the nucleotide sequence **TTACGTAA (SEQ ID NO: 34).**

In more specific embodiments,

a) the DNA-binding protein is AP-1 or a member of the Fos/Jun subfamily of transcription factors (such as c-Jun), or a DNA-binding fragment thereof, and the at least one binding site is a TPA response element (TRE) having the nucleotide sequence **TGACTCA (SEQ ID NO: 5)** or **TGAGTCA (SEQ ID NO: 6);**

b) the DNA-binding protein is a bHLH transcription factor, such as c-Myc or Max, or a DNA-binding fragment thereof, and the at least one binding site is an Ebox response element having the nucleotide sequence **CACGTG (SEQ ID NO: 7)** or **CACATG (SEQ ID NO: 8);**

c) the DNA-binding protein is a member of the C/EBP subfamily of transcription factors (such as C/EBP alpha), or a DNA-binding fragment thereof, and the at least one binding site is a CCAAT binding site having the nucleotide sequence **ATTGCGCAAT (SEQ ID NO: 9);** or

d) the DNA-binding protein is BZLF1, or a DNA-binding fragment thereof, and the at least one binding site is a TPA response element (TRE) having the nucleotide sequence **TGACTCA** (**SEQ ID NO: 5)** or **TGAGTCA (SEQ ID NO: 6),** or a CCAAT binding site having the nucleotide sequence **ATTGCGCAAT (SEQ ID NO: 9).**

In particular embodiments, the DNA-binding protein is AP-1, a protein of the Fos/Jun subfamily (e.g. c-Jun), or a DNA-binding fragment thereof, and the at least one binding site is a TPA response element (TRE) having the nucleotide sequence **TGACTCA (SEQ ID NO: 5)** or **TGAGTCA (SEQ ID NO: 6).** An example of a DNA-binding fragment of AP-1 is the basic motif of any of the proteins that are members of the Fos/Jun subfamily, e.g. a fragment containing the basic motif of c-Jun, as used in the examples. The basic motif of c-Jun may be the sequence set forth in positions 252-279 of UniProt accession P05412, version 2. As described elsewhere, bZIP transcription factors bind as a homodimer or heterodimer and therefore the bZIP transcription factor may contain a dimerization domain, such as a leucine zipper motif, in addition to its DNA-binding domain.

As described in the examples, a reporter expression cassette encoding murine DHFR as a reporter protein

was generated, where the reporter expression cassette contained 15 TREs in its protein coding sequence. This exemplified protein coding sequence of this reporter expression cassette has the sequence set forth in **SEQ ID NO: 4.**

Thus, in some embodiments the reporter expression cassette comprises a protein coding sequence that is at least 90%, at least 95%, at least 98%, or 100% identical to the sequence set forth in **SEQ ID NO: 4** and the DNA-binding protein is AP-1 or a DNA-binding fragment thereof.

*Cells*

The method for screening for a functionally active antagonist of the invention functions in isolated live cells, i.e. the methods are performed *in cellulo* unless the context clearly dictates otherwise. The term *"in cellulo"* is intended to encompass experiments that take place involving cells and may be on cultured cells or may be on cells or tissues that have been taken from an organism. The methods of the invention are not practiced on the human or animal body.

Any cell suitable for the expression of expression products may be used for the screening method described herein. The cell may be a prokaryote or eukaryote. Typically the cells are isolated cells.

The cell used in the screening method may be a bacterial cell. In some embodiments, the bacterial cell is an *Escherichia coli cell,* for example BL21 (DE3), XL-1, RV308, or DH5alpha cells. Screening methods where the cell is a bacterial cell may involve culturing the bacterial cell in suitable media. Such techniques are well known to those of skill in the art.

Alternatively, the cell is a eukaryotic cell such as a yeast cell, a plant cell, insect cell or a mammalian cell. In some embodiments, the cell is a mammalian cell, for example a human cell. Mammalian cells, especially human cells, may be somatic cells. Screening methods where the cell is a eukaryotic cell may involve culture or fermentation of the eukaryotic cell. The culture or fermentation may be performed in a bioreactor provided with an appropriate supply of nutrients, air/oxygen and/or growth factors. Culture, fermentation and separation techniques are well known to those of skill in the art.

As described above, it is expected that test compounds that are functionally active antagonists of DNA-binding proteins, in particular transcription factors that are known to be dysregulated in human cancers, will have therapeutic use in the treatment of diseases such as cancer. In some embodiments where a mammalian cell is used, the DNA-binding protein is naturally produced by the mammalian cell, i.e. the DNA-binding protein is not administered to the cell, e.g. in the form of a DNA-binding protein expression vector. In particular, the mammalian cell may be a human cell isolated from a cancer patient where the DNA-binding protein is suspected of being or known to be dysregulated in the cell. This is believed to be advantageous, as the method will test whether the test compound can functionally antagonise the natural expression of the DNA-binding protein and therefore the results obtained are expected to be reflective of what may occur *in vivo.*

Methods where the cell is a human cell have the additional advantage in that as well as screening for functionally active antagonists of the DNA-binding protein, the method will simultaneously profile the test compound for further desirable properties that are conducive to drug development. For example, it can be used to determine if the test compound is toxic, if it is selective for the DNA-binding protein in question and whether the test compound is stable in human cells. This compares favourably to known methods for identifying inhibitors of PPI that function as therapeutic compounds in human cells, where a first step would be to identify a PPI inhibitor, the second step to confirm that the PPI inhibitor ablates protein function and then the third step to check that it functions in human cells. The present invention therefore advantageously allows all these individual steps to be combined into an intracellular screening step in human cells.

For example, as described in the reporter protein section above, where the cell is a mammalian cell the reporter protein may be mammalian DHFR, e.g. murine DHFR that has been modified such that it is rendered resistant to the anti-folate drug methotrexate (MTX), for example as described by Remy et al. (2007). In this way, cell survival can be used as a readout to determine whether the test compound is a functionally active antagonist of the DNA-binding protein in question in the human cell.

*Test compound*

The test compounds for use with the screening method of the invention are not particularly limited. In some embodiments, the test compound is peptidic. "Peptidic" as used herein includes compounds that are composed of or comprise a linear chain of amino acids linked by peptide bonds and include peptides and polypeptides. In this specification the term "peptide" is intended to mean molecules that consist of between 2 and 50 amino acids and the term "polypeptide" is intended to mean molecules that are made up of more than 50 amino acids. In other embodiments, the test compound is a small molecule, synthetic or naturally occurring. A small molecule is compound (typically an organic compound) that has a molecular weight of 500 daltons or less.

In some embodiments, the test compound is a peptide mimetic. The terms "peptide mimetic", "peptidomimetic"

and "peptide analogue" are used interchangeably and refer to a chemical compound that is not entirely composed of amino acids but has substantially the same characteristics as a peptidic compound that is entirely composed of amino acids. A peptide mimetic may be peptidic, in that it is a chimeric molecule that it is made up of both natural peptide amino acids and non-natural analogues of amino acids. Alternatively, a peptide mimetic may not be peptidic, in that it is entirely composed of synthetic, non-natural analogues of amino acids. Peptide mimetics may be classified as set out in Pelay-Gimeno et al. (2015). Briefly 'class A' mimetics correspond to peptidic compounds that are mainly formed by amino acids with minor side chain or backbone alterations; 'class B' mimetics correspond to peptidic compounds with various backbone and side chain alterations; 'class C' mimetics correspond to small molecule-like scaffolds that project substituents in analogy to peptide side chains; and 'class D' mimetics correspond to molecules that mimic the mode of action of a peptide without a direct link to its side chains.

In some embodiments, the test compound is a peptidic test compound that is expressed intracellularly from a nucleotide sequence. For example, the nucleotide sequence may be an expression cassette (also termed a "test compound expression cassette", which may be contained in a vector present in the cell, or may be incorporated into the genome of the cell as described above.

The screening method of the invention is expected to have use with genetically encoded peptidic libraries. Genetically encoded peptidic libraries are known and have been used in screening methods for identifying inhibitors of DNA-binding proteins. See, for example, Mern et al. (2010). Briefly, such libraries are formed from libraries of test compound expression cassettes, each of which encodes and is capable of directing expression of a different peptidic test compound. By transforming the library into cells containing the DNA-binding protein and reporter expression cassette, it is possible to determine whether a given library member can act as a functionally active antagonist of the DNA-binding protein. Such genetically encoded peptidic libraries can be used with the method of the present invention to rapidly screen multiple different test compounds at the same time.

Thus in some embodiments, the cell used in the method was obtained from a pool of cells that were transformed with a genetically encoded library of peptidic test compounds, such that the cell expresses the peptidic test compound intracellularly.

The present inventors have also recognised that the screening method can be used with test compounds that are added extracellularly. For example, cells containing the DNA-binding protein and reporter expression cassette can be cultured and plated onto microtiter plates (e.g. 1536 well plates) and test compound libraries screened by direct addition to each well. Addition of the test compound libraries to the wells can occur before or after addition of the cells. This method can be used to rapidly screen multiple different test compounds and has the additional advantage of allowing the user to move away from standard peptide libraries, for example allowing the user to profile for helix constrained peptides, peptidomimetics, non-natural amino acids, or even small molecule libraries. Test compounds that are added extracellularly must be able to cross the cell membrane (and cell wall, if present) in order to enter the cell and be screened to determine if they are functionally active antagonists using the methods of the invention. This means that the extracellular test compound addition method allows the user to profile for cell penetrance concomitantly with functional antagonism of the DNA-binding protein as an increase in expression of the reporter expression product will indicate that the test compound is capable of entering the cell and capable of inhibiting DNA-binding activity of the DNA-binding protein. Compounds used in a therapeutic setting in humans will need to enter the cells in order to have a therapeutic effect. Thus, without wishing to be bound by theory, it is expected that those extracellularly-added test compounds that result in an increase in expression of reporter expression product using the methods described herein represent good candidates for taking forward as potential therapeutic agents. Furthermore, because cell penetrance and functional antagonism is determined concomitantly, this compares favourably to methods that require separate assays to test for cell penetrance and for *in cellulo* functional antagonism.

In some embodiments, transcription of the DNA-binding protein and reporter expression product in the cell may be initiated first (e.g. through activation of the promoter(s) that is/are operably linked to the DNA-binding protein expression cassette and reporter expression cassette), followed by administering or expressing the test compound in the cell. That is, in these embodiments the DNA-binding protein is transcribed, translated and allowed to bind its DNA-binding site(s) to form a DNA-bound complex in the reporter expression cassette before the test compound is present in the cell. Without wishing to be bound by theory, it is believed that in these embodiments, an increase in expression of the reporter expression product after the test compound is added provides an indication that the test compound is able to bind and dissociate the DNA-bound complex.

Thus, in some embodiments, the method comprises administering the test compound extracellularly in order to obtain a cell that comprises the test compound. For example, the test compound may be added to culture media that the cell is being cultured in. In embodiments where the test compound is administered extracellularly, an increase in expression of the reporter expression product indicates that the test compound is capable of entering the cell as well as being capable of inhibiting DNA-binding activity of the DNA-binding protein.

In some embodiments, the test compound is one that has previously been identified as being able to interact with the DNA-binding protein, or is suspected of being able to inhibit activity of the DNA-binding protein. For example,

the test compound may be suspected to be an inhibitor based on a PCA assay and the method described herein can then be used to confirm that the inhibitor is a functionally active antagonist of the DNA-binding protein. As described in the examples, a peptide ('FosW') identified using a PCA assay was then classed as a functionally active antagonist based on the method described herein.

The residues present on the surface of a protein that are responsible for PPIs are associated with protein secondary structure motifs, such as alpha-helix, beta-sheets and beta-turns. Of note, alpha-helices are thought to comprise approximately 60% of all secondary structures in protein complexes (Jochim and Arora, 2010). Additionally, alpha-helices have been shown to mediate a large number of key therapeutically relevant PPI interfaces, of which 60% bind to one face of the helix (Raj et al., 2013). Alpha-helices contain a hydrogen bond between the carbonyl group (C=O) of a given amino acid and the amino group (NH) of an amino acid three or four residues away.

Constraining peptides in a helical conformation has been reported to confer benefits that include enhances protease resistance, stability in cells, increases cellular uptake, enhanced biophysical properties and are anticipated to bind their targets with higher potency in comparison to wild-type peptide sequences (Azzarito et al. 2013). As a result, peptides that contain constrained alpha-helices (also termed "helix-constrained peptides") have been of great interest for identifying PPI inhibitors (Robertson and Spring, 2018).

Thus, in some embodiments, the peptidic test compound comprises a helix-constrained peptide.

The term "helix-constrained peptide" is intended to mean a peptide having at least one chemical modification that results in an intramolecular cross-link between two amino acids in order to produce a stabilised alpha-helix. Generally, the cross-link extends across the length of one or two helical turns (i.e. about 3-3.6 or about 7 amino acids). Accordingly, amino acids positioned at *i* and one of: *i+3*, *i+4*, and *i+7* are ideal candidates for cross-linking. Thus, for example, where a peptide has the sequence ... X1, X2, X3, X4, X5, X6, X7, X8, X9 ... , and the amino acid X is independently selected for each position, cross-links between X1 and X4, or between X1 and X5, or between X1 and X8 are useful as are cross-links between X2 and X5, or between X2 and X6, or between X2 and X9, etc. The use of multiple cross-links (e.g., 2, 3, 4 or more) is also contemplated.

Chemical modification includes a chemical modification to incorporate a molecular tether, such as a hydrocarbon staple, and a chemical modification to promote the formation of a disulphide bridge. The cross-link can be an ionic, covalent or hydrogen bond that links the two residues together, preferably the cross-link is a covalent bond.

The presence of a stabilised alpha-helix can be determined using methods such as circular dichroism spectroscopy for an alpha-helix, for example as described in Jo et al. (2012). Circular dichroism be used to measure a helicity increase, i.e. linear to cyclic. In situations where the cross-linking occurs through the formation of a disulphide bridge between two thiol groups, such as between two cysteine residues, the presence of a stabilised alpha-helix can also be determined using an assay that determining if thiols in the sample are free or conjugated. For example, free thiols can be assayed via reaction with Ellman's reagent (5,5'-dithiobis(2-nitrobenzoic acid; DNTB) (Sigma)) and monitoring absorbance at 412 nm.

Methods of inducing cross-links between amino acids are well known and include methods that induce cross-links between the peptide backbone, e.g. between the carbonyl group and amino group as in natural alpha-helices, as well as between side-chains of the peptides. Methods include disulphide bond formation (e.g. as described in Leduc et al. (2003)), hydrogen bond surrogates (e.g. as described in Wang et al. (2005)), ring-closing metathesis (e.g. as described in Walensky et al. (2004)), cysteine alkylation using $\alpha$-haloacetamide derivatives (e.g. as described in Woolley (2005)) or biaryl halides (e.g. as described in Muppidi et al. (2011)), lactam ring formation (e.g. as described in Fujimoto et al. (2008)), hydrazine linkage (e.g. as described in Cabezas & Satterthwait (1999)), oxime linkage (e.g. as described in Haney et al. (2011)), metal chelation (e.g. as described in Ruan et al. (1990)), and "click" chemistry (e.g. as described in Holland-Nell & Meldal (2011)).

In some embodiments, the cross-link is introduced between the amino acids in the peptidic test compound to produce a helix-constrained peptide prior to administering the test compound to the cell, e.g. administering the test compound extracellularly.

The present inventors have also made the surprising discovery that it is possible to introduce the intramolecular cross-link into the test compound intracellularly. Thus, a method where the peptidic test compound are cross-linked during the intracellular selection step could be used to directly screen for helix-constrained peptides within the cell. Since the helix-constrained peptide is present within the cell, the cells can immediately be used for subsequent screening for whether the test compound is able to disrupt PPIs and/or whether the test compound is a functionally-active antagonist using the screening method described herein. Furthermore, this method is applicable for polypeptides that contain the helix-constrained peptide, allowing the helix-constrained peptide to be screened to determine if it can disrupt PPIs in the context of the polypeptide.

Thus, in some embodiments of the screening method described herein where the test compound comprises a peptide, the method further comprises administering a cross-linking agent into the cell, wherein the cross-linking agent chemically modifies the peptide to introduce a cross-link between two amino acid residues to produce a stabilised alpha-helix, thereby producing the test compound comprising the helix-constrained peptide. The test compound may be expressed intracellularly from a test compound expression cassette.

In some embodiments, the cross-link is formed between amino acids at positions *i* and *i+3*, *i* and *i+4,* or *i* and *i*+7 in the amino acid sequence of the peptide. In some embodiments, the cross-link is between cysteine (C) residues located at these positions. In other embodiments, the cross-link is between lysine (K) and aspartic acid (D) residues at these positions. Preferably, the cross-link is formed between amino acids at positions *i* and *i+4.*

In some embodiments, the method comprises determining expression of the reporter expression product both before and after the addition of the cross-linking agent. In this way, it can be determined whether the peptide or polypeptide is able to as a functionally active antagonist both before and after cross-linking, therefore providing an indication of the functional effect that constraining the alpha-helix in the peptide is having.

In preferred embodiments, the peptide comprises a cysteine (C) at positions *i* and *i+4* in its amino acid sequence. As described in Jo et al. (2012), the introduction of cysteine residues at *i* and *i+4* positions is useful because this spacing brings two thioether residues into proximity when in the alpha-helix. Suitable cross-linking agents for stabilising the alpha-helix within the peptide containing a cysteine (C) at position *i* and *i+4* are described in Jo et al. (2012). For example, the cross-linking agent could be a cross-linker selected from the group consisting of an alkyl bromide, an alkyl iodide, a benzyl bromide, an allyl bromide, a maleimide, and an electrophilic difluorobenzene. In preferred embodiments, the cross-linking agent is an *m*-xylene based, *o*-xylene based, or *p*-xylene based benzyl bromide, more preferably a *m*-xylene based benzyl bromide. In particularly preferred embodiments, the cross-linking agent is 1,3-dibromomethylbenzene (DBMB) having the following chemical formula:

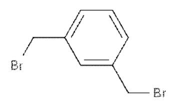

.

In some embodiments, the peptide comprises a lysine (K) and aspartic acid (D) at *i* and *i+4* positions in its amino acid sequence. That is, position *i* is a lysine (K) and position *i+4* is an aspartic acid (D), or position *i* is an aspartic acid (D) and position *i+4* is a lysine (K). Methods of carrying out K-D lactamisation are described, for example, in de Araujo et al. (2014).

The method may comprise adding the cross-linking agent at a pH of between 7.5 and 8.5, preferably a pH of 8.0. This can be achieved using various buffers, as is well understood in the art. The method may additionally comprise treating the cells with tris(2-carboxyethyl) phosphine (TCEP), which may help drive specific bi-alkylation. In particular exemplary methods, the DBMB cross-linking agent may be added to the test compound comprising a helix-constrained peptide with TCEP and ammonium bicarbonate, and reacted at pH 8.0 and room temperature for 4 to 5 hours in the dark.

*Cell-free method*

In another aspect, the present invention provides a cell-free method for screening for an antagonist of a DNA-binding protein, the method comprising:

i) contacting a test compound with a DNA-binding protein and a reporter expression cassette that encodes a reporter expression product,
wherein the reporter expression cassette comprises at least one binding site for the DNA-binding protein such that binding of the DNA-binding protein to the binding site inhibits expression of the reporter expression product; and

ii) determining expression of the reporter expression product;

wherein an increase in expression of the reporter expression product in the presence of the test compound indicates that the test compound is capable of inhibiting DNA-binding activity of the DNA-binding protein,
wherein the method is carried out outside a cell in an *in vitro* system that comprises the components required for expression of the reporter expression product, and
wherein some or all of the binding site(s) are located in the transcribed sequence of the reporter expression cassette.

Such methods are carried out using *in vitro* expression systems comprising the components required for expression of the reporter. Although described as "cell free", cells may be present. However, expression of the reporter does not take place within cells. Such methods are typically carried out *in vitro* and in the absence of cells. Such expression systems contain the components required to synthesise proteins from DNA *in vitro,* i.e. they contain enzymes including RNA polymerase, ribosomes, tRNAs, amino acids, initiation, elongation and termination factors, etc. and only require

the addition of template DNA. Commercially available *in vitro* transcription-translation kits can be used. An example of a commercially available *in vitro* transcription-translation kits is the PURExpress® *in vitro* Protein Synthesis Kit available from New England Biolabs (Catalogue number E6800).

In such cell-free methods, the reporter protein can be any protein that provides an observable phenotype, for example a fluorescent reporter protein or a protein that provides a colorimetric signal. Further details about suitable reporter proteins are described above.

Alternatively, the reporter protein could be DHFR and NADPH could be monitored in order to determine protein expression. DHFR is an enzyme that reduces dihydrofolic acid to tetrahydrofolic acid, using NADPH as electron donor, meaning that as tetrahydrofolic acid is produced NADPH is oxidised to NADP+. The oxidation of NADPH to NADP+ is accompanied by a decrease in absorbance at 340 nM (A340), which can be monitored by spectrophotometry. Thus, when the reporter protein is DHFR, an increase in protein expression can revealed by a decrease in absorbance at 340 nM *Antagonists, cells, kits and libraries*

In some embodiments, the methods for screening described herein further comprise isolating the test compound that has been indicated as a functionally active antagonist as a DNA-binding protein. As noted above, functionally active antagonists of DNA-binding proteins provided by the methods of the present invention may be useful in inhibiting the DNA-binding protein in a therapeutic setting. Thus, the functionally active antagonists may have utility in the treatment per se as pharmaceuticals, or may be valuable lead compounds for modification and improvement. Thus the aspects of the present disclosure described above may further comprise the step of formulating the agent identified by the screen with a pharmaceutically acceptable excipient. The pharmaceutical compositions may be formulated and intended for administration by any number of routes including, but not limited to, oral, intravenous, intramuscular, intra-articular, intra-arterial, intramedullary, intrathecal, intraventricular, transdermal, subcutaneous, intraperitoneal, intranasal, enteral, topical, sublingual, or rectal means.

The invention includes the combination of the aspects and preferred features described except where such a combination is clearly impermissible or expressly avoided.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

***Summary of the Figures***

Embodiments and experiments illustrating the principles of the invention will now be discussed with reference to the accompanying figures in which:

**Figure 1. Transcription-Block Survival (TBS) Assay to derive functionally active AP-1 inhibitors.** Schematic illustrating the principles of the TBS assay utilising mDHFR as a reporter expression cassette and TPA response elements (TREs) as DNA-binding sites. **A)** An mDHFR gene was generated that contains fifteen TREs introduced into its transcribed region, operably linked to a promoter (e.g. the lac operon).. **B)** Basic-cJun can form DNA-bound homodimers that bind to the TREs and prevent mDHFR transcription, inhibiting colony formation under selective conditions where mDHFR expression is necessary for cell survival. **C)** Peptides that bind to the coiled-coil region of basic-cJun (e.g. cFos) but do not dissociate the DNA-bound complex are not "functional antagonists" and will not rescue transcription of the mDHFR gene and therefore will not rescue colony formation under selective conditions. **D)** Expression of a functionally active inhibitor results in basic-cJun dissociating from TRE sites on the mDHFR gene leading to the restoration of mDHFR transcription-translation and colony formation.

**Figure 2. Luciferase reporter assay.**

AP-1 driven Luciferase gene reporter assay with no transfection (control), transfected dummy vector (dummy-helix), acidic c-Jun, acidic c-Fos and library-derived peptide (acidic-FosW) treated. The acidic AP-1 proteins reduce the AP-1 driven luciferase activity where the peptide inhibitor from **Fig 1D** has a dramatic reduction in the signal. The asterisk (*) indicates the results obtained when the peptide inhibitor from **Fig 1D** was used. This data suggests that peptides which can dissociate DNA-bound AP-1 proteins in the bacterial TBS assay results in a strong phenotypic effect in eukaryotic cells.

**Figure 3. Quantification of results from TBS assay using TRE DHFR.**

This figure provides quantification of colony formation in a TBS assay utilising the DHFR gene engineered to contain 15 TRE binding sites (TRE mDHFR) as a reporter and AP-1 as a DNA-binding protein. Bacterial cells transfected with Bacterial cells transfected with TRE mDHFR, the leucine zipper part of cJun (cJun LZ) and the leucine zipper part of cFos (cFos LZ) resulted in a large number (>300) of surviving bacterial colonies. Cells transfected with TRE mDHFR, cJun containing the basic DNA-binding domain (cJun bZIP) and cFos LZ resulted in a very low number of colonies (<20). This demonstrates that the basic DNA-binding domain of cJun is able to bind to the TRE sites in TRE mDHFR and inhibit expression of the DHFR protein. Cells transfected with TRE mDHFR, cJun bZIP and a peptide (Acidic FosW) that is able to dissociate the cJun bZIP protein from the TRE sites in TRE mDHFR results in substantial increase in bacterial colonies (>200).

## *Examples*

*EXAMPLE 1 - Development* of a *generalised approach to derive functionally active peptide inhibitors of transcription factor activity*

Many rational design approaches, randomised screening approaches, and selection systems result in the successful identification of compounds capable of binding to given protein targets. However, what is much more difficult to ensure, is that binding to said target will result in ablating target protein function. There are many instances where formation of a protein-protein interaction (PPI) has not ensured loss of function. To address this major bottleneck in antagonist screening and design, and to accelerate the design of functionally active antagonists, we have taken inspiration from the transcription factor DNA-binding system and reversed their role in transcription.

### *Introducing DNA-binding sites into the DHFR gene*

It can be difficult to predict whether a compound that is derived to bind to given protein target will antagonise its function. To tackle this we have taken the gene corresponding to the essential enzyme, dihydrofolate reductase (DHFR), and introduced 15 TPA response elements (TREs) into the gene. This has been achieved using a combination of both silent and conserved mutations, such that the activity of the enzyme is preserved.

All changes have been made in solvent exposed regions of the molecule to minimise the structural perturbations, with several proposed changes removed via close inspection of the accessible surface area (ASA) within the pdb file (PDBid = 2FZJ (Cody et al. (2006)). This was done by inputting the pdb file into the ASA calculator at http://cib.cf.ocha.ac.jp/bitool/ASA/. A cut-off value of 20 was used - residues that had an ASA value lower than this were considered to be buried and not modified; residues that had an ASA value greater than this are considered exposed.

No changes have been made in residues deemed important for catalysis or NADPH binding. Methods of identifying the solvent exposed regions of the reporter protein are known. For example, it is possible to take the coordinate files for the reporter protein, e.g. a protein databank (PDB) file and use a program that calculates the accessible surface area (ASA) which informs the user how exposed / buried residues are within a structure. An exemplary ASA program can be found at http://cib.cf.ocha.ac.jp/bitool/ASA/. An exemplary cut-off value of 20 can be used, such that residues that are lower than this are considered to be buried and greater than this are considered exposed. In this way, the locations of solvent exposed residues can be identified and codons modified accordingly.

Shown below is the sequence of the mDHFR gene **(SEQ ID NO: 11)** with DNA mutations bold and underlined and changes within the translated protein sequence **(SEQ ID NO: 31)** shown. Shown in bold italics are the NheI and HindIII sites used for subcloning the gene into the pES300d vector. Mutations were made by inspection of the desired consensus sequences (TGACTCA or TGAGTCA) and all three frames and the corresponding changes to the amino acid sequence upon making the necessary single base-pair changes. For example, either of the two desired sequences above can be put into any one of the three reading frames and the corresponding amino acid sequence and tolerated variations can be given:

| | | | | | |
|---|---|---|---|---|---|
| ***i)*** | Frame 1: | TGA CTC Axx | 1=stop | 2=LV | 3=I/M/T/N/K/S/R |
| ***ii)*** | Frame 2: | xTG ACT CAx | 1=LMV | 2=TS | 3=HQ |
| ***iii)*** | Frame 3: | xxT GAC TCA | 1=FSYCLPHRITNVADG | 2=DE | 3=S |

This gives rise to a number of codons to be identified for silent mutation and consequently a number of options for conserved or semi-conserved mutations that would permit the introduction of TREs into the mDHFR gene:

***i)*** No options
***ii)*** LSH, LSQ, LTH, LTQ, MSH, MSQ, MTH, MTQ, VSH, VSQ, VTH, VTQ
***iii)*** ADS, AES, CDS, CES, DDS, DES, FDS, FES, GDS, GES, HDS, HES, IDS, IES, LDS, LES, NDS, NES, PDS, PES, RDS, RES, SDS, SES, TDS, TES, VDS, VES, YDS, YES

From this we were able to implement the following changes into the mDHFR gene to give minimum perturbation to the overall sequence. Where possible mutations were silent or conservative. All mutations were also placed at solvent exposed sites and away from the catalytic centre (E116) and away from residues required for NADPH/substrate binding (A10/R71). This resulted in the introduction of 15 TREs into the mDHFR gene:

| | | | |
|---|---|---|---|
| 1. | VSQ | (silent) | = **GTG AGT CAG** |
| 2. | NEF→NES | (F32S) | = **AAT GAG TCA** |
| 3. | MTT→MTQ | (T40Q) | = **ATG ACT CAG** |
| 4. | TSS→TDS | (S42D) | = **ACT GAC TCA** |
| 5. | VEG→VES | (G46S) | = **GTT GAG TCA** |
| 6. | PEK→PES | (K64S) | = **CCT GAG TCA** |
| 7. | LSR→LSQ | (R78Q) | = **CTG AGT CAA** |
| 8. | IEQ→IES | (Q103S) | = **ATT GAG TCA** |
| 9. | VDM→VDS | (M112S) | = **GTT GAC TCA** |
| 10. | MNQ→MTQ | (N127T) | = **ATG ACT CAA** |
| 11. | VTR→VTQ | (R138Q) | = **GTG ACT CAG** |
| 12. | FES | (silent) | = **TTT GAG TCA** |
| 13. | IDL→IDS | (L154S) | = **ATT GAC TCA** |
| 14. | PEY→PES | (Y163S) | = **CCT GAG TCA** |
| 15. | LSE→LSQ | (E169Q) | = **CTG AGT CAG** |

This design process gave rise to the following sequence:

A S V R P L N C I V A V S Q N M G
***GCT AGC*** GTT CGA CCA TTG AAC TGC ATC GTC GCC ***GTG AGT CAG*** AAT ATG GGG
I G K N G D L P W P P L R N E S K
ATT GGC AAG AAC GGA GAC CTA CCC TGG CCT CCG CTC AGG ***AAT GAG TCA*** AAG
Y F Q R M T Q T D S V E S K Q N L
TAC TTC CAA AGA ***ATG ACT CAG ACT GAC TCA GTT GAG TCA*** AAA CAG AAT CTG
V I M G R K T W F S I P E S N R P
GTG ATT ATG GGT AGG AAA ACC TGG TTC TCC ATT ***CCT GAG TCA*** AAT CGA CCT
L K D R I N I V L S Q E L K E P P
TTA AAG GAC AGA ATT AAT ATA GTT ***CTG AGT CAA*** GAA CTC AAA GAA CCA CCA
R G A H F L A K S L D D A L R L I
CGA GGA GCT CAT TTT CTT GCC AAA AGT TTG GAT GAT GCC TTA AGA CTT ***ATT***
E S P E L A S K V D S V W I V G G
***GAG TCA*** CCG GAA TTG GCG AGC AAA ***GTT GAC TCA*** GTT TGG ATC GTC GGA GGC
S S V Y Q E A M T Q P G H L R L F
AGT TCT GTT TAC CAG GAA GCC ***ATG ACT CAA*** CCA GGC CAC CTT AGA CTC TTT
V T Q I M Q E F E S D T F F P E I
***GTG ACT CAG*** ATC ATG CAG GAA ***TTT GAG TCA*** GAC ACG TTT TTC CCA GAA ***ATT***
D S G K Y K L L P E S P G V L S Q
***GAC TCA*** GGG AAA TAT AAA CTT CTC ***CCT GAG TCA*** CCA GGC GTC ***CTG AGT CAG***
V Q E E K G I K Y K F E V Y E K K
GTC CAG GAG GAA AAA GGC ATC AAG TAT AAG TTT GAA GTC TAC GAG AAG AAA
D * A *
GAC T***AA GCT T***AA

We have introduced 15 TREs via silent and conserved mutations into solvent exposed positions within the gene coding for the essential enzyme dihydofolate reductase (DHFR; **Figure 1A).** We demonstrate that these changes result in a functional enzyme. Under selective conditions introduction of AP-1 prevents DHFR expression by binding to TRE sites within the gene, blocking transcription, and preventing colony formation under selective conditions. In contrast, attenuated versions of AP-1 that lack a basic DNA-binding region fail to prevent colony formation. Significantly, introducing peptides that can both bind AP-1 ***and*** antagonise function can be used to reverse this effect, leading to the formation of bacterial colonies. This represents a powerful approach for the selection of functionally active peptides that can bind to AP-1 components and abolish their function, leading to rapid library screening and identification of therapeutically interesting sequences.

***Testing functionality of DHFR protein***

The selection system is based on the fact that bacterial DHFR can be specifically inhibited using trimethoprim, rendering cells dependent upon murine DHFR (mDHFR) activity for their survival. The first test of the system was to establish that mDHFR protein refolds and is active. SDS-PAGE analysis was used to confirm that the protein is highly expressed upon addition of IPTG. Further evidence that the protein is expressed, folds, and is functionally active was verified by transformation of bacterial cells and confirmed by the presence of multiple colonies in minimal media containing trimethoprim (data not shown).

***Establishing the TBS assay***

It was next necessary to establish that introduction of an AP-1 component (in this case basic-cJun) would result in binding to the 15 TRE's introduced within the mDHFR gene and therefore failure of the gene to be transcribed.

Three plasmids were used for the TBS Assay. These are i) p300-mDHFR (Cm; **SEQ ID NO: 42**) to express the 15xconsensus sequence containing mDHFR, which is under control of the lac-operon; ii) p230d-basic-cJun (Amp; **SEQ ID NO: 43**) which is also under control of the lac-operon; iii) pREP4 (Kan; **SEQ ID NO: 44**) to express the lac repressor.

Cells were grown under non-selective conditions (i.e. LB/LB agar) containing Cm/Amp/Kan up until the time of the Assay. During TBS Selection Cells are grown in M9 minimal media (Agar or Broth) in the presence of Cm/Amp/Kan, as well as Tmp (to inhibit the bacterial copies of DHFR) and IPTG (to induce expression of mDHFR and bZIP proteins). During Assay selection, media-lacking ITPG is used to serve as a negative control to ensure that cell survival is exclusively driven by the loss on interaction between bZIP target protein and the consensus sequences located within the mDHFR gene. M9 agar plates with Tmp in the absence of IPTG do not form colonies (data not shown). Using IPTG to express mDHFR comprising the 15 TREs confers survival in M9 minimal medium containing Tmp to inhibit bacterial DHFR (data

not shown).

As expected, overexpression of basic-cJun on the second plasmid resulted in a complete loss of bacterial colonies in minimal media (data not shown). Without wishing to be bound by theory, it is believed that this works because AP-1 binds to the multiple TREs found within the mDHFR gene and therefore works in the opposite way to its natural function. Rather it works by blocking transcription and preventing the machinery from moving along the DNA **(Figure 1B).** As a control a version of cJun containing the leucine zipper, but lacking in the DNA-binding basic region (**SEQ ID NO: 45**), was tested. As expected this version did not prevent bacterial colony formation in minimal media (data not shown).

Lastly, we added compounds known to bind to the cJun coiled coil to establish if they are also functionally active peptides - i.e. capable of sequestering basic-cJun as a non-functional heterodimer and therefore prevent DNA-binding and rescue transcription. The c-Jun homodimer/DNA complex forms a very tight interaction, therefore a bonafide c-Jun inhibitor is required not only to bind to the c-Jun homodimer in solution but also to disassociate the DNA bound complex to prevent AP-1 transcriptional activity. Peptides which bind to the coiled coil domain (the dimerization motif of c-Jun homodimer) do not necessarily dissociate the DNA-bound complex **(Figure 1C).** Those that do not will therefore not rescue bacterial growth of TMP/IPTG treated cells (data not shown). However, peptides which lead to dissociation of the c-Jun homodimer from the DNA will enable the transcription of the mDHFR gene and rescue TMP/IPTG bacterial cells and grow. This is demonstrated by using the FosW peptide that is known to bind to cJun with high affinity ($K_D$= 90 nM; Mason et al. 2006). Expression of the FosW peptide resulted in the formation of colonies (data not shown), demonstrating that the FosW peptide was able to rescue transcription of the mDHFR gene and therefore is a functionally active antagonist **(Figure 1D).**

This assay allows for the selection of specific peptides capable not only of binding to the coiled coil region predicted to be required for driving AP-1 dimerization, but importantly that function by shutting down the DNA-binding activity of the protein. Thus the peptides must be functionally active to be selected and can be readily isolated from binders that are not of use by using cell survival as a marker for success.

***In vitro activity in mammalian cells***

Wild-type AP-1 proteins have a long stretch of basic amino acids at the N-terminal of the protein which enables DNA binding. Acidic versions of AP-1 proteins can be generated with a long stretch of negatively charged amino acids (in place of the positive) which can serve as a DNA mimic. Acidic AP-1 peptide variants can therefore form heterodimers with wild type AP-1 proteins driven by both the dimerization motif and the negative/positive charge interaction, resulting in their sequestration from the DNA.

In order to test whether the peptides identified as being functional antagonists in the TBS assay are also likely to have the same activity in eukaryotic cells, a AP-1 driven luciferase gene reporter assay was carried out. This assay was carried out using no transfection (control), transfected dummy vector, acidic c-Jun, acidic c-Fos and library-derived peptide treated (FosW). The results are shown in **Figure 2.** The acidic AP-1 proteins reduce the AP-1 driven luciferase activity where the peptide inhibitor from Example 1 (FosW) has a dramatic reduction in the signal. This data suggests that peptides which can dissociate DNA-bound AP-1 proteins in the bacterial TBS assay results in a strong phenotypic effect in eukaryotic cells.

***Discussion***

There are many assays to derive high affinity PPI's using library based approaches, but very few that guarantee loss of function within the target protein. Using AP-1 as our exemplar system, we have developed an assay to derive functionally active sequences capable of shutting down transcription factor activity. We have shown using the essential enzyme mDHFR that **i)** enzymatic activity is preserved upon introduction of 15 TREs into the gene ***ii)*** under selective conditions activity becomes lost when basic-cJun is introduced ***iii)*** the basic region within basic-cJun is an absolute requirement for this loss of mDHFR activity, and ***iv)*** peptides derived to bind to cJun can be separated into those that result in loss of AP-1 DNA binding activity (and therefore function) and those that do not. This assay therefore uses cell survival as a marker to allow rapid screening of peptide libraries and consequently the derivation of functionally active antagonists of transcription factor function.

*EXAMPLE 2 - Library creation*

***TBS Assay - Genetically Encoded Library Construction:***

As described above, three plasmids were used for the TBS Assay. These are i) p300-mDHFR (Cm) to express the 15xconsensus sequence containing mDHFR, which is under control of the lac-operon; ii) p230d-basic-cJun (Amp) which is also under control of the lac-operon; iii) pREP4 (Kan) to express the lac repressor.

Genetically encoded libraries are created using overlap extension PCR, subcloned into the p410d vector (Tet) and plated out. Each colony then represents a member of the library. We typically collect 2-5x the library size in colony numbers to gain approx. 95% total coverage. The maximum library size screenable using the approach is $10^6$. Once the library is complete colonies are pooled and mini-preparation of DNA performed. Finally the plasmid library is transformed into cells containing p300/p230/pREP4. During single step selection cells are plated onto LB agar (to demonstrate successful transformation), M9 agar lacking IPTG (as a negative control where no bZIP or mDHFR is expressed) and finally onto M9 agar containing Cm/Amp/Kan/Tet/Tmp/IPTG to drive production of bZIP/mDHFR/Library such that cell viability is only restored if a given library member can prevent the bZIP target from interacting with the cognate sequences within the mDHFR gene. Surviving colonies can next be pooled, grown and serially diluted in liquid cultures under selective conditions (M9 minimal medium with 1 μg/ml trimethoprim). Fastest growth, and hence the highest affinity interacting partners dominated the pool. Library pools as well as colonies from individual clones were sequenced to verify the arrival at one sequence. To assess library quality we sequence pools and single clones to find approximately equal distributions of varied amino acids. Pooled colonies exceeded the library size 5-10 fold. Using more recent ligation methods (Topo/Gibson/Gateway) it may be possible to move into TBS directly from ligation, giving the significant advantage of being able to screen larger libraries (possibly up to $10^{10}$ or $10^{11}$), however processes will need to be put into place (e.g. next gen sequencing) to ensure that library size and quality is fully represented prior to transformation into the TBS assay.

Another possibility is to use pET24a as an alternative to the pREP4 vector used to express the lac repressor. This would allow the expression of both the lac repressor and library member / antagonist off a single plasmid, i.e. avoiding the need for another antibiotic.

***TBS Assay - Extracellular compound addition:***

For extracellular libraries, cells containing p300-mDHFR plasmid are grown in the presence of p230d-bZIP and pREP4 plasmids under non-selective conditions (LB agar/media). Once ready for assay overnights can then be placed into each well of microtitre plates (96, 384, 1536) at $A_{600}$ = 0.05 and compound libraries screened by direct addition to each well. Plates are incubated at 37°C and with shaking and successful compounds identified by monitoring of the absorbance signal at 600 nm. This ***extracellular compound addition*** method has the advantage of allowing the user to move away from standard peptide libraries (e.g. one can profile for helix constrained peptides, peptidomimetics, non-natural amino acids etc, or even small molecule libraries) and importantly allows the user to profile for cell penetrance concomitantly with functional antagonism of the bZIP target. Once again, all proteins are under control of a lac promoter, and expression was induced with Isopropyl β-D-1-thiogalactopyranoside (IPTG).

***Selection of winner peptides***

Briefly, during TBS only peptides (intracellular) or compounds (extracellular) that can interact with the bZIP target and dissociate it from the consensus sequences within the mDHFR gene will result in colony formation / cell growth on M9 minimal medium plates / media with 1 μg/ml trimethoprim to inhibit bacterial DHFR.

*EXAMPLE 3 - Further TBS assay experiment*

A further experiment was carried out to establish the TBS assay as a suitable assay for identifying functional antagonists.

This experiment used similar plasmids to those set out in Example 1, with the exception that pET24a was used to express both the lac repressor and test compound using a single plasmid. Specifically, three plasmids were used: i) p300d expressing the mDHFR gene modified to include 15 TREs (TRE mDHFR, generated as described in Example 1); ii) p230d expressing either the leucine zipper part of cJun (cJun LZ) or cJun with the basic region and leucine zipper (cJun bZip); iii) pET24a expressing the lac repressor and either the leucine zipper part of cFos (cFos LZ) or the acidic FosW peptide (acidic FosW).

Plates were generated as set out in the following table:

| Plate | p300d | p230d | pET24a |
|---|---|---|---|
| 1 | TRE mDHFR | cJun LZ | cFos LZ |
| 2 | TRE mDHFR | cJun bZIP | cFos LZ |
| 3 | TRE mDHFR | cJun bZIP | Acidic FosW |

The number of colonies produced in each plate were counted and this quantification is illustrated in **Figure 3**. The cJun LZ expressed in control plate 1 is unable to bind and inhibit expression of TRE DHFR, therefore resulting in a large number (>300) bacterial colonies. cJun with the basic DNA-binding domain (cJun bZip) is able to bind and inhibit expression of DHFR and therefore when expressed in plate 2 resulted in a substantial reduction in the number of bacterial colonies (<20) compared to plate 1. Co-expression with a peptide that binds to the coiled-coil region of cJun (cFos LZ) does not dissociate the DNA-bound complex and therefore is not able to rescue expression of the DHFR protein in the TBS assay. However, co-expression with a peptide (acidic FosW) that is able to dissociate the DNA-bound AP-1 transcription factor from its binding sites in the TRE DHFR gene results in a substantial increase in the number of colonies compared to plate 2

Overall, these results provide further evidence that the TBS assay can be used to identify antagonists that are able to inhibit DNA-binding activity of a transcription factor such as AP-1.

*EXAMPLE 4 - Introducing the CRE, CCAA T and Ebox binding sites into the DHFR gene*

Constructs were designed whereby the CRE, CCAAT and Ebox binding sites, respectively, were inserted into the DHFR gene. These constructs can be tested in the TBS assay as described in Examples 1 and 3.

***Inserting the CRE binding site into the DHFR gene***

CRE is usually defined as **TGACGTCA** (SEQ ID NO: 10). Mutations in the DHFR gene can be made by inspection of the desired consensus sequence and all three frames and the corresponding changes to the amino acid sequence upon making the necessary single base-pair changes. The CRE is 8 bp as so can span four codons. For example, the sequence defined above can be put into any one of the three reading frames and the corresponding amino acid sequence and tolerated variations can be given:

| | | | | | |
|---|---|---|---|---|---|
| A:Frame 1: | TGA CGT CAx | 1=stop | 2:R | 3:H/Q | |
| B:Frame 2: | xTG ACG TCA | 1:LMV | 2:T | 3:S | |
| C:Frame 3: | xxT GAC GTC Axx | 1:FLIVSPTAYHNDCRG | 2:D | 3:V | 4:IMTNKSR |

From this it is possible to implement changes into the mDHFR gene to give minimal perturbation to the overall sequence. Mutations should be placed at solvent exposed sites and away from the catalytic centre and where possible mutations should be silent or conservative.

An example of an mDHFR gene that is modified to contain CRE binding sites is shown as follows:

ATGGTTCGACCATTGAACTGCATCGTCGCCGTGTCCCAAAATATGGGGATTGGCAAGAACGGAGACCTAC
CCTGGCCTCCGCTCAGGAACGAGTTCAAGTACTTCCAAAGAA**TGACGTCA**ACCTCTTCAGTGGAAGGTAA
ACAGAATCTGGTGATTATGGGTAGGAAAACCTGGTTCTCCATTCCTGAGAAGAATCGACCTTTAAAGGAC
AGAATTAATATAG**TGACGTCA**AGAGAACTCAAAGAACCACCACGAGGAGCTCATTTTCTTGCCAAAAGTT
TGGATGATGCCTTAAGACTTATTGAACAACCGGAAT**TGACGTCA**AAAGTAGACATGGTTTGGATCGTCGG
AGGCAGTTCTGTTTACCAGGAAGCCATGAATCAACCAGGCCACCTTAGACTCTTTG**TGACGTCA**ATCATG
CAGGAATTTGAAAGTGACACGTTTTTCCCAGAAATTGATTTGGGGAAATATAAACTTCTCCCAGAATACC
CAGGCG**TGACGTCA**GAGGTCCAGGAGGAAAAAGGCATCAAGTATAAGTTTGAAGTCTACGAGAAGAAAGA
CTAAGCTTAA

Nucleotide residues in **bold underline** indicate consensus CRE binding sites.

Nucleotide residues in lowercase and italics correspond to the restriction enzyme sites for Ascl and HindIll at the 5' and 3' ends of the sequence, respectively.

The resulting amino acid sequence is shows as follows:

| | | | | | | | | | | | | | | | | | | | |
|---|---|---|---|---|---|---|---|---|---|---|---|---|---|---|---|---|---|---|---|
| *M* | *V* | *R* | P | L | N | C | I | V | A | V | S | *Q* | *N* | *M* | G | *I* | G | *K* | *N* |
| *G* | *D* | *L* | *P* | W | *P* | *P* | L | *R* | *N* | E | *F* | *K* | Y | *F* | *Q* | *R* | *M* | T | ***S*** |
| *T* | *S* | *S* | *V* | *E* | *G* | *K* | *Q* | N | L | V | I | M | G | *R* | *K* | *T* | W | *F* | *S* |
| *I* | *P* | *E* | *K* | *N* | *R* | *P* | L | *K* | *D* | R | I | N | I | V | ***T*** | S | *R* | *E* | *L* |
| *K* | *E* | *P* | P | *R* | *G* | A | *H* | *F* | *L* | A | *K* | *S* | L | *D* | *D* | A | *L* | *R* | *L* |
| I | *E* | *Q* | *P* | *E* | L | ***T*** | *S* | *K* | V | *D* | *M* | V | W | I | V | G | *G* | S | *S* |
| V | Y | *Q* | *E* | A | M | *N* | *Q* | *P* | G | *H* | L | *R* | L | F | V | T | ***S*** | *I* | *M* |
| *Q* | *E* | F | *E* | S | *D* | *T* | *F* | F | *P* | *E* | *I* | *D* | *L* | *G* | *K* | Y | *K* | *L* | *L* |
| *P* | *E* | *Y* | *P* | *G* | *V* | ***T*** | *S* | *E* | *V* | *Q* | *E* | *E* | *K* | *G* | I | *K* | Y | *K* | *F* |
| *E* | V | Y | *E* | K | *K* | *D* | | | | | | | | | | | | | |

Amino acid residues in *italics* are solvent exposed residues. The other residues are classed as buried residues.

Amino acid residues in **bold underline** are residues that have been altered as a result of the insertion of CRE into the nucleotide sequence.

A summary of the amino acid changes is provided as follows:

| | | | | |
|---|---|---|---|---|
| 1. | MTT→MTS | (T40S) | = **ATG ACG TCA** | ASA at posn = 36 |
| 2. | VLS→VTS | (L76T) | = **GTG ACG TCA** | ASA at posn = 21 |
| 3. | LAS→LTS | (A107T) | = **TTG ACG TCA** | ASA at posn = 37 |
| 4. | VTR→VTS | (R138S) | = **GTG ACG TCA** | ASA at posn = 57 |
| 5. | VLS→VTS | (L167T) | = **GTG ACG TCA** | ASA at posn = 99 |

***Inserting the CCAAT binding site into the DHFR gene***

CCAAT is usually defined as **ATTGCGCAAT** (SEQ ID NO: 9). Mutations in the DHFR gene can be made by inspection of the desired consensus sequence and all three frames and the corresponding changes to the amino acid sequence upon making the necessary single base-pair changes. The CCAAT is 10 bp and so can span five codons. For example, the sequence defined above can be put into any one of the three reading frames and the corresponding amino acid sequence and tolerated variations can be given:

| | | | | | |
|---|---|---|---|---|---|
| A:Frame 1: | ATT GCG CAA Txx | 1:1 | 2:A | 3:Q | 4:FLSYCW* |
| B:Frame 2: | xAT TGC GCA ATx | 1:YHND | 2:C | 3:A | 4:IM |
| C:Frame 3: | xxA TTG CGC AAT | 1:LIVSPTAQKERG* | 2:L | 3:R | 4:N |

From this it is possible to implement changes into the mDHFR gene to give minimal perturbation to the overall sequence. Mutations should be placed at solvent exposed sites and away from the catalytic centre and where possible mutations should silent or conservative.

An example of an mDHFR gene that is modified to contain CCAAT is shown as follows:

ATGGTTCGACCATTGAACTGCATCGTCGCCGTGTCCCAAAATATGGGGATTGGCAAGAACGGAGACCTAC

CCTGGCCTCC**ATTGCGCAAT**GAGTTCAAGTACTTCCAAAGAATGACCACAACCTCTTCAGTGGAAGGTAA

ACAGAATCTGGTGATTATGGGTAGGAAAACCTGGTTCTCCATTCCTGAGAAGAATCGACC**ATTGCGCAAT**AGAATTAATATAGTTCTCAGTAGAGA**ATTGCGCAAT**CCACCACGAGGAGCTCATTTT**ATTGCGCAAT**CCTTGGATGATGC**ATTGCGCAAT**ATTGAACAACCGGAATTGGCGAGCAAAGTAGACATGGTTTGGATCGTCGGAGGCAGTTCTGTTTACCAGGAAGCCATGAATCAACCAGGCCACCTTAGACTCTTTGTGACAAGGATCATGCAGGAATTTGAAAGTGACACGTTTTTCCCAGAAATTGATTTGGGGAAATATAAACTTCTCCCAGAATACCCAGGCGTCCTCTCTGA**ATTGCGCAAT**GAAAAAGGCATCAAGTATAAGTTTGAAGTCTACGAGAAGAAAGACTAAGCTTAA

Nucleotide residues in **bold underline** indicate CCAAT consensus binding sites.

Nucleotide residues in lowercase and italics correspond to the restriction enzyme sites for Ascl and Hindlll at the 5' and 3' ends of the sequence, respectively.

The resulting amino acid sequence is shows as follows:

| | | | | | | | | | | | | | | | | | | | |
|---|---|---|---|---|---|---|---|---|---|---|---|---|---|---|---|---|---|---|---|
| *M* | *V* | *R* | P | L | N | C | I | V | A | V | S | *Q* | *N* | *M* | G | *I* | G | *K* | *N* |
| *G* | *D* | *L* | *P* | W | *P* | *P* | L | *R* | *N* | E | *F* | *K* | Y | *F* | *Q* | *R* | *M* | T | *T* |
| *T* | *S* | *S* | *V* | *E* | *G* | *K* | *Q* | N | L | V | I | M | G | *R* | *K* | *T* | W | *F* | *S* |
| *I* | *P* | *E* | *K* | *N* | *R* | *P* | L | ***R*** | ***N*** | R | I | N | I | V | *L* | S | *R* | *E* | *L* |
| ***R*** | ***N*** | *P* | P | *R* | *G* | A | *H* | *F* | ***I*** | A | ***Q*** | *S* | L | *D* | *D* | A | *L* | *R* | ***N*** |
| I | *E* | *Q* | *P* | *E* | L | *A* | *S* | *K* | V | *D* | *M* | V | W | I | V | G | *G* | S | *S* |
| V | Y | *Q* | *E* | A | M | *N* | *Q* | *P* | G | *H* | L | *R* | L | F | V | T | *R* | *I* | *M* |
| *Q* | *E* | F | *E* | S | *D* | *T* | *F* | F | *P* | *E* | *I* | *D* | *L* | *G* | *K* | Y | *K* | *L* | *L* |
| *P* | *E* | *Y* | *P* | *G* | *V* | *L* | *S* | *E* | *V* | *Q* | *E* | *E* | *K* | *G* | I | *K* | Y | *K* | *F* |
| *E* | V | Y | *E* | K | *K* | *D* | | | | | | | | | | | | | |

Amino acid residues in *italics* are solvent exposed residues. The other residues are classed as buried residues.

Amino acid residues in **bold underline** are residues that have been altered as a result of the insertion of CCAAT into the nucleotide sequence.

A summary of the amino acid changes is provided as follows:

1. PLRN (silent)
2. PLKD→PLRN (K69R, D70N) = ASA at posns = 90, 97
3. ELKE→ELRN (K81R, E82N) = ASA at posns = 175,131
4. LAKS→IAQS (L90I, K92Q) = ASA at posns = 47,139
5. ALRL→ALRN (L100N) = ASA at posn = 46

A further CCAAT site could be inserted to make the following mutation:

6. EVQE→ELRN (V170L, Q171R, E172N)

***Inserting the Ebox binding site into the DHFR gene***

In the context of c-Myc, Ebox is usually defined as CACGTG (SEQ ID NO: 7) or CACATG (SEQ ID NO: 8). Mutations in the DHFR gene can be made by inspection of the desired consensus sequence and all three frames and the corresponding changes to the amino acid sequence upon making the necessary single base-pair changes. For example, the sequences defined above can be put into any one of the three reading frames and the corresponding amino acid sequence and tolerated variations can be given:

A:Frame 1: CAC GTG xxx = 1:H 2:V 3:Anything

(continued)

| | | | | |
|---|---|---|---|---|
| B:Frame 2: | xCA CGT Gxx = | 1:S/P/T/A | 2:R | 3:V/A/D/E/G |
| C:Frame 3: | xxC ACG TGx = | 1:FLIVSPTAYHNDCRSG | 2:T | 3:C/W/* |

| | | | | |
|---|---|---|---|---|
| A:Frame 1: | CAC ATG xxx = | 1:H | 2:M | 3:anything |
| B:Frame 2: | xCA CAT Gxx = | 1:S/P/T/A | 2:H | 3:V/A/D/E/G |
| C:Frame 3: | xxC ACA TGx = | 1:FLIVSPTAYHNDCRSG | 2:T | 3:C/W/* |

From this it is possible to implement changes into the mDHFR gene to give minimal perturbation to the overall sequence. Mutations should be placed at solvent exposed sites and away from the catalytic centre and where possible mutations should silent or conservative.

An example of an mDHFR gene that is modified to contain Eboxes is shown as follows:

ATGGTTCGACCATTGAACTGCATCGTCGCCGTGTCCCAAAATATGGGGATTGGCAAGAACGGAGACCTAC
CCTGGCCTCCGCTCAGGAACGAGTTCAAGTACTTCCAAAGAATGACCACAACCTCTTCAGTGGAAGGTAA
ACAGAATCTGGTGATTATGGGTAGGCG**CACGTG**GTTCTCCATTCCTGAGAAGAATCGACCTTTAAAGGAC
AGAATTAATATAGTTCTCT**CACGTG**AACTCAAAGAACCAC**CACGTG**GAGCT**CACGTG**CTTGCCAAATCAC
TGGATGATGCATTAAGACTTATTGAACAACCGGAATTGGCGT**CACGTG**TAGACATGGTTTGGATCGTCGG
AGGCAGTTCTGTTTACCAGGAAGCCATGAATCAACCAGGC**CACGTG**AGACTCTTTGTGA**CACGTG**TCATG
CAGGAATTTGAAAGTGACACGTTTTTCCCAGAAATTGATTTGGGGAAATATAAACTTCTCCCAGAATACC
CAGGCGTCCTCT**CACGTG**TCCAGGAGGAAAAAGGCATCAAGTATAAGTTTGAAGTCTACGAGAAGAAAGA
CTAAGCTTAA

Nucleotide residues in **bold underline** indicate Ebox consensus binding sites.

Nucleotide residues in lowercase and italics correspond to the restriction enzyme sites for Ascl and Hindlll at the 5' and 3' ends of the sequence, respectively.

The resulting amino acid sequence is shows as follows:

| | | | | | | | | | | | | | | | | | | | |
|---|---|---|---|---|---|---|---|---|---|---|---|---|---|---|---|---|---|---|---|
| *M* | *V* | *R* | P | L | N | C | I | V | A | V | S | *Q* | *N* | *M* | G | *I* | G | *K* | *N* |
| *G* | *D* | *L* | *P* | W | *P* | *P* | L | *R* | *N* | E | *F* | *K* | Y | *F* | *Q* | *R* | *M* | T | *T* |
| *T* | *S* | *S* | *V* | *E* | *G* | *K* | *Q* | N | L | V | I | M | G | *R* | ***R*** | *T* | W | *F* | *S* |
| *I* | *P* | *E* | *K* | *N* | *R* | *P* | L | *K* | *D* | R | I | N | I | V | *L* | S | *R* | *E* | *L* |
| *K* | *E* | *P* | P | *R* | *G* | A | *H* | ***V*** | *L* | A | *K* | *S* | L | *D* | *D* | A | *L* | *R* | *L* |
| I | *E* | *Q* | *P* | *E* | L | *A* | *S* | ***R*** | V | *D* | *M* | V | W | I | V | G | *G* | S | *S* |
| V | Y | *Q* | *E* | A | M | *N* | *Q* | *P* | G | *H* | **V** | *R* | L | F | V | T | *R* | **V** | *M* |
| *Q* | *E* | F | *E* | S | *D* | *T* | *F* | F | *P* | *E* | *I* | *D* | *L* | *G* | *K* | Y | *K* | *L* | *L* |
| *P* | *E* | *Y* | *P* | *G* | *V* | *L* | *S* | ***R*** | *V* | *Q* | *E* | *E* | *K* | *G* | I | *K* | Y | *K* | *F* |
| *E* | V | Y | *E* | K | *K* | *D* | | | | | | | | | | | | | |

Amino acid residues in *italics* are solvent exposed residues. The other residues are classed as buried residues.

Amino acid residues in **bold underline** are residues that have been altered as a result of the insertion of Ebox into the nucleotide sequence.

A summary of the amino acid changes is provided as follows:

| | | | | | |
|---|---|---|---|---|---|
| 1. | KTW→RTW | (K56R) | = CG**C ACG TG**G | ASA at posn = 143 | (exposed) |
| 2. | SRE | (silent) | = T**CA CGT G**AA | ASA at posn = N/A | |

(continued)

| | | | | | |
|---|---|---|---|---|---|
| 3. PRG | (silent) | = C**CA CGT G**GA | ASA at posn = N/A | |
| 4. HFL→HVL | (F89V) | = **CAC GTG** CTT | ASA at posn = 71 | (exposed) |
| 5. SKV→SRV | (K109R) | = A**CA CGT G**TA | ASA at posn = 109 | (exposed) |
| 6. HLR→HVR | (L132V) | = **CAC GTG** AGA | ASA at posn = 1.6 | |
| 7. TRI→TRV | (I139V) | = A**CA CGT G**TC | ASA at posn = 1.6 | |
| 8. SEV→SRV | (E151R) | = A**CA CGT G**TC | ASA at posn = 141 | (exposed) |

Changes 6 and 7 are located at residues that are classed as buried. Accordingly, constructs could be made that contain all 8 Ebox sites, one that is lacking site '6', one that is lacking site '7' and one that is lacking both sites '6' and '7' in order to determine whether the mutation at these 'buried' sites affect the function of the resultant DHFR protein.

*EXAMPLE 5 - Expanding the TBS assay for use with additional transcription factor targets*

Experiments were carried out to establish the TBS assay for use in identifying functional inhibitors of transcription factors other than AP-1. The following modified DHFR genes were generated:

| mDHFR TBS mutant | Nucleotide sequence of binding site | Total number of binding sites | Nucleotide sequence of mDHFR mutant: | Notes |
|---|---|---|---|---|
| TRE mDHFR | TGA(C/G)TCA | 15 | SEQ ID NO: 4 | See Example 1 for construction details |
| CCAAT mDHFR | ATTGCGCAAT | 6 | SEQ ID NO: 38 | See Example 4 for construction details |
| EBOX mDHFR | CAC(G/A)TG | 8 | SEQ ID NO: 40 | See Example 4 for construction details |
| CRE mDHFR | TGACGTCA | 5 | SEQ ID NO: 36 | See Example 4 for construction details |

As set out in Example 1, expression of mDHFR TRE was able to restore bacterial colonies in the presence of TMP, indicating that the protein produced by this mDHFR mutant was functional. Similar experiments were carried out to determine if the other mDHFR mutants (mDHFR CCAAT, mDHFR EBOX, mDHFR CRE) were also active. These experiments revealed that expression of the mDHFR mutant in the presence of TMP resulted in an increased number of colonies compared to plates where TMP was present without the mDHFR mutant being expressed (data not shown). These experiments confirm that the DHFR proteins produced by the mDHFR mutants were functional and able to confer survival in the presence of TMP.

Experiments were also carried out to determine whether the TBS assay could be used to identify functional antagonists of C/EBP alpha and BZLF1. C/EBP alpha is a bZip transcription factor that binds CCAAT sites and upregulation in human cells is associated with colorectal cancer growth, metastasis and indicates poor survival outcome. BZLF1 is a bZip transcription factor that binds TRE and CCAAT sites and is associated with Burkitt's lymphoma, Hodgkin's disease, non-Hodgkin's lymphoma, nasopharyngeal carcinoma and lymphomas.

For C/EBP alpha, p230d plasmids were generated that encoded either C/EBP alpha with the DNA-binding basic region (C/EBPa incl. basic region) or without the basic region (C/EBPa minus basic region). Cells were then plated with p230d expressing either of these C/EBP alpha constructs together with p300d expressing mDHFR CCAAT and an empty pET24a plasmid. The experiment was carried out using 8 μM TMP and in the absence and presence of 1 mM IPTG (to induce expression of the C/EBP alpha and DHFR proteins).

Cells were first transfected with CCAAT mDHFR, a plasmid encoding C/EBPa incl. basic region, and an empty pET24a plasmid in the presence of 8 μM trimethoprim (TMP) but without IPTG. TMP inhibits bacterial DHFR, resulting in no bacterial colonies (data not shown). Addition of 1 mM IPTG induces transcription of mDHFR and C/EBPa proteins and resulted in a very low number of bacterial colonies (approx. 8; data not shown) being observed. This result suggests that the DNA-binding domain of C/EBPa was able to bind to the CCAAT binding sites and inhibit expression of mDHFR, as expected based on previous results using the TBS assay (see Example 1 and 3). Expression of mDHFR and C/EBPa minus basic region results in a large number (>100) of bacterial colonies. This suggests that the absence of a DNA-binding domain prevents the C/EBPa protein from binding the CCAAT sites in CCAAT mDHFR, permitting expression of the mDHFR protein and allowing bacterial cell survival.

The experiment revealed that co-expression of mDHFR CCAAT with the C/EBP alpha protein that contains the basic region resulted in substantially fewer colonies compared to the plate containing cells that express mDHFR CCAAT and C/EBP alpha lacking the basic region. This result indicates that the DNA-binding basic region of the C/EBP alpha protein was able to bind the CCAAT binding sites in mDHFR CCAAT and inhibit production of mDHFR, suggesting that these constructs could be used in the TBS assay to identify compounds that are capable of inhibiting DNA-binding activity of the C/EBP alpha protein.

A similar experiment was carried out to assess whether the constructs described above could be used to identify compounds capable of inhibiting the DNA-binding activity of BZLF1. p230d plasmids were generated that encoded either BZLF1 with the DNA-binding basic region (BZLF1 incl. basic region) or without the basic region (BZLF1 minus basic region). Cells were then plated with p230d expressing either of these BZLF1 constructs together with p300d expressing mDHFR TRE and an empty pET24a plasmid. The experiment was carried out using 8 $\mu$M TMP and in the absence and presence of 1mM IPTG (to induce expression of the BZLF1 and DHFR proteins).

The results demonstrated that co-expression of mDHFR TRE with the BZLF1 protein that contains the basic region resulted in substantially fewer colonies compared to the plate containing mDHFR TRE and BZLF1 lacking the basic region (data not shown). This result indicates that the DNA-binding basic region of the BZLF1 protein was able to bind the TRE binding sites in mDHFR TRE and inhibit production of mDHFR, suggesting that these constructs could be used in the TBS assay to identify compounds that are capable of inhibiting DNA-binding activity of the BZLF1 protein.

In summary, these results provide evidence that the TBS assay can be used to identify compounds capable of inhibiting DNA-binding activity of the transcription factors other than AP-1, such as the C/EBP alpha and BZLF1 transcription factors. This further demonstrates that the TBS assay can be utilised as a general method for identifying functional antagonists of DNA-binding proteins.

***References***

A number of publications are cited above in order to more fully describe and disclose the invention and the state of the art to which the invention pertains. Full citations for these references are provided below.


Cabezas, E.; Satterthwait, A. C. (1999) J. Am. Chem. Soc., 121, 3862.

Cody, V. et al. (2006) New insights into DHFR interactions: Analysis of Pneumocystis carinii and mouse DHFR complexes with NADPH and two highly potent 5-(omega-carboxy(alkyloxy) trimethoprim derivatives reveals conformational correlations with activity and novel parallel ring stacking interactions. Proteins 65(4): 959-969

de Araujo A.D. et al. (2014) Comparative $\alpha$-helicity of cyclic pentapeptides in water. Angew Chem Int Ed Engl. 53(27):6965-9

Fujimoto, K. et al. (2008) Development of a series of cross-linking agents that effectively stabilize alpha-helical structures in various short peptides. Chemistry 14(3):857-63.

Gritz L & Davies J. (1983) Plasmid-encoded hygromycin B resistance: the sequence of hygromycin B phosphotransferase gene and its expression in Escherichia coli and Saccharomyces cerevisiae. Gene. 25(2-3):179-88.

Haney, C.M. et al. (2011) Promoting peptide $\alpha$-helix formation with dynamic covalent oxime side-chain cross-links. Chem Commun (Camb).47(39):10915-7.

Holland-Nell, K.; Meldal, M. Maintaining biological activity by using triazoles as disulfide bond mimetics. Angew Chem Int Ed Engl. 50(22):5204-6.

Heinis, C. et al. (2009) Phage-encoded combinatorial chemical libraries based on bicyclic peptides. Nat Chem Biol. 5(7): 502-507

Jo, H. et al. (2012) Development of $\alpha$-helical calpain probes by mimicking a natural protein-protein interaction. J Am Chem Soc. 134(42):17704-17713

Jochim, A.L.; Arora, P.S. (2010) Systematic analysis of helical protein interfaces reveals targets for synthetic inhibitors. ACS Chem. Biol., 5, 919-923.

Kaufman et al. (1986) Selection and amplification of heterologous genes encoding adenosine deaminase in mammalian cells. Proc Natl Acad Sci USA. 83(10): 3136-3140.

Leduc, A.M. et al. (2003) Helix-stabilized cyclic peptides as selective inhibitors of steroid receptor-coactivator interactions. Proc Natl Acad Sci USA. 100(20):11273-8

Ma. Y. et al (2014) Split focal adhesion kinase for probing protein-protein interactions. Biochemical Engineering Journal. 90: 272-278

Mason, J. et al. (2006) Semirational design of Jun-Fos coiled coils with increased affinity: Universal implications for leucine zipper prediction and design. Proc Natl Acad Sci USA. 103(24): 8989-94.

McCormick, F. (2015) KRAS as a Therapeutic Target. Clin Cancer Res. 21(8): 1797-1801.

Mern, D.S. et al. (2010) Inhibition of Id proteins by a peptide aptamer induces cell-cycle arrest and apoptosis in ovarian cancer cells. Br J Cancer. 103(8): 1237-1244.

Miranda, E. et al. (2011) Deciphering interactions used by the influenza virus NS1 protein to silence the host antiviral sensor protein RIG-I using a bacterial reverse two-hybrid system. Mol. BioSyst., 7, 1042-1045

Muppidi, A. et al. (2011) Achieving cell penetration with distance-matching cysteine cross-linkers: a facile route to cell-permeable peptide dual inhibitors of Mdm2/Mdmx. Chem Commun (Camb). 47(33):9396-8. Newman & Keating (2003) Comprehensive identification of human bZIP interactions with coiled-coil arrays. Science. 300(5628):2097-101

Olive, M. et al. (1997) A Dominant Negative to Activation Protein-1 (AP1) That Abolishes DNA Binding and Inhibits Oncogenesis. J Biol Chem. 272(30): 18586-94.

Park, J.H. (2007) Bacterial beta-lactamase fragmentation complementation strategy can be used as a method for identifying interacting protein pairs. Journal of Microbiology and Biotechnology. 17 (10): 1607-15.

Pelay-Gimeno, M. et al. (2015) Structure-Based Design of Inhibitors of Protein-Protein Interactions: Mimicking Peptide Binding Epitopes. Angew Chem Int Ed Engl. 54(31):8896-927

Pelletier J.N., Campbell-Valois F.X., Michnick S.W. (1998) Oligomerization domain-directed reassembly of active dihydrofolate reductase from rationally designed fragments. Proc Natl Acad Sci U S A. 95(21): 12141-6.

Soucek, L. et al. (2013) Inhibition of Myc family proteins eradicates KRas-driven lung cancer in mice. Genes. Dev. 27(5): 504-13.

Raj, M. et al. (2013) Plucking the high hanging fruit: A systematic approach for targeting protein-protein interactions. Bioorg. Med. Chem. 21, 4051-4057.

Remy, I. et al. (2007) Detection of protein-protein interactions using a simple survival protein-fragment complementation assay based on the enzyme dihydrofolate reductase. Nat Protoc. 2(9): 2120-5. Robertson and Spring (2018) Using Peptidomimetics and Constrained Peptides as Valuable Tools for Inhibiting Protein-Protein Interactions. Molecules 23(4): 959.

Rodriguez-Martinez et al. (2017). Combinatorial bZIP dimers display complex DNA-binding specificity landscapes. Elife. 6 e19272

Ruan, F. et al. (1990) Metal ion-enhanced helicity in synthetic peptides containing unnatural, metal-ligating residues J. Am. Chem. Soc., 112 (25): 9403-9404

Vaquerizas, J.M. et al. (2009) A census of human transcription factors: function, expression and evolution. Nat Rev Genet. 10(4): 252-63

Vinson, C. et al. (2002) Classification of human B-ZIP proteins based on dimerization properties. Mol Cell Biol.

22(18):6321-35.

Walensky, L.D. et al. (2004) Activation of apoptosis in vivo by a hydrocarbon-stapled BH3 helix. Science. 305(5689):1466-70.

Wang, D. et al. (2005) Enhanced metabolic stability and protein-binding properties of artificial alpha helices derived from a hydrogen-bond surrogate: application to Bcl-xL. Angew Chem Int Ed Engl. 44(40):6525-9.

Wehr, M.C. & Rossner, M.J. (2016) Split protein biosensor assays in molecular pharmacological studies. Drug Discov Today. 21(3):415-29.

Wehr, M.C. et al. (2006) Monitoring regulated protein-protein interactions using split TEV. Nat Methods. 3(12):985-93.

Woolley, G.A. (2005) Photocontrolling peptide alpha helices. Acc Chem Res; 38(6):486-93.

Young,K.H. (1998) Yeast two-hybrid: so many interactions, (in) so little time... Biol Reprod. 58(2):302-11.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press

***Sequence Annex***

*Amino acid sequence of wild-type murine dihydrofolate reductase (SEQ ID NO: 1)*

MVRPLNCIVAVSQNMGIGKNGDLPWPPLRNEFKYFQRMTTTSSVEGKQNLVIMGRKTWFSIPEKNRPLKD
RINIVLSRELKEPPRGAHFLAKSLDDALRLIEQPELASKVDMVWIVGGSSVYQEAMNQPGHLRLFVTRIM
QEFESDTFFPEIDLGKYKLLPEYPGVLSEVQEEKGIKYKFEVYEKKD

*Amino acid sequence of murine dihydrofolate reductase engineered to contain TRE sites (SEQ ID NO: 2)*

MVRPLNCIVAVSQNMGIGKNGDLPWPPLRNESKYFQRMTQTDSVESKQNLVIMGRKTWFSIPESNRPLKD
RINIVLSQELKEPPRGAHFLAKSLDDALRLIESPELASKVDSVWIVGGSSVYQEAMTQPGHLRLFVTQIM
QEFESDTFFPEIDSGKYKLLPESPGVLSQVQEEKGIKYKFEVYEKKD

*Amino acid sequence of wild-type human dihydrofolate reductase (SEQ ID NO: 3)*

MVGSLNCIVAVSQNMGIGKNGDLPWPPLRNEFRYFQRMTTTSSVEGKQNLVIMGKKTWFSIPEKNRPLKG
RINLVLSRELKEPPQGAHFLSRSLDDALKLTEQPELANKVDMVWIVGGSSVYKEAMNHPGHLKLFVTRIM
QDFESDTFFPEIDLEKYKLLPEYPGVLSDVQEEKGIKYKFEVYEKND

*Nucleic acid sequence for the protein coding sequence of murine dihydrofolate reductase engineered to contain TRE sites (SEQ ID NO: 4)*

ATGGTTCGACCATTGAACTGCATCGTCGCCGTGAGTCAGAATATGGGGATTGGCAAGAACGGAGACCTACCCTGGCC
TCCGCTCAGGAATGAGTCAAAGTACTTCCAAAGAATGACTCAGACTGACTCAGTTGAGTCAAAACAGAATCTGGTGA
TTATGGGTAGGAAAACCTGGTTCTCCATTCCTGAGTCAAATCGACCTTTAAAGGACAGAATTAATATAGTTCTGAGT
CAAGAACTCAAAGAACCACCACGAGGAGCTCATTTTCTTGCCAAAAGTTTGGATGATGCCTTAAGACTTATTGAGTC
ACCGGAATTGGCGAGCAAAGTTGACTCAGTTTGGATCGTCGGAGGCAGTTCTGTTTACCAGGAAGCCATGACTCAAC
CAGGCCACCTTAGACTCTTTGTGACTCAGATCATGCAGGAATTTGAGTCAGACACGTTTTTCCCAGAAATTGACTCA
GGGAAATATAAACTTCTCCCTGAGTCACCAGGCGTCCTGAGTCAGGTCCAGGAGGAAAAAGGCATCAAGTATAAGTT
TGAAGTCTACGAGAAGAAAGACTAA

*Nucleic acid sequences of TPA response elements (TRE)*

TGACTCA (SEQ ID NO: 5)
TGAGTCA (SEQ ID NO: 6)

*Nucleic acid sequences of Ebox response elements*

CACGTG (SEQ ID NO: 7)
CACATG (SEQ ID NO: 8)

*Nucleic acid sequence of C/EBP protein response element*
ATTGCGCAAT (SEQ ID NO: 9)
*Nucleic acid sequence of cAMP response element (CRE)*
TGACGTCA (SEQ ID NO: 10)
*Nucleic acid sequences* of *Maf recognition elements (MAREs)*

TGCTGA$^{G}/_{C}$TCAGCA (SEQ ID NO: 32)
TGCTGA$^{GC}/_{CG}$TCAGCA (SEQ ID NO: 33)

*Nucleic acid sequence of PAP/CREB-2/PAR binding site*
TTACGTAA (SEQ ID NO: 34)
*Nucleic acid sequence of polynucleotide encoding engineered murine dihydrofolate reductase including restriction enzyme sites (SEQ ID NO: 11)*

GCTAGCGTTCGACCATTGAACTGCATCGTCGCCGTGAGTCAGAATATGGGGATTGGCAAGAACGGAGACCTACCCTG
GCCTCCGCTCAGGAATGAGTCAAAGTACTTCCAAAGAATGACTCAGACTGACTCAGTTGAGTCAAAACAGAATCTGG
TGATTATGGGTAGGAAAACCTGGTTCTCCATTCCTGAGTCAAATCGACCTTTAAAGGACAGAATTAATATAGTTCTG
AGTCAAGAACTCAAAGAACCACCACGAGGAGCTCATTTTCTTGCCAAAAGTTTGGATGATGCCTTAAGACTTATTGA
GTCACCGGAATTGGCGAGCAAAGTTGACTCAGTTTGGATCGTCGGAGGCAGTTCTGTTTACCAGGAAGCCATGACTC
AACCAGGCCACCTTAGACTCTTTGTGACTCAGATCATGCAGGAATTTGAGTCAGACACGTTTTTCCCAGAAATTGAC
TCAGGGAAATATAAACTTCTCCCTGAGTCACCAGGCGTCCTGAGTCAGGTCCAGGAGGAAAAAGGCATCAAGTATAA
GTTTGAAGTCTACGAGAAGAAAGACTAA

*Nucleic acid sequences of example reading frames*

Example reading frame 1: TGA CTC Axx (SEQ ID NO: 12)
Example reading frame 2: xTG ACT CAx (SEQ ID NO: 13)
Example reading frame 3: xxT GAC TCA (SEQ ID NO: 14)

*Amino acid sequence of example reading frame 3 (SEQ ID NO: 15)*
FSYCLPHRITNVADG
*Amino acid sequence of example codon triplets containing TREs*

GTGAGTCAG (SEQ ID NO: 16)
AATGAGTCA (SEQ ID NO: 17)
ATGACTCAG (SEQ ID NO: 18)
ACTGACTCA (SEQ ID NO: 19)
GTTGAGTCA (SEQ ID NO: 20)
CCTGAGTCA (SEQ ID NO: 21)
CTGAGTCAA (SEQ ID NO: 22)
ATTGAGTCA (SEQ ID NO: 23)
GTTGACTCA (SEQ ID NO: 24)
ATGACTCAA (SEQ ID NO: 25)
GTGACTCAG (SEQ ID NO: 26)
TTTGAGTCA (SEQ ID NO: 27)

ATTGACTCA (SEQ ID NO: 28)
CCTGAGTCA (SEQ ID NO: 29)
CTGAGTCAG (SEQ ID NO: 30)

*Amino acid sequence of engineered murine dihydrofolate reductase used during design process (SEQ ID NO: 31)*

* = stop codon

ASVRPLNCIVAVSQNMGIGKNGDLPWPPLRNESKYFQRMTQTDSVESKQNLVIMGRKTWFSIPESNRPLK
DRINIVLSQELKEPPRGAHFLAKSLDDALRLIESPELASKVDSVWIVGGSSVYQEAMTQPGHLRLFVTQI
MQEFESDTFFPEIDSGKYKLLPESPGVLSQVQEEKGIKYKFEVYEKKD*A*

*Nucleotide sequence of an exemplary murine dihydrofolate reductase gene engineered to include CRE binding sites (SEQ ID NO: 36)*

ATGGTTCGACCATTGAACTGCATCGTCGCCGTGTCCCAAAATATGGGGATTGGCAAGAACGGAGACCTAC
CCTGGCCTCCGCTCAGGAACGAGTTCAAGTACTTCCAAAGAATGACGTCAACCTCTTCAGTGGAAGGTAA
ACAGAATCTGGTGATTATGGGTAGGAAAACCTGGTTCTCCATTCCTGAGAAGAATCGACCTTTAAAGGAC
AGAATTAATATAGTGACGTCAAGAGAACTCAAAGAACCACCACGAGGAGCTCATTTTCTTGCCAAAAGTT
TGGATGATGCCTTAAGACTTATTGAACAACCGGAATTGACGTCAAAAGTAGACATGGTTTGGATCGTCGG
AGGCAGTTCTGTTTACCAGGAAGCCATGAATCAACCAGGCCACCTTAGACTCTTTGTGACGTCAATCATG
CAGGAATTTGAAAGTGACACGTTTTTCCCAGAAATTGATTTGGGGAAATATAAACTTCTCCCAGAATACC
CAGGCGTGACGTCAGAGGTCCAGGAGGAAAAAGGCATCAAGTATAAGTTTGAAGTCTACGAGAAGAAAGA
CTAAGCTTAA

*Amino acid sequence of an exemplary murine dihydrofolate reductase engineered to include CRE binding sites (SEQ ID NO: 37)*

MVRPLNCIVAVSQNMGIGKNGDLPWPPLRNEFKYFQRMTSTSSVEGKQNLVIMGRKTWFSIPEKNRPLKDRINIVTS
RELKEPPRGAHFLAKSLDDALRLIEQPELTSKVDMVWIVGGSSVYQEAMNQPGHLRLFVTSIMQEFESDTFFPEIDL
GKYKLLPEYPGVTSEVQEEKGIKYKFEVYEKKD

*Nucleotide sequence* of *an exemplary murine dihydrofolate reductase gene engineered to include CCAA T binding sites (SEQ ID NO: 38)*

ATGGTTCGACCATTGAACTGCATCGTCGCCGTGTCCCAAAATATGGGGATTGGCAAGAACGGAGACCTACCCTGGCC
TCCATTGCGCAATGAGTTCAAGTACTTCCAAAGAATGACCACAACCTCTTCAGTGGAAGGTAAACAGAATCTGGTGA
TTATGGGTAGGAAAACCTGGTTCTCCATTCCTGAGAAGAATCGACCATTGCGCAATAGAATTAATATAGTTCTCAGT
AGAGAATTGCGCAATCCACCACGAGGAGCTCATTTTATTGCGCAATCCTTGGATGATGCATTGCGCAATATTGAACA
ACCGGAATTGGCGAGCAAAGTAGACATGGTTTGGATCGTCGGAGGCAGTTCTGTTTACCAGGAAGCCATGAATCAAC
CAGGCCACCTTAGACTCTTTGTGACAAGGATCATGCAGGAATTTGAAAGTGACACGTTTTTCCCAGAAATTGATTTG
GGGAAATATAAACTTCTCCCAGAATACCCAGGCGTCCTCTCTGAATTGCGCAATGAAAAAGGCATCAAGTATAAGTT
TGAAGTCTACGAGAAGAAAGACTAAGCTTAA

*Amino acid sequence of an exemplary murine dihydrofolate reductase engineered to include CCAAT binding sites (SEQ ID NO: 39)*

MVRPLNCIVAVSQNMGIGKNGDLPWPPLRNEFKYFQRMTTTSSVEGKQNLVIMGRKTWFSIPEKNRPLRNRINIVLS
RELRNPPRGAHFIAQSLDDALRNIEQPELASKVDMVWIVGGSSVYQEAMNQPGHLRLFVTRIMQEFESDTFFPEIDL
GKYKLLPEYPGVLSEVQEEKGIKYKFEVYEKKD

*Nucleotide sequence of an exemplary murine dihydrofolate reductase gene engineered to include Eboxes (SEQ ID NO: 40)*

ATGGTTCGACCATTGAACTGCATCGTCGCCGTGTCCCAAAATATGGGGATTGGCAAGAACGGAGACCTAC
CCTGGCCTCCGCTCAGGAACGAGTTCAAGTACTTCCAAAGAATGACCACAACCTCTTCAGTGGAAGGTAA
ACAGAATCTGGTGATTATGGGTAGGCGCACGTGGTTCTCCATTCCTGAGAAGAATCGACCTTTAAAGGAC
AGAATTAATATAGTTCTCTCACGTGAACTCAAAGAACCACCACGTGGAGCTCACGTGCTTGCCAAATCAC
TGGATGATGCATTAAGACTTATTGAACAACCGGAATTGGCGTCACGTGTAGACATGGTTTGGATCGTCGG
AGGCAGTTCTGTTTACCAGGAAGCCATGAATCAACCAGGCCACGTGAGACTCTTTGTGACACGTGTCATG
CAGGAATTTGAAAGTGACACGTTTTTCCCAGAAATTGATTTGGGGAAATATAAACTTCTCCCAGAATACC
CAGGCGTCCTCTCACGTGTCCAGGAGGAAAAAGGCATCAAGTATAAGTTTGAAGTCTACGAGAAGAAAGA
CTAAGCTTAA

*Amino acid sequence of an exemplary murine dihydrofolate reductase engineered to include Eboxes (SEQ ID NO: 41)*

MVRPLNCIVAVSQNMGIGKNGDLPWPPLRNEFKYFQRMTTTSSVEGKQNLVIMGRRTWFSIPEKNRPLKDRINIVLS
RELKEPPRGAHVLAKSLDDALRLIEQPELASRVDMVWIVGGSSVYQEAMNQPGHVRLFVTRVMQEFESDTFFPEIDL
GKYKLLPEYPGVLSRVQEEKGIKYKFEVYEKKD

*Nucleotide sequence of p300-mDHFR plasmid used in Examples (SEQ ID NO: 42)*

CTCGAGAAATCATAAAAAATTTATTTGCTTTGTGAGCGGATAACAATTATAATAGATTCAATTGTGAGCGGATAACA
ATTTCACACAGAATTCATTAAAGAGGAGAAATTAAGCATGCACCATCACCATCACCATgctagcgttcgaccattga
actgcatcgtcgccgtgagtcagaatatggggattggcaagaacggagacctaccctggcctccgctcaggaatgag
tcaaagtacttccaaagaatgactcagactgactcagttgagtcaaaacagaatctggtgattatgggtaggaaaac
ctggttctccattcctgagtcaaatcgacctttaaaggacagaattaatatagttctgagtcaagaactcaaagaac
caccacgaggagctcatttcttgccaaaagtttggatgatgccttaagacttattgagtcaccggaattggcgagc
aaagttgactcagtttggatcgtcggaggcagttctgtttaccaggaagccatgactcaaccaggccaccttagact
ctttgtgactcagatcatgcaggaatttgagtcagacacgtttttcccagaaattgactcagggaaatataaacttc
tccctgagtcaccaggcgtcctgagtcaggtccaggaggaaaaaggcatcaagtataagtttgaagtctacgagaag
aaagactaagcttAATTAGCTGAGCTTGGACTCCTGTTGATAGATCCAGTAATGACCTCAGAACTCCATCTGGATTT
GTTCAGAACGCTCGGTTGCCGCCGGGCGTTTTTTATTGGTGAGAATCCAAGCTAGTTTGGGAGGTTCCAACTTTCAC
CATAATGAAATAAGATCACTACCGGGCGTATTTTTTGAGTTATCGAGATTTTCAGGAGCTAAGGAAGCTAAAATGGA
GAAAAAAATCACTGGATATACCACCGTTGATATATCCCAATGGCATCGTAAAGAACATTTTGAGGCATTTCAGTCAG
TTGCTCAATGTACCTATAACCAGACCGTTCAGCTGGATATTACGGCCTTTTTAAAGACCGTAAAGAAAAATAAGCAC
AAGTTTTATCCGGCCTTTATTCACATTCTTGCCCGCCTGATGAATGCTCATCCGGAGTTCCGTATGGCAATGAAAGA
CGGTGAGCTGGTGATATGGGATAGTGTTCACCCTTGTTACACCGTTTTCCATGAGCAAACTGAAACGTTTTCATCGC
TCTGGAGTGAATACCACGACGATTTCCGGCAGTTTCTACACATATATTCGCAAGATGTGGCGTGTTACGGTGAAAAC
CTGGCCTATTTCCCTAAAGGGTTTATTGAGAATATGTTTTTCGTCTCAGCCAATCCCTGGGTGAGTTTCACCAGTTT
TGATTTAAACGTGGCCAATATGGACAACTTCTTCGCCCCCGTTTTCACCATGGGCAAATATTATACGCAAGGCGACA
AGGTGCTGATGCCGCTGGCGATTCAGGTTCATCATGCCGTTTGTGATGGCTTCCATGTCGGCAGAATGCTTAATGAA
TTACAACAGTACTGCGATGAGTGGCAGGGCGGGGCGTAATTTTTTTAAGGCAGTTATTGGTGCCCTTAAACGCCTGG
GGTAATGACTCTCTAGCTTGAGGCATCAAATAAAACGAAAGGCTCAGTCGAAAGACTGGGCCTTTCGTTTTATCTGT
TGTTTGTCGGTGAACGCTCTCCTGAGTAGGACAAATCCGCCCTCTAGAGCTGCCTCGCGCGTTTCGGTGATGACGGT
GAAAACCTCTGACACATGCAGCTCCCGGAGACGGTCACAGCTTGTCTGTAAGCGGATGCCGGGAGCAGACAAGCCCG
TCAGGGCGCGTCAGCGGGTGTTGGCGGGTGTCGGGGCGCAGCCATGACCCAGTCACGTAGCGATAGCGGAGTGTATA
CTGGCTTAACTATGCGGCATCAGAGCAGATTGTACTGAGAGTGCACCATATGCGGTGTGAAATACCGCACAGATGCG
TAAGGAGAAAATACCGCATCAGGCGCTCTTCCGCTTCCTCGCTCACTGACTCGCTGCGCTCGGTCGTTCGGCTGCGG
CGAGCGGTATCAGCTCACTCAAAGGCGGTAATACGGTTATCCACAGAATCAGGGGATAACGCAGGAAAGAACATGTG
AGCAAAAGGCCAGCAAAAGGCCAGGAACCGTAAAAAGGCCGCGTTGCTGGCGTTTTTCCATAGGCTCCGCCCCCCTG
ACGAGCATCACAAAAATCGACGCTCAAGTCAGAGGTGGCGAAACCCGACAGGACTATAAAGATACCAGGCGTTTCCC
CCTGGAAGCTCCCTCGTGCGCTCTCCTGTTCCGACCCTGCCGCTTACCGGATACCTGTCCGCCTTTCTCCCTTCGGG
AAGCGTGGCGCTTTCTCATAGCTCACGCTGTAGGTATCTCAGTTCGGTGTAGGTCGTTCGCTCCAAGCTGGGCTGTG

TGCACGAACCCCCCGTTCAGCCCGACCGCTGCGCCTTATCCGGTAACTATCGTCTTGAGTCCAACCCGGTAAGACAC
GACTTATCGCCACTGGCAGCAGCCACTGGTAACAGGATTAGCAGAGCGAGGTATGTAGGCGGTGCTACAGAGTTCTT
GAAGTGGTGGCCTAACTACGGCTACACTAGAAGGACAGTATTTGGTATCTGCGCTCTGCTGAAGCCAGTTACCTTCG
GAAAAAGAGTTGGTAGCTCTTGATCCGGCAAACAAACCACCGCTGGTAGCGGTGGTTTTTTTGTTTGCAAGCAGCAG
ATTACGCGCAGAAAAAAAGGATCTCAAGAAGATCCTTTGATCTTTTCTACGGGGTCTGACGCTCAGTGGAACGAAAA
CTCACGTTAAGGGATTTTGGTCATGAGATTATCAAAAAGGATCTTCACCTAGATCCTTTTAAATTAAAAATGAAGTT
TTAAATCAATCTAAAGTATATATGAGTAAACTTGGTCTGACAGTTACCAATGCTTAATCAGTGAGGCACCTATCTCA
GCGATCTGTCTATTTCGTTCATCCATAGTTGCCTGACTCCCCGTCGTGTAGATAACTACGATACGGGAGGGCTTACC
ATCTGGCCCCAGTGCTGCAATGATACCGCGAGACCCACGCTCACCGGCTCCAGATTTATCAGCAATAAACCAGCCAG
CCGCGTTCTTCGGGGCGAAAACTCTCAAGGATCTTACCGCTGTTGAGATCCAGTTCGATGTAACCCACTCGTGCACC
CAACTGATCTTCAGCATCTTTTACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATTATTGAAGCATTTATCAGGGT
TATTGTCTCATGAGCGGATACATATTTGAATGTATTTAGAAAAATAAACAAATAGGGGTTCCGCGCACATTTCCCCG
AAAAGTGCCACCTGACGTCTAAGAAACCATTATTATCATGACATTAACCTATAAAAATAGGCGTATCACGAGGCCCT
TTCGTCTTCAC

*<u>Nucleotide sequence of p230d-basic-cJun plasmid used in Examples (SEQ ID NO: 43)</u>*

CTCGAGAAATCATAAAAAATTTATTTGCTTTGTGAGCGGATAACAATTATAATAGATTCAATTGTGAGCGGATAACA
ATTTCACACAGAATTCATTAAAGAGGAGAAATTAAGCATGCGCATTAAAGCCGAACGCAAACGGATGCGCAACCGCA
TCGCAGCCTCCAAGTGCCGCAAACGCAAATTGGAGCGCATCGCCCGCTTGGAAGAAAAGGTGAAAACCCTGAAAGCA
CAGAACTATGAGCTGGCCTCCACCGCCAACATGTTGCGCGAACAGGTGGCCCAGCTCGGCGCGCCTCATCACCATCA
CCATCACTGATAAAGCGCGCCTTGATAAGCTTAATTAGCTGAGCTTGGACTCCTGTTGATAGATCCAGTAATGACCT
CAGAACTCCATCTGGATTTGTTCAGAACGCTCGGTTGCCGCCGGGCGTTTTTTATTGGTGAGAATCCAGGCGAGATT
TTCAGGAGCTAAGGAAGCTAAAATGGAGAAAAAAATCACTGGATATACCACCGTTGATATATCCCAATGGCATCGTA
AAGAACATTTTGAGGCATTTCAGTCAGTTGCTCAATGTACCTATAACCAGACCGTTCAGCTGGATATTACGGCCTTT
TTAAAGACCGTAAAGAAAAATAAGCACAAGTTTTATCCGGCCTTTATTCACATTCTTGCCCGCCTGATGAATGCTCA
TCCGGAATTTCGTATGGCAATGAAAGACGGTGAGCTGGTGATATGGGATAGTGTTCACCCTTGTTACACCGTTTTCC
ATGAGCAAACTGAAACGTTTTCATCGCTCTGGAGTGAATACCACGACGATTTCCGGCAGTTTCTACACATATATTCG
CAAGATGTGGCGTGTTACGGTGAAAACCTGGCCTATTTCCCTAAAGGGTTTATTGAGAATATGTTTTTCGTCTCAGC
CAATCCCTGGGTGAGTTTCACCAGTTTTGATTTAAACGTGGCCAATATGGACAACTTCTTCGCCCCCGTTTTCACCA
TGGGCAAATATTATACGCAAGGCGACAAGGTGCTGATGCCGCTGGCGATTCAGGTTCATCATGCCGTTTGTGATGGC
TTCCATGTCGGCAGAATGCTTAATGAATTACAACAGTACTGCGATGAGTGGCAGGGCGGGGCGTAATTTTTTTAAGG
CAGTTATTGGTGCCCTTAAACGCCTGGGGTAATGACTCTCTAGCTTGAGGCATCAAATAAAACGAAAGGCTCAGTCG
AAAGACTGGGCCTTTCGTTTTATCTGTTGTTTGTCGGTGAACGCTCTCCTGAGTAGGACAAATCCGCCCTCTAGAGC
TGCCTCGCGCGTTTCGGTGATGACGGTGAAAACCTCTGACACATGCAGCTCCCGGAGACGGTCACAGCTTGTCTGTA
AGCGGATGCCGGGAGCAGACAAGCCCGTCAGGGCGCGTCAGCGGGTGTTGGCGGGTGTCGGGGCGCAGCCATGACCC
AGTCACGTAGCGATAGCGGAGTGTATACTGGCTTAACTATGCGGCATCAGAGCAGATTGTACTGAGAGTGCACCATA
TGCGGTGTGAAATACCGCACAGATGCGTAAGGAGAAAATACCGCATCAGGCGCTCTTCCGCTTCCTCGCTCACTGAC
TCGCTGCGCTCGGTCGTTCGGCTGCGGCGAGCGGTATCAGCTCACTCAAAGGCGGTAATACGGTTATCCACAGAATC
AGGGGATAACGCAGGAAAGAACATGTGAGCAAAAGGCCAGCAAAAGGCCAGGAACCGTAAAAAGGCCGCGTTGCTGG
CGTTTTTCCATAGGCTCCGCCCCCCTGACGAGCATCACAAAAATCGACGCTCAAGTCAGAGGTGGCGAAACCCGACA
GGACTATAAAGATACCAGGCGTTTCCCCCTGGAAGCTCCCTCGTGCGCTCTCCTGTTCCGACCCTGCCGCTTACCGG
ATACCTGTCCGCCTTTCTCCCTTCGGGAAGCGTGGCGCTTTCTCATAGCTCACGCTGTAGGTATCTCAGTTCGGTGT

AGGTCGTTCGCTCCAAGCTGGGCTGTGTGCACGAACCCCCCGTTCAGCCCGACCGCTGCGCCTTATCCGGTAACTAT
CGTCTTGAGTCCAACCCGGTAAGACACGACTTATCGCCACTGGCAGCAGCCACTGGTAACAGGATTAGCAGAGCGAG
GTATGTAGGCGGTGCTACAGAGTTCTTGAAGTGGTGGCCTAACTACGGCTACACTAGAAGGACAGTATTTGGTATCT
GCGCTCTGCTGAAGCCAGTTACCTTCGGAAAAAGAGTTGGTAGCTCTTGATCCGGCAAACAAACCACCGCTGGTAGC
GGTGGTTTTTTTGTTTGCAAGCAGCAGATTACGCGCAGAAAAAAAGGATCTCAAGAAGATCCTTTGATCTTTTCTAC
GGGGTCTGACGCTCAGTGGAACGAAAACTCACGTTAAGGGATTTTGGTCATGAGATTATCAAAAAGGATCTTCACCT
AGATCCTTTTAAATTAAAAATGAAGTTTTAAATCAATCTAAAGTATATATGAGTAAACTTGGTCTGACAGTTACCAA
TGCTTAATCAGTGAGGCACCTATCTCAGCGATCTGTCTATTTCGTTCATCCATAGTTGCCTGACTCCCCGTCGTGTA
GATAACTACGATACGGGAGGGCTTACCATCTGGCCCCAGTGCTGCAATGATACCGCGAGACCCACGCTCACCGGCTC
CAGATTTATCAGCAATAAACCAGCCAGCCGGAAGGGCCGAGCGCAGAAGTGGTCCTGCAACTTTATCCGCCTCCATC
CAGTCTATTAATTGTTGCCGGGAAGCTAGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCTCCGGTTCCCAACGATCAAGGCGAGTTA
CATGATCCCCCATGTTGTGCAAAAAAGCGGTTAGCTCCTTCGGTCCTCCGATCGTTGTCAGAAGTAAGTTGGCCGCA
GTGTTATCACTCATGGTTATGGCAGCACTGCATAATTCTCTTACTGTCATGCCATCCGTAAGATGCTTTTCTGTGAC
TGGTGAGTACTCAACCAAGTCATTCTGAGAATAGTGTATGCGGCGACCGAGTTGCTCTTGCCCGGCGTCAATACGGG
ATAATACCGCGCCACATAGCAGAACTTTAAAAGTGCTCATCATTGGAAAACGTTCTTCGGGGCGAAAACTCTCAAGG
ATCTTACCGCTGTTGAGATCCAGTTCGATGTAACCCACTCGTGCACCCAACTGATCTTCAGCATCTTTTACTTTCAC
CAGCGTTTCTGGGTGAGCAAAAACAGGAAGGCAAAATGCCGCAAAAAAGGGAATAAGGGCGACACGGAAATGTTGAA
TACTCATACTCTTCCTTTTTCAATATTATTGAAGCATTTATCAGGGTTATTGTCTCATGAGCGGATACATATTTGAA
TGTATTTAGAAAAATAAACAAATAGGGGTTCCGCGCACATTTCCCCGAAAAGTGCCACCTGACGTCTAAGAAACCAT
TATTATCATGACATTAACCTATAAAAATAGGCGTATCAC*GAGGCCCTTTCGTCTTCAC*

*Nucleotide sequence of pREP4 expressing the lac repressor used in Examples (SEQ ID NO: 44)*

AAGCTTCACGCTGCCGCAAGCACTCAGGGCGCAAGGGCTGCTAAAGGAAGCGGAACACGTAGAAAGCCAGTCCGCAG
AAACGGTGCTGACCCCGGATGAATGTCAGCTACTGGGCTATCTGGACAAGGGAAAACGCAAGCGCAAAGAGAAAGCA
GGTAGCTTGCAGTGGGCTTACATGGCGATAGCTAGACTGGGCGGTTTTATGGACAGCAAGCGAACCGGAATTGCCAG
CTGGGGCGCCCTCTGGTAAGGTTGGGAAGCCCTGCAAAGTAAACTGGATGGCTTTCTTGCCGCCAAGGATCTGATGG
CGCAGGGGATCAAGATCTGATCAAGAGACAGGATGACGGTCGTTTCGCATGCTTGAACAAGATGGATTGCACGCAGG
TTCTCCGGCCGCTTGGGTGGAGAGGCTATTCGGCTATGACTGGGCACAACAGACAATCGGCTGCTCTGATGCCGCCG
TGTTCCGGCTGTCAGCGCAGGGGCGCCCGGTTCTTTTTGTCAAGACCGACCTGTCCGGTGCCCTGAATGAACTGCAG
GACGAGGCAGCGCGGCTATCGTGGCTGGCCACGACGGGCGTTCCTTGCGCAGCTGTGCTCGACGTTGTCACTGAAGC
GGGAAGGGACTGGCTGCTATTGGGCGAAGTGCCGGGGCAGGATCTCCTGTCATCTCACCTTGCTCCTGCCGAGAAAG
TATCCATCATGGCTGATGCAATGCGGCGGCTGCATACGCTTGATCCGGCTACCTGCCCATTCGACCACCAAGCGAAA
CATCGCATCGAGCGAGCACGTACTCGGATGGAAGCCGGTCTTGTCGATCAGGATGATCTGGACGAAGAGCATCAGGG
GCTCGCGCCAGCCGAACTGTTCGCCAGGCTCAAGGCGCGCATGCCCGACGGCGAGGATCTCGTCGTGACCCATGGCG
ATGCCTGCTTGCCGAATATCATGGTGGAAAATGGCCGCTTTTCTGGATTCATCGACTGTGGCCGGCTGGGTGTGGCG
GACCGCTATCAGGACATAGCGTTGGCTACCCGTGATATTGCTGAAGAGCTTGGCGGCGAATGGGCTGACCGCTTCCT
CGTGCTTTACGGTATCGCCGCTCCCGATTCGCAGCGCATCGCCTTCTATCGCCTTCTTGACGAGTTCTTCTGAGCGG
GACTCTGGGGTTCGAAATGACCGACCAAGCGACGCCCAACCTGCCATCACGAGATTTCGATTCCACCGCCGCCTTCT
ATGAAAGGTTGGGCTTCGGAATCGTTTTCCGGGACGCCGGCTGGATGATCCTCCAGCGCGGGGATCTCATGCTGGAG
TTCTTCGCCCACCCCGGGCTCGATCCCCTCGCGAGTTGGTTCAGCTGCTGCCTGAGGCTGGACGACCTCGCGGAGTT
CTACCGGCAGTGCAAATCCGTCGGCATCCAGGAAACCAGCAGCGGCTATCCGCGCATCCATGCCCCCGAACTGCAGG

AGTGGGGAGGCACGATGGCCGCTTTGGTCGACAATTCGCGCTAACTTACATTAATTGCGTTGCGCTCACTGCCCGCT
TTCCAGTCGGGAAACCTGTCGTGCCAGCTGCATTAATGAATCGGCCAACGCGCGGGGAGAGGCGGTTTGCGTATTGG
GCGCCAGGGTGGTTTTTCTTTTCACCAGTGAGACGGGCAACAGCTGATTGCCCTTCACCGCCTGGCCCTGAGAGAGT
TGCAGCAAGCGGTCCACGCTGGTTTGCCCCAGCAGGCGAAAATCCTGTTTGATGGTGGTTAACGGCGGGATATAACA
TGAGCTGTCTTCGGTATCGTCGTATCCCACTACCGAGATATCCGCACCAACGCGCAGCCCGGACTCGGTAATGGCGC
GCATTGCGCCCAGCGCCATCTGATCGTTGGCAACCAGCATCGCAGTGGGAACGATGCCCTCATTCAGCATTTGCATG
GTTTGTTGAAAACCGGACATGGCACTCCAGTCGCCTTCCCGTTCCGCTATCGGCTGAATTTGATTGCGAGTGAGATA
TTTATGCCAGCCAGCCAGACGCAGACGCGCCGAGACAGAACTTAATGGGCCCGCTAACAGCGCGATTTGCTGGTGAC
CCAATGCGACCAGATGCTCCACGCCCAGTCGCGTACCGTCTTCATGGGAGAAAATAATACTGTTGATGGGTGTCTGG
TCAGAGACATCAAGAAATAACGCCGGAACATTAGTGCAGGCAGCTTCCACAGCAATGGCATCCTGGTCATCCAGCGG
ATAGTTAATGATCAGCCCACTGACGCGTTGCGCGAGAAGATTGTGCACCGCCGCTTTACAGGCTTCGACGCCGCTTC
GTTCTACCATCGACACCACCACGCTGGCACCCAGTTGATCGGCGCGAGATTTAATCGCCGCGACAATTTGCGACGGC
GCGTGCAGGGCCAGACTGGAGGTGGCAACGCCAATCAGCAACGACTGTTTGCCCGCCAGTTGTTGTGCCACGCGGTT
GGGAATGTAATTCAGCTCCGCCATCGCCGCTTCCACTTTTTCCCGCGTTTTCGCAGAAACGTGGCTGGCCTGGTTCA
CCACGCGGGAAACGGTCTGATAAGAGACACCGGCATACTCTGCGACATCGTATAACGTTACTGGTTTCACATTCACC
ACCCTGAATTGACTCTCTTCCGGGCGCTATCATGCCATACCGCGAAAGGTTTTGCGCCATTCGATGGTGTCAACGTA
AATGCATGCCGCTTCGCCTTCGCGCGCGAATTGTCGACCCTGTCCCTCCTGTTCAGCTACTGACGGGGTGGTGCGTA
ACGGCAAAAGCACCGCCGGACATCAGCGCTAGCGGAGTGTATACTGGCTTACTATGTTGGCACTGATGAGGGTGTCA
GTGAAGTGCTTCATGTGGCAGGAGAAAAAAGGCTGCACCGGTGCGTCAGCAGAATATGTGATACAGGATATATTCCG
CTTCCTCGCTCACTGACTCGCTACGCTCGGTCGTTCGACTGCGGCGAGCGGAAATGGCTTACGAACGGGGCGGAGAT
TTCCTGGAAGATGCCAGGAAGATACTTAACAGGGAAGTGAGAGGGCCGCGGCAAAGCCGTTTTTCCATAGGCTCCGC
CCCCCTGACAAGCATCACGAAATCTGACGCTCAAATCAGTGGTGGCGAAACCCGACAGGACTATAAAGATACCAGGC
GTTTCCCCTGGCGGCTCCCTCGTGCGCTCTCCTGTTCCTGCCTTTCGGTTTACCGGTGTCATTCCGCTGTTATGGCC
GCGTTTGTCTCATTCCACGCCTGACACTCAGTTCCGGGTAGGCAGTTCGCTCCAAGCTGGACTGTATGCACGAACCC
CCCGTTCAGTCCGACCGCTGCGCCTTATCCGGTAACTATCGTCTTGAGTCCAACCCGGAAAGACATGCAAAAGCACC
ACTGGCAGCAGCCACTGGTAATTGATTTAGAGGAGTTAGTCTTGAAGTCATGCGCCGGTTAAGGCTAAACTGAAAGG
ACAAGTTTTGGTGACTGCGCTCCTCCAAGCCAGTTACCTCGGTTCAAAGAGTTGGTAGCTCAGAGAACCTTCGAAAA
ACCGCCCTGCAAGGCGGTTTTTTCGTTTTCAGAGCAAGAGATTACGCGCAGACCAAAACGATCTCAAGAAGATCATC
TTATTAATCAGATAAAATATTTCTAGATTTCAGTGCAATTTATCTCTTCAAATGTAGCACCTGAAGTCAGCCCCATA
CGATATAAGTTGTTAATTCTCATGTTTGACAGCTTATCATCGAT

*Nucleotide sequence of control cJun plasmid lacking the DNA-binding basic region used in Examples (SEQ ID NO: 45)*

CTCGAGAAATCATAAAAAATTTATTTGCTTTGTGAGCGGATAACAATTATAATAGATTCAATTGTGAGCGGATAACA
ATTTCACACAGAATTCATTAAAGAGGAGAAATTAAGCATGCACCATCACCATCACCATGCTAGCATCGCCCGGCTGG
AGGAAAAAGTGAAGACCTTGAAGGCCCAGAACTATGAGCTGGCGTCCACGGCCAACATGCTCCGGGAACAGGTGGCA
CAGCTTGGCGCGCCTTAAGGTAGCTCTAAGCTTAATTAGCTGAGCTTGGACTCCTGTTGATAGATCCAGTAATGACC
TCAGAACTCCATCTGGATTTGTTCAGAACGCTCGGTTGCCGCCGGGCGTTTTTTATTGGTGAGAATCCAGGCGAGAT
TTTCAGGAGCTAAGGAAGCTAAAATGGAGAAAAAAATCACTGGATATACCACCGTTGATATATCCCAATGGCATCGT
AAAGAACATTTTGAGGCATTTCAGTCAGTTGCTCAATGTACCTATAACCAGACCGTTCAGCTGGATATTACGGCCTT
TTTAAAGACCGTAAAGAAAAATAAGCACAAGTTTTATCCGGCCTTTATTCACATTCTTGCCCGCCTGATGAATGCTT
AATGAATTACAACAGTACTGCGATGAGTGGCAGGGCGGGGCGTAATTTTTTTAAGGCAGTTATTGGTGCCCTTAAAC
GCCTGGGGTAATGACTCTCTAGCTTGAGGCATCAAATAAAACGAAAGGCTCAGTCGAAAGACTGGGCCTTTCGTTTT

ATCTGTTGTTTGTCGGTGAACGCTCTCCTGAGTAGGACAAATCCGCCCTCTAGAGCTGCCTCGCGCGTTTCGGTGAT
GACGGTGAAAACCTCTGACACATGCAGCTCCCGGAGACGGTCACAGCTTGTCTGTAAGCGGATGCCGGGAGCAGACA
AGCCCGTCAGGGCGCGTCAGCGGGTGTTGGCGGGTGTCGGGGCGCAGCCATGACCCAGTCACGTAGCGATAGCGGAG
TGTATACTGGCTTAACTATGCGGCATCAGAGCAGATTGTACTGAGAGTGCACCATATGCGGTGTGAAATACCGCACA
GATGCGTAAGGAGAAAATACCGCATCAGGCGCTCTTCCGCTTCCTCGCTCACTGACTCGCTGCGCTCGGTCGTTCGG
CTGCGGCGAGCGGTATCAGCTCACTCAAAGGCGGTAATACGGTTATCCACAGAATCAGGGGATAACGCAGGAAAGAA
CATGTGAGCAAAAGGCCAGCAAAAGGCCAGGAACCGTAAAAAGGCCGCGTTGCTGGCGTTTTTCCATAGGCTCCGCC
CCCCTGACGAGCATCACAAAAATCGACGCTCAAGTCAGAGGTGGCGAAACCCGACAGGACTATAAAGATACCAGGCG
TTTCCCCCTGGAAGCTCCCTCGTGCGCTCTCCTGTTCCGACCCTGCCGCTTACCGGATACCTGTCCGCCTTTCTCCC
TTCGGGAAGCGTGGCGCTTTCTCATAGCTCACGCTGTAGGTATCTCAGTTCGGTGTAGGTCGTTCGCTCCAAGCTGG
GCTGTGTGCACGAACCCCCCGTTCAGCCCGACCGCTGCGCCTTATCCGGTAACTATCGTCTTGAGTCCAACCCGGTA
AGACACGACTTATCGCCACTGGCAGCAGCCACTGGTAACAGGATTAGCAGAGCGAGGTATGTAGGCGGTGCTACAGA
GTTCTTGAAGTGGTGGCCTAACTACGGCTACACTAGAAGGACAGTATTTGGTATCTGCGCTCTGCTGAAGCCAGTTA
CCTTCGGAAAAAGAGTTGGTAGCTCTTGATCCGGCAAACAAACCACCGCTGGTAGCGGTGGTTTTTTTGTTTGCAAG
CAGCAGATTACGCGCAGAAAAAAAGGATCTCAAGAAGATCCTTTGATCTTTTCTACGGGGTCTGACGCTCAGTGGAA
CGAAAACTCACGTTAAGGGATTTTGGTCATGAGATTATCAAAAAGGATCTTCACCTAGATCCTTTTAAATTAAAAAT
GAAGTTTTAAATCAATCTAAAGTATATATGAGTAAACTTGGTCTGACAGTTACCAATGCTTAATCAGTGAGGCACCT
ATCTCAGCGATCTGTCTATTTCGTTCATCCATAGTTGCCTGACTCCCCGTCGTGTAGATAACTACGATACGGGAGGG
CTTACCATCTGGCCCCAGTGCTGCAATGATACCGCGAGACCCACGCTCACCGGCTCCAGATTTATCAGCAATAAACC
AGCCAGCCGGAAGGGCCGAGCGCAGAAGTGGTCCTGCAACTTTATCCGCCTCCATCCAGTCTATTAATTGTTGCCGG
GAAGCTAGAGTAAGTAGTTCGCCAGTTAATAGTTTGCGCAACGTTGTTGCCATTGCTACAGGCATCGTGGTGTCACG
CTCGTCGTTTGGTATGGCTTCATTCAGCTCCGGTTCCCAACGATCAAGGCGAGTTACATGATCCCCCATGTTGTGCA
AAAAAGCGGTTAGCTCCTTCGGTCCTCCGATCGTTGTCAGAAGTAAGTTGGCCGCAGTGTTATCACTCATGGTTATG
GCAGCACTGCATAATTCTCTTACTGTCATGCCATCCGTAAGATGCTTTTCTGTGACTGGTGAGTACTCAACCAAGTC
ATTCTGAGAATAGTGTATGCGGCGACCGAGTTGCTCTTGCCCGGCGTCAATACGGGATAATACCGCGCCACATAGCA
GAACTTTAAAAGTGCTCATCATTGGAAAACGTTCTTCGGGGCGAAAACTCTCAAGGATCTTACCGCTGTTGAGATCC
AGTTCGATGTAACCCACTCGTGCACCCAACTGATCTTCAGCATCTTTTACTTTCACCAGCGTTTCTGGGTGAGCAAA
AACAGGAAGGCAAAATGCCGCAAAAAAGGGAATAAGGGCGACACGGAAATGTTGAATACTCATACTCTTCCTTTTTC
AATATTATTGAAGCATTTATCAGGGTTATTGTCTCATGAGCGGATACATATTTGAATGTATTTAGAAAAATAAACAA
ATAGGGGTTCCGCGCACATTTCCCCGAAAAGTGCCACCTGACGTCTAAGAAACCATTATTATCATGACATTAACCTA
TAAAAATAGGCGTATCACGAGGCCCTTTCGTCTTCAC

*Nucleotide sequence of pET24a plasmid containing FosW (SEQ ID NO: 46)*

ATCCGGATATAGTTCCTCCTTTCAGCAAAAAACCCCTCAAGACCCGTTTAGAGGCCCCAAGGGGTTATGCTAGTTAT
TGCTCAGCGGTGGCAGCAGCCAACTCAGCTTCCTTTCGGGCTTTGTTAGCAGCCGGATCTCAGTGGTGGTGGTGGTG
GTGCTCGAGTGCGGCCGCAAGCTTGTCGACGGAGCTCGAATTCGGATCCTTAAGGCGCGCCCAGTTTCTCCAGCTGT
TTCTGGAGGTCTTCGATCTCTTTGCGCAAGGCATAGTTGCGTTCTTCCAGCTGTTCAATCTCGGCCTGCAGTTCATC
GAGTTCCTGTTCGAGCTCTTCGGCCTCTTTTTCCAATTCTTCGTTCTCGCGGGCCAGTTCTTCGGCCCGCTGTTCCA
GGCTAGCCATATGTATATCTCCTTCTTAAAGTTAAACAAAATTATTTCTAGAGGGGAATTGTTATCCGCTCACAATT
CCCCTATAGTGAGTCGTATTAATTTCGCGGGATCGAGATCTCGATCCTCTACGCCGGACGCATCGTGGCCGGCATCA
CCGGCGCCACAGGTGCGGTTGCTGGCGCCTATATCGCCGACATCACCGATGGGGAAGATCGGGCTCGCCACTTCGGG
CTCATGAGCGCTTGTTTCGGCGTGGGTATGGTGGCAGGCCCCGTGGCCGGGGGACTGTTGGGCGCCATCTCCTTGCA
TGCACCATTCCTTGCGGCGGCGGTGCTCAACGGCCTCAACCTACTACTGGGCTGCTTCCTAATGCAGGAGTCGCATA

AGGGAGAGCGTCGAGATCCCGGACACCATCGAATGGCGCAAAACCTTTCGCGGTATGGCATGATAGCGCCCGGAAGA
GAGTCAATTCAGGGTGGTGAATGTGAAACCAGTAACGTTATACGATGTCGCAGAGTATGCCGGTGTCTCTTATCAGA
CCGTTTCCCGCGTGGTGAACCAGGCCAGCCACGTTTCTGCGAAAACGCGGGAAAAAGTGGAAGCGGCGATGGCGGAG
CTGAATTACATTCCCAACCGCGTGGCACAACAACTGGCGGGCAAACAGTCGTTGCTGATTGGCGTTGCCACCTCCAG
TCTGGCCCTGCACGCGCCGTCGCAAATTGTCGCGGCGATTAAATCTCGCGCCGATCAACTGGGTGCCAGCGTGGTGG
TGTCGATGGTAGAACGAAGCGGCGTCGAAGCCTGTAAAGCGGCGGTGCACAATCTTCTCGCGCAACGCGTCAGTGGG
CTGATCATTAACTATCCGCTGGATGACCAGGATGCCATTGCTGTGGAAGCTGCCTGCACTAATGTTCCGGCGTTATT
TCTTGATGTCTCTGACCAGACACCCATCAACAGTATTATTTTCTCCCATGAAGACGGTACGCGACTGGGCGTGGAGC
ATCTGGTCGCATTGGGTCACCAGCAAATCGCGCTGTTAGCGGGCCCATTAAGTTCTGTCTCGGCGCGTCTGCGTCTG
GCTGGCTGGCATAAATATCTCACTCGCAATCAAATTCAGCCGATAGCGGAACGGGAAGGCGACTGGAGTGCCATGTC
CGGTTTTCAACAAACCATGCAAATGCTGAATGAGGGCATCGTTCCCACTGCGATGCTGGTTGCCAACGATCAGATGG
CGCTGGGCGCAATGCGCGCCATTACCGAGTCCGGGCTGCGCGTTGGTGCGGATATCTCGGTAGTGGGATACGACGAT
ACCGAAGACAGCTCATGTTATATCCCGCCGTTAACCACCATCAAACAGGATTTTCGCCTGCTGGGGCAAACCAGCGT
GGACCGCTTGCTGCAACTCTCTCAGGGCCAGGCGGTGAAGGGCAATCAGCTGTTGCCCGTCTCACTGGTGAAAAGAA
AAACCACCCTGGCGCCCAATACGCAAACCGCCTCTCCCCGCGCGTTGGCCGATTCATTAATGCAGCTGGCACGACAG
GTTTCCCGACTGGAAAGCGGGCAGTGAGCGCAACGCAATTAATGTAAGTTAGCTCACTCATTAGGCACCGGGATCTC
GACCGATGCCCTTGAGAGCCTTCAACCCAGTCAGCTCCTTCCGGTGGGCGCGGGGCATGACTATCGTCGCCGCACTT
ATGACTGTCTTCTTTATCATGCAACTCGTAGGACAGGTGCCGGCAGCGCTCTGGGTCATTTTCGGCGAGGACCGCTT
TCGCTGGAGCGCGACGATGATCGGCCTGTCGCTTGCGGTATTCGGAATCTTGCACGCCCTCGCTCAAGCCTTCGTCA
CTGGTCCCGCCACCAAACGTTTCGGCGAGAAGCAGGCCATTATCGCCGGCATGGCGGCCCCACGGGTGCGCATGATC
GTGCTCCTGTCGTTGAGGACCCGGCTAGGCTGGCGGGGTTGCCTTACTGGTTAGCAGAATGAATCACCGATACGCGA
GCGAACGTGAAGCGACTGCTGCTGCAAAACGTCTGCGACCTGAGCAACAACATGAATGGTCTTCGGTTTCCGTGTTT
CGTAAAGTCTGGAAACGCGGAAGTCAGCGCCCTGCACCATTATGTTCCGGATCTGCATCGCAGGATGCTGCTGGCTA
CCCTGTGGAACACCTACATCTGTATTAACGAAGCGCTGGCATTGACCCTGAGTGATTTTTCTCTGGTCCCGCCGCAT
CCATACCGCCAGTTGTTTACCCTCACAACGTTCCAGTAACCGGGCATGTTCATCATCAGTAACCCGTATCGTGAGCA
TCCTCTCTCGTTTCATCGGTATCATTACCCCCATGAACAGAAATCCCCCTTACACGGAGGCATCAGTGACCAAACAG
GAAAAAACCGCCCTTAACATGGCCCGCTTTATCAGAAGCCAGACATTAACGCTTCTGGAGAAACTCAACGAGCTGGA
CGCGGATGAACAGGCAGACATCTGTGAATCGCTTCACGACCACGCTGATGAGCTTTACCGCAGCTGCCTCGCGCGTT
TCGGTGATGACGGTGAAAACCTCTGACACATGCAGCTCCCGGAGACGGTCACAGCTTGTCTGTAAGCGGATGCCGGG
AGCAGACAAGCCCGTCAGGGCGCGTCAGCGGGTGTTGGCGGGTGTCGGGGCGCAGCCATGACCCAGTCACGTAGCGA
TAGCGGAGTGTATACTGGCTTAACTATGCGGCATCAGAGCAGATTGTACTGAGAGTGCACCATATATGCGGTGTGAA
ATACCGCACAGATGCGTAAGGAGAAAATACCGCATCAGGCGCTCTTCCGCTTCCTCGCTCACTGACTCGCTGCGCTC
GGTCGTTCGGCTGCGGCGAGCGGTATCAGCTCACTCAAAGGCGGTAATACGGTTATCCACAGAATCAGGGGATAACG
CAGGAAAGAACATGTGAGCAAAAGGCCAGCAAAAGGCCAGGAACCGTAAAAAGGCCGCGTTGCTGGCGTTTTTCCAT
AGGCTCCGCCCCCCTGACGAGCATCACAAAAATCGACGCTCAAGTCAGAGGTGGCGAAACCCGACAGGACTATAAAG
ATACCAGGCGTTTCCCCCTGGAAGCTCCCTCGTGCGCTCTCCTGTTCCGACCCTGCCGCTTACCGGATACCTGTCCG
CCTTTCTCCCTTCGGGAAGCGTGGCGCTTTCTCATAGCTCACGCTGTAGGTATCTCAGTTCGGTGTAGGTCGTTCGC
TCCAAGCTGGGCTGTGTGCACGAACCCCCCGTTCAGCCCGACCGCTGCGCCTTATCCGGTAACTATCGTCTTGAGTC
CAACCCGGTAAGACACGACTTATCGCCACTGGCAGCAGCCACTGGTAACAGGATTAGCAGAGCGAGGTATGTAGGCG
GTGCTACAGAGTTCTTGAAGTGGTGGCCTAACTACGGCTACACTAGAAGGACAGTATTTGGTATCTGCGCTCTGCTG
AAGCCAGTTACCTTCGGAAAAAGAGTTGGTAGCTCTTGATCCGGCAAACAAACCACCGCTGGTAGCGGTGGTTTTTT
TGTTTGCAAGCAGCAGATTACGCGCAGAAAAAAAGGATCTCAAGAAGATCCTTTGATCTTTTCTACGGGGTCTGACG
CTCAGTGGAACGAAAACTCACGTTAAGGGATTTTGGTCATGAACAATAAAACTGTCTGCTTACATAAACAGTAATAC

AAGGGGTGTTATGAGCCATATTCAACGGGAAACGTCTTGCTCTAGGCCGCGATTAAATTCCAACATGGATGCTGATT
TATATGGGTATAAATGGGCTCGCGATAATGTCGGGCAATCAGGTGCGACAATCTATCGATTGTATGGGAAGCCCGAT
GCGCCAGAGTTGTTTCTGAAACATGGCAAAGGTAGCGTTGCCAATGATGTTACAGATGAGATGGTCAGACTAAACTG
GCTGACGGAATTTATGCCTCTTCCGACCATCAAGCATTTTATCCGTACTCCTGATGATGCATGGTTACTCACCACTG
CGATCCCCGGGAAAACAGCATTCCAGGTATTAGAAGAATATCCTGATTCAGGTGAAAATATTGTTGATGCGCTGGCA
GTGTTCCTGCGCCGGTTGCATTCGATTCCTGTTTGTAATTGTCCTTTTAACAGCGATCGCGTATTTCGTCTCGCTCA
GGCGCAATCACGAATGAATAACGGTTTGGTTGATGCGAGTGATTTTGATGACGAGCGTAATGGCTGGCCTGTTGAAC
AAGTCTGGAAAGAAATGCATAAACTTTTGCCATTCTCACCGGATTCAGTCGTCACTCATGGTGATTTCTCACTTGAT
AACCTTATTTTTGACGAGGGGAAATTAATAGGTTGTATTGATGTTGGACGAGTCGGAATCGCAGACCGATACCAGGA
TCTTGCCATCCTATGGAACTGCCTCGGTGAGTTTTCTCCTTCATTACAGAAACGGCTTTTTCAAAAATATGGTATTG
ATAATCCTGATATGAATAAATTGCAGTTTCATTTGATGCTCGATGAGTTTTTCTAAGAATTAATTCATGAGCGGATA
CATATTTGAATGTATTTAGAAAAATAAACAAATAGGGGTTCCGCGCACATTTCCCCGAAAAGTGCCACCTGAAATTG
TAAACGTTAATATTTTGTTAAAATTCGCGTTAAATTTTTGTTAAATCAGCTCATTTTTTAACCAATAGGCCGAAATC
GGCAAAATCCCTTATAAATCAAAAGAATAGACCGAGATAGGGTTGAGTGTTGTTCCAGTTTGGAACAAGAGTCCACT
ATTAAAGAACGTGGACTCCAACGTCAAAGGGCGAAAAACCGTCTATCAGGGCGATGGCCCACTACGTGAACCATCAC
CCTAATCAAGTTTTTTGGGGTCGAGGTGCCGTAAAGCACTAAATCGGAACCCTAAAGGGAGCCCCCGATTTAGAGCT
TGACGGGGAAAGCCGGCGAACGTGGCGAGAAAGGAAGGGAAGAAAGCGAAAGGAGCGGGCGCTAGGGCGCTGGCAAG
TGTAGCGGTCACGCTGCGCGTAACCACCACACCCGCCGCGCTTAATGCGCCGCTACAGGGCGCGTCCCATTCGCCA

*Nucleotide sequence of pET24a plasmid containing cFos (SEQ ID NO: 47)*

ATCCGGATATAGTTCCTCCTTTCAGCAAAAAACCCCTCAAGACCCGTTTAGAGGCCCCAAGGGGTTATGCTAGTTAT
TGCTCAGCGGTGGCAGCAGCCAACTCAGCTTCCTTTCGGGCTTTGTTAGCAGCCGGATCTCAGTGGTGGTGGTGGTG
GTGCTCGAGTGCGGCCGCAAGCTTGTCGACGGAGCTCGAATTCGGATCCTTAAGGCGCGCCGAGTTTTTCCTTCTCC
TTCAGCAGGTTGGCAATTTCGGTCTGCAGGGCGTACTTCTCATCTTCCAGTTGGTCTGTCTCCGCTTGGAGTGTATC
AGTGCTAGCCATATGTATATCTCCTTCTTAAAGTTAAACAAAATTATTTCTAGAGGGGAATTGTTATCCGCTCACAA
TTCCCCTATAGTGAGTCGTATTAATTTCGCGGGATCGAGATCTCGATCCTCTACGCCGGACGCATCGTGGCCGGCAT
CACCGGCGCCACAGGTGCGGTTGCTGGCGCCTATATCGCCGACATCACCGATGGGGAAGATCGGGCTCGCCACTTCG
GGCTCATGAGCGCTTGTTTCGGCGTGGGTATGGTGGCAGGCCCCGTGGCCGGGGGACTGTTGGGCGCCATCTCCTTG
CATGCACCATTCCTTGCGGCGGCGGTGCTCAACGGCCTCAACCTACTACTGGGCTGCTTCCTAATGCAGGAGTCGCA
TAAGGGAGAGCGTCGAGATCCCGGACACCATCGAATGGCGCAAAACCTTTCGCGGTATGGCATGATAGCGCCCGGAA
GAGAGTCAATTCAGGGTGGTGAATGTGAAACCAGTAACGTTATACGATGTCGCAGAGTATGCCGGTGTCTCTTATCA
GACCGTTTCCCGCGTGGTGAACCAGGCCAGCCACGTTTCTGCGAAAACGCGGGAAAAAGTGGAAGCGGCGATGGCGG
AGCTGAATTACATTCCCAACCGCGTGGCACAACAACTGGCGGGCAAACAGTCGTTGCTGATTGGCGTTGCCACCTCC
AGTCTGGCCCTGCACGCGCCGTCGCAAATTGTCGCGGCGATTAAATCTCGCGCCGATCAACTGGGTGCCAGCGTGGT
GGTGTCGATGGTAGAACGAAGCGGCGTCGAAGCCTGTAAAGCGGCGGTGCACAATCTTCTCGCGCAACGCGTCAGTG
GGCTGATCATTAACTATCCGCTGGATGACCAGGATGCCATTGCTGTGGAAGCTGCCTGCACTAATGTTCCGGCGTTA
TTTCTTGATGTCTCTGACCAGACACCCATCAACAGTATTATTTTCTCCCATGAAGACGGTACGCGACTGGGCGTGGA
GCATCTGGTCGCATTGGGTCACCAGCAAATCGCGCTGTTAGCGGGCCCATTAAGTTCTGTCTCGGCGCGTCTGCGTC
TGGCTGGCTGGCATAAATATCTCACTCGCAATCAAATTCAGCCGATAGCGGAACGGGAAGGCGACTGGAGTGCCATG
TCCGGTTTTCAACAAACCATGCAAATGCTGAATGAGGGCATCGTTCCCACTGCGATGCTGGTTGCCAACGATCAGAT
GGCGCTGGGCGCAATGCGCGCCATTACCGAGTCCGGGCTGCGCGTTGGTGCGGATATCTCGGTAGTGGGATACGACG
ATACCGAAGACAGCTCATGTTATATCCCGCCGTTAACCACCATCAAACAGGATTTTCGCCTGCTGGGGCAAACCAGC
GTGGACCGCTTGCTGCAACTCTCTCAGGGCCAGGCGGTGAAGGGCAATCAGCTGTTGCCCGTCTCACTGGTGAAAAG

AAAAACCACCCTGGCGCCCAATACGCAAACCGCCTCTCCCCGCGCGTTGGCCGATTCATTAATGCAGCTGGCACGAC
AGGTTTCCCGACTGGAAAGCGGGCAGTGAGCGCAACGCAATTAATGTAAGTTAGCTCACTCATTAGGCACCGGGATC
TCGACCGATGCCCTTGAGAGCCTTCAACCCAGTCAGCTCCTTCCGGTGGGCGCGGGGCATGACTATCGTCGCCGCAC
TTATGACTGTCTTCTTTATCATGCAACTCGTAGGACAGGTGCCGGCAGCGCTCTGGGTCATTTTCGGCGAGGACCGC
TTTCGCTGGAGCGCGACGATGATCGGCCTGTCGCTTGCGGTATTCGGAATCTTGCACGCCCTCGCTCAAGCCTTCGT
CACTGGTCCCGCCACCAAACGTTTCGGCGAGAAGCAGGCCATTATCGCCGGCATGGCGGCCCCACGGGTGCGCATGA
TCGTGCTCCTGTCGTTGAGGACCCGGCTAGGCTGGCGGGGTTGCCTTACTGGTTAGCAGAATGAATCACCGATACGC
GAGCGAACGTGAAGCGACTGCTGCTGCAAAACGTCTGCGACCTGAGCAACAACATGAATGGTCTTCGGTTTCCGTGT
TTCGTAAAGTCTGGAAACGCGGAAGTCAGCGCCCTGCACCATTATGTTCCGGATCTGCATCGCAGGATGCTGCTGGC
TACCCTGTGGAACACCTACATCTGTATTAACGAAGCGCTGGCATTGACCCTGAGTGATTTTTCTCTGGTCCCGCCGC
ATCCATACCGCCAGTTGTTTACCCTCACAACGTTCCAGTAACCGGGCATGTTCATCATCAGTAACCCGTATCGTGAG
CATCCTCTCTCGTTTCATCGGTATCATTACCCCCATGAACAGAAATCCCCCTTACACGGAGGCATCAGTGACCAAAC
AGGAAAAAACCGCCCTTAACATGGCCCGCTTTATCAGAAGCCAGACATTAACGCTTCTGGAGAAACTCAACGAGCTG
GACGCGGATGAACAGGCAGACATCTGTGAATCGCTTCACGACCACGCTGATGAGCTTTACCGCAGCTGCCTCGCGCG
TTTCGGTGATGACGGTGAAAACCTCTGACACATGCAGCTCCCGGAGACGGTCACAGCTTGTCTGTAAGCGGATGCCG
GGAGCAGACAAGCCCGTCAGGGCGCGTCAGCGGGTGTTGGCGGGTGTCGGGGCGCAGCCATGACCCAGTCACGTAGC
GATAGCGGAGTGTATACTGGCTTAACTATGCGGCATCAGAGCAGATTGTACTGAGAGTGCACCATATATGCGGTGTG
AAATACCGCACAGATGCGTAAGGAGAAAATACCGCATCAGGCGCTCTTCCGCTTCCTCGCTCACTGACTCGCTGCGC
TCGGTCGTTCGGCTGCGGCGAGCGGTATCAGCTCACTCAAAGGCGGTAATACGGTTATCCACAGAATCAGGGGATAA
CGCAGGAAAGAACATGTGAGCAAAAGGCCAGCAAAAGGCCAGGAACCGTAAAAAGGCCGCGTTGCTGGCGTTTTTCC
ATAGGCTCCGCCCCCCTGACGAGCATCACAAAAATCGACGCTCAAGTCAGAGGTGGCGAAACCCGACAGGACTATAA
AGATACCAGGCGTTTCCCCCTGGAAGCTCCCTCGTGCGCTCTCCTGTTCCGACCCTGCCGCTTACCGGATACCTGTC
CGCCTTTCTCCCTTCGGGAAGCGTGGCGCTTTCTCATAGCTCACGCTGTAGGTATCTCAGTTCGGTGTAGGTCGTTC
GCTCCAAGCTGGGCTGTGTGCACGAACCCCCCGTTCAGCCCGACCGCTGCGCCTTATCCGGTAACTATCGTCTTGAG
TCCAACCCGGTAAGACACGACTTATCGCCACTGGCAGCAGCCACTGGTAACAGGATTAGCAGAGCGAGGTATGTAGG
CGGTGCTACAGAGTTCTTGAAGTGGTGGCCTAACTACGGCTACACTAGAAGGACAGTATTTGGTATCTGCGCTCTGC
TGAAGCCAGTTACCTTCGGAAAAAGAGTTGGTAGCTCTTGATCCGGCAAACAAACCACCGCTGGTAGCGGTGGTTTT
TTTGTTTGCAAGCAGCAGATTACGCGCAGAAAAAAAGGATCTCAAGAAGATCCTTTGATCTTTTCTACGGGGTCTGA
CGCTCAGTGGAACGAAAACTCACGTTAAGGGATTTTGGTCATGAACAATAAAACTGTCTGCTTACATAAACAGTAAT
ACAAGGGGTGTTATGAGCCATATTCAACGGGAAACGTCTTGCTCTAGGCCGCGATTAAATTCCAACATGGATGCTGA
TTTATATGGGTATAAATGGGCTCGCGATAATGTCGGGCAATCAGGTGCGACAATCTATCGATTGTATGGGAAGCCCG
ATGCGCCAGAGTTGTTTCTGAAACATGGCAAAGGTAGCGTTGCCAATGATGTTACAGATGAGATGGTCAGACTAAAC
TGGCTGACGGAATTTATGCCTCTTCCGACCATCAAGCATTTTATCCGTACTCCTGATGATGCATGGTTACTCACCAC
TGCGATCCCCGGGAAAACAGCATTCCAGGTATTAGAAGAATATCCTGATTCAGGTGAAAATATTGTTGATGCGCTGG
CAGTGTTCCTGCGCCGGTTGCATTCGATTCCTGTTTGTAATTGTCCTTTTAACAGCGATCGCGTATTTCGTCTCGCT
CAGGCGCAATCACGAATGAATAACGGTTTGGTTGATGCGAGTGATTTTGATGACGAGCGTAATGGCTGGCCTGTTGA
ACAAGTCTGGAAAGAAATGCATAAACTTTTGCCATTCTCACCGGATTCAGTCGTCACTCATGGTGATTTCTCACTTG
ATAACCTTATTTTTGACGAGGGGAAATTAATAGGTTGTATTGATGTTGGACGAGTCGGAATCGCAGACCGATACCAG
GATCTTGCCATCCTATGGAACTGCCTCGGTGAGTTTTCTCCTTCATTACAGAAACGGCTTTTTCAAAAATATGGTAT
TGATAATCCTGATATGAATAAATTGCAGTTTCATTTGATGCTCGATGAGTTTTTCTAAGAATTAATTCATGAGCGGA
TACATATTTGAATGTATTTAGAAAAATAAACAAATAGGGGTTCCGCGCACATTTCCCCGAAAAGTGCCACCTGAAAT
TGTAAACGTTAATATTTTGTTAAAATTCGCGTTAAATTTTTGTTAAATCAGCTCATTTTTTAACCAATAGGCCGAAA
TCGGCAAAATCCCTTATAAATCAAAAGAATAGACCGAGATAGGGTTGAGTGTTGTTCCAGTTTGGAACAAGAGTCCA

CTATTAAAGAACGTGGACTCCAACGTCAAAGGGCGAAAAACCGTCTATCAGGGCGATGGCCCACTACGTGAACCATC
ACCCTAATCAAGTTTTTTGGGGTCGAGGTGCCGTAAAGCACTAAATCGGAACCCTAAAGGGAGCCCCCGATTTAGAG
CTTGACGGGGAAAGCCGGCGAACGTGGCGAGAAAGGAAGGGAAGAAAGCGAAAGGAGCGGGCGCTAGGGCGCTGGCA
AGTGTAGCGGTCACGCTGCGCGTAACCACCACACCCGCCGCGCTTAATGCGCCGCTACAGGGCGCGTCCCATTCGCC
A

| | | |
|---|---|---|
| CAAT box | GGCCAATCT | (SEQ ID NO: 48) |
| CArG box | CC(A/T$_6$GG | (SEQ ID NO: 49) |
| E2 box | CAGGTG and CACCTG | (SEQ ID NOs: 50 and 51) |
| HY box | TG(A/T)GGG | (SEQ ID NO: 52) |
| T box | TCACACCT | (SEQ ID NO: 53) |
| TATA box | TATAAA | (SEQ ID NO: 54) |
| X box | GTTGGCATGGCAAC | (SEQ ID NO: 55) |
| Y box | (A/G) CTAACC (A/G) (A/G) (C/T) | (SEQ ID NO: 56) |
| ATA box | AAATAT | (SEQ ID NO: 57) |
| CGCG box | (A/C/G) CGCG (C/G/T) | (SEQ ID NO: 58) |
| DREB box | TACCGACAT | (SEQ ID NO: 59) |
| Fur box | GATAATGATAATCATTATC | (SEQ ID NO: 60) |
| G box | GCCACGTGGC | (SEQ ID NO: 61) |
| GCC box | AGCCGCC | (SEQ ID NO: 62) |
| H box | ACACCA | (SEQ ID NO: 63) |
| Prolamin box | TGTAAAG | (SEQ ID NO: 64) |
| Pyrimidine box | CCTTTT | (SEQ ID NO: 65) |
| TACTAAC box | ATTTACTAAC | (SEQ ID NO: 66) |

## Claims

**1.** A method for screening for an antagonist of a DNA-binding protein, the method comprising:

i) providing a cell, wherein the cell comprises a test compound, a DNA-binding protein, and a reporter expression cassette that encodes a reporter expression product,
wherein the reporter expression cassette comprises at least one binding site for the DNA-binding protein such that binding of the DNA-binding protein to the binding site inhibits expression of the reporter expression product; and
ii) determining expression of the reporter expression product in the presence of the test compound;
wherein an increase in expression of the reporter expression product in the presence of the test compound indicates that the test compound is capable of inhibiting DNA-binding activity of the DNA-binding protein, and wherein some or all of the binding site(s) are located in the transcribed sequence of the reporter expression cassette.

**2.** The method of claim 1, wherein the reporter expression product is a reporter protein, optionally wherein the reporter protein is a cell survival protein, a cell reproduction protein a fluorescent protein, a bioluminescent protein, a protease, an enzyme that acts on a substrate to produce a colorimetric signal, a protein kinase, a transcriptional activator, or a regulatory protein such as ubiquitin.

**3.** The method of claim 2, wherein the reporter protein is a cell survival protein, optionally wherein the cell survival protein is an exogenous cell survival protein that is able to compensate for a deficiency in an endogenous cell survival protein; and
wherein the method is performed under selection conditions such that survival of the cell is dependent upon activity of the exogenous cell survival protein.

**4.** The method of claim 3, wherein the cell survival protein is dihydrofolate reductase (DHFR), optionally wherein the

DHFR has an amino acid sequence that is at least 80% identical to the sequence set forth in SEQ ID NO: 1.

**5.** The method of any one of the preceding claims, wherein:

i) the reporter expression cassette comprises between 1 and 5, between 1 and 10, between 1 and 15, between 1 and 20, between 5 and 10, between 5 and 15, between 5 and 20, between 10 and 15, between 10 and 20, between 10 and 18 or between 12 and 16 binding sites; and/or
ii) some or all of the binding site(s) are located in the protein coding sequence of the reporter expression cassette.

**6.** The method of any one of the preceding claims, wherein the DNA-binding protein is a transcription factor or a DNA-binding fragment thereof, optionally wherein the DNA-binding protein is a eukaryotic transcription factor or a DNA-binding fragment thereof.

**7.** The method of any one of the preceding claims, wherein the DNA-binding protein is a basic leucine zipper (bZIP) transcription factor, a basic helix-loop helix (bHLH) transcription factor, a bHLH leucine zipper (bHLH-ZIP) transcription factor, or a DNA-binding fragment thereof, and optionally wherein:

a) the at least one binding site is a TPA response element (TRE) having the nucleotide sequence **TGACTCA** (**SEQ ID NO: 5**) or **TGAGTCA** (**SEQ ID NO: 6**);
b) the at least one binding site is an Ebox response element having the nucleotide sequence **CACGTG** (**SEQ ID NO: 7**) or **CACATG (SEQ ID NO: 8);**
c) the at least one binding site is a CCAAT binding site having the nucleotide sequence **ATTGCGCAAT** (**SEQ ID NO: 9**);
d) the at least one binding site is a cAMP response element (CRE) having the nucleotide sequence **TGACGTCA (SEQ ID NO: 10);**
e) the at least one binding site is a Maf recognition element (MARE) having the nucleotide sequence **TGCTGA$^{G}/_{C}$TCAGCA** (**SEQ ID NO: 32**) or **TGCTGA$^{GC}/_{CG}$TCAGCA** **(SEQ ID NO: 33);** or
f) the at least one binding site is a PAP/CREB-2/PAR binding site having the nucleotide sequence **TTACGTAA** (**SEQ ID NO: 34**).

**8.** The method of any one of the preceding claims, wherein:

i) the cell is a bacterial cell, optionally an *Escherichia coli* cell; or
ii) the cell is a eukaryotic cell, optionally a mammalian cell.

**9.** The method of claim 8 ii), wherein the eukaryotic cell was isolated from a human patient and wherein the DNA-binding protein is naturally produced by the cell, and optionally wherein the DNA-binding protein is suspected of being or known to be dysregulated in the cell.

**10.** The method of any one of claims 1 to 8, wherein the method comprises administering a DNA-binding protein expression cassette that encodes the DNA-binding protein in order to provide the cell comprising the DNA-binding protein.

**11.** The method of any one of the preceding claims, wherein the test compound is a peptidic test compound.

**12.** The method of claim 11, wherein the peptidic test compound is expressed intracellularly from a test compound expression cassette, optionally wherein the method comprises providing the test compound expression cassette to the cell.

**13.** The method of claim 11 or claim 12, wherein the method comprises administering a cross-linking agent into the cell in order to introduce a cross-link between two amino acid residues in an alpha helix of the peptidic test compound to produce a helix-constrained peptidic compound.

**14.** The method of any one of claims 1 to 11, wherein the method comprises administering the test compound extracellularly in order to provide the cell comprising the test compound, optionally wherein the test compound is a peptidic test compound, wherein the peptidic test compound comprises a helix-constrained peptide, and wherein the helix-constrained peptide comprises a cross-link between two amino acid residues.

**15.** A cell-free method for screening for an antagonist of a DNA-binding protein, the method comprising:

i) contacting a test compound with a DNA-binding protein and a reporter expression cassette that encodes a reporter expression product,
wherein the reporter expression cassette comprises at least one binding site for the DNA-binding protein such that binding of the DNA-binding protein to the binding site inhibits expression of the reporter expression product; and
ii) determining expression of the reporter expression product;
wherein an increase in expression of the reporter expression product in the presence of the test compound indicates that the test compound is capable of inhibiting DNA-binding activity of the DNA-binding protein,
wherein the method is carried out outside a cell in an *in vitro* system that comprises the components required for expression of the reporter expression product, and

wherein some or all of the binding site(s) are located in the transcribed sequence of the reporter expression cassette.

**Patentansprüche**

**1.** Verfahren zum Screening nach einem Antagonisten eines DNA-bindenden Proteins, wobei das Verfahren Folgendes umfasst:

i) Bereitstellen einer Zelle, wobei die Zelle eine Testverbindung, ein DNA-bindendes Protein und eine Reporterexpressionskassette, die für ein Reporterexpressionsprodukt kodiert, umfasst,
wobei die Reporterexpressionskassette zumindest eine Bindungsstelle für das DNA-bindende Protein umfasst, sodass das Binden des DNA-bindenden Proteins an die Bindungsstelle die Expression des Reporterexpressionsprodukts hemmt; und
ii) Bestimmen der Expression des Reporterexpressionsprodukts in Gegenwart der Testverbindung;
wobei ein Anstieg der Expression des Reporterexpressionsprodukts in Gegenwart der Testverbindung anzeigt, dass die Testverbindung in der Lage ist, die DNA-Bindungsaktivität des DNA-bindenden Proteins zu hemmen, und
wobei ein Teil oder die Gesamtheit der Bindungsstelle(n) sich in der transkribierten Sequenz der Reporterexpressionskassette befindet.

**2.** Verfahren nach Anspruch 1, wobei das Reporterexpressionsprodukt ein Reporterprotein ist, wobei das Reporterprotein gegebenenfalls ein Protein für Zellüberleben, ein Protein für Zellreproduktion, ein fluoreszierendes Protein, ein biolumineszierendes Protein, eine Protease, ein Enzym, das auf einem Substrat wirkt, um ein kolorimetrisches Signal zu erzeugen, eine Proteinkinase, ein Transkriptionsaktivator oder ein regulatorisches Protein wie Ubiquitin ist.

**3.** Verfahren nach Anspruch 2, wobei das Reporterprotein ein Protein für Zellüberleben ist, wobei das Protein für Zellüberleben gegebenenfalls ein exogenes Protein für Zellüberleben ist, das in der Lage ist, einen Mangel an endogenem Protein für Zellüberleben zu kompensieren; und
wobei das Verfahren unter derartigen Selektionsbedingungen durchgeführt wird, dass das Überleben der Zelle von der Aktivität des exogenen Proteins für Zellüberleben abhängig ist.

**4.** Verfahren nach Anspruch 3, wobei das Protein für Zellüberleben Dihydrofolatreduktase (DHFR) ist, wobei die DHFR gegebenenfalls eine Aminosäuresequenz aufweist, die zumindest zu 80 % identisch mit der in SEQ ID NO:1 dargelegten Sequenz ist.

**5.** Verfahren nach einem der vorangegangenen Ansprüche, wobei:

1) die Reporterexpressionskassette zwischen 1 und 5, zwischen 1 und 10, zwischen 1 und 15, zwischen 1 und 20, zwischen 5 und 10, zwischen 5 und 15, zwischen 5 und 20, zwischen 10 und 15, zwischen 10 und 20, zwischen 10 und 18 oder zwischen 12 und 16 Bindungsstellen umfasst; und/oder
ii) ein Teil oder die Gesamtheit der Bindungsstelle(n) sich in der Proteinkodierungssequenz der Reporterexpressionskassette befindet.

**6.** Verfahren nach einem der vorangegangenen Ansprüche, wobei das DNA-bindende Protein ein Transkriptionsfaktor oder ein DNA-bindendes Fragment davon ist, wobei das DNA-bindende Protein gegebenenfalls ein eukaryotischer

Transkriptionsfaktor oder ein DNA-bindendes Fragment davon ist.

7. Verfahren nach einem der vorangegangenen Ansprüche, wobei das DNA-bindende Protein ein Basic-Leucin-Zipper-(bZIP-) Transkriptionsfaktor, ein Basic-Helix-Loop-Helix-(bHLH-) Transkriptionsfaktor, ein bHLH-Leucin-Zipper-(bHLH-ZIP-) Transkriptionsfaktor oder ein DNA-bindendes Fragment davon ist und wobei gegebenenfalls:

a) die zumindest eine Bindungsstelle ein TPA-Response-Element (TRE) mit der Nucleotidsequenz TGACTCA (SEQ ID NO: 5) oder TGAGTCA (SEQ ID NO: 6) ist;
b) die zumindest eine Bindungsstelle ein Ebox-Response-Element mit der Nucleotidsequenz CACGTG (SEQ ID NO: 7) oder CACATG (SEQ ID NO: 8) ist;
c) die zumindest eine Bindungsstelle eine CCAAT-Bindungsstelle mit der Nucleotidsequenz ATTGCGCAAT (SEQ ID NO: 9) ist;
d) die zumindest eine Bindungsstelle ein cAMP-Response-Element (CRE) mit der Nucleotidsequenz TGACGTCA (SEQ ID NO: 10) ist;
e) die zumindest eine Bindungsstelle ein Maf-Erkennungselement (MARE) mit der Nucleotidsequenz TGCTGA$^{G}/_{C}$TCAGCA (SEQ ID NO: 32) oder TGCTGA$^{GC}/_{CG}$TCAGCA (SEQ ID NO: 33) ist; oder
f) die zumindest eine Bindungsstelle eine PAP/CREB-2/PAR-Bindungsstelle mit der Nucleotidsequenz TTACGTAA (SEQ ID NO: 34) ist.

8. Verfahren nach einem der vorangegangenen Ansprüche, wobei:

(i) die Zelle eine Bakterienzelle, gegebenenfalls eine *Escherichia-coli*-Zelle, ist; oder
(ii) die Zelle eine eukaryotische Zelle, gegebenenfalls eine Säugetierzelle, ist.

9. Verfahren nach Anspruch 8 ii), wobei die eukaryotische Zelle von einem menschlichen Patienten isoliert wurde und wobei das DNA-bindende Protein auf natürliche Weise durch die Zelle erzeugt wird und wobei gegebenenfalls der Verdacht besteht oder nachgewiesen ist, dass das DNA-bindende Protein in der Zelle fehlreguliert ist.

10. Verfahren nach einem der Ansprüche 1 bis 8, wobei das Verfahren das Verabreichen einer Expressionskassette des DNA-bindenden Proteins, die für das DNA-bindende Protein kodiert, umfasst, um die Zelle bereitzustellen, die das DNA-bindende Protein umfasst.

11. Verfahren nach einem der vorangegangenen Ansprüche, wobei die Testverbindung eine Peptid-Testverbindung ist.

12. Verfahren nach Anspruch 11, wobei die Peptid-Testverbindung intrazellulär aus einer Testverbindungsexpressionskassette exprimiert wird, wobei das Verfahren gegebenenfalls das Bereitstellen der Testverbindungsexpressionskassette für die Zelle umfasst.

13. Verfahren nach Anspruch 11 oder 12, wobei das Verfahren das Verabreichen eines Vernetzungsmittels in die Zelle umfasst, um eine Vernetzung zwischen zwei Aminosäureresten in einer $\alpha$-Helix der Peptidtestverbindung einzuführen, um eine Helix-eingeschränkte Peptidverbindung herzustellen.

14. Verfahren nach einem der Ansprüche 1 bis 11, wobei das Verfahren das extrazelluläre Verabreichen der Testverbindung umfasst, um die die Testverbindung umfassende Zelle bereitzustellen, wobei die Testverbindung gegebenenfalls eine Peptidtestverbindung ist, wobei die Peptidtestverbindung ein Helix-eingeschränktes Peptid umfasst und wobei das Helix-eingeschränkte Peptid eine Vernetzung zwischen zwei Aminosäureresten umfasst.

15. Zellfreies Verfahren zum Screening nach einem Antagonisten eines DNA-bindenden Proteins, wobei das Verfahren Folgendes umfasst:

i) In-Kontakt-Bringen einer Testverbindung mit einem DNA-bindenden Protein und einer Reporterexpressionskassette, die für ein Reporterexpressionsprodukt kodiert,
wobei die Reporterexpressionskassette zumindest eine Bindungsstelle für das DNA-bindende Protein umfasst, sodass das Binden des DNA-bindenden Proteins an die Bindungsstelle die Expression des Reporterexpressionsprodukts hemmt; und
ii) Bestimmen der Expression des Reporterexpressionsprodukts,
wobei ein Anstieg der Expression des Reporterexpressionsprodukts in Gegenwart der Testverbindung anzeigt,

dass die Testverbindung in der Lage ist, die DNA-Bindungsaktivität des DNA-bindenden Proteins zu hemmen, wobei das Verfahren außerhalb einer Zelle in einem In-vitro-System durchgeführt wird, das die Komponenten umfasst, welche zur Expression des Reporterexpressionsprodukts erforderlich sind, und
wobei ein Teil oder die Gesamtheit der Bindungsstelle(n) sich in der transkribierten Sequenz der Reporterexpressionskassette befindet.

**Revendications**

1. Procédé de criblage d'un antagoniste d'une protéine de liaison à l'ADN, le procédé comprenant les étapes consistant à :

   i) fournir une cellule, dans lequel la cellule comprend un composé de test, une protéine de liaison à l'ADN et une cassette d'expression de rapporteur qui code pour un produit d'expression de rapporteur,
   dans lequel la cassette d'expression de rapporteur comprend au moins un site de liaison pour la protéine de liaison à l'ADN de telle sorte que la liaison de la protéine de liaison à l'ADN au site de liaison inhibe l'expression du produit d'expression de rapporteur ; et
   ii) déterminer l'expression du produit d'expression de rapporteur en présence du composé de test ;
   dans lequel une augmentation de l'expression du produit d'expression de rapporteur en présence du composé de test indique que le composé de test est capable d'inhiber l'activité de liaison à l'ADN de la protéine de liaison à l'ADN, et
   dans lequel certains ou tous les sites de liaison sont situés dans la séquence transcrite de la cassette d'expression de rapporteur.

2. Procédé selon la revendication 1, dans lequel le produit d'expression de rapporteur est une protéine rapporteur, facultativement dans lequel la protéine rapporteur est une protéine de survie de cellule, une protéine de reproduction cellulaire, une protéine fluorescente, une protéine bioluminescente, une protéase, une enzyme qui agit sur un substrat pour produire un signal colorimétrique, une protéine kinase, un activateur transcriptionnel ou une protéine régulatrice telle que l'ubiquitine.

3. Procédé selon la revendication 2, dans lequel la protéine rapporteur est une protéine de survie de cellule, facultativement dans lequel la protéine de survie de cellule est une protéine de survie de cellule exogène qui est capable de compenser une carence en une protéine de survie de cellule endogène ; et
   dans lequel le procédé est effectué dans des conditions de sélection telles que la survie de la cellule dépend de l'activité de la protéine de survie de cellule exogène.

4. Procédé selon la revendication 3, dans lequel la protéine de survie de cellule est la dihydrofolate réductase (DHFR), facultativement dans lequel la DHFR a une séquence d'acides aminés qui est au moins 80 % identique à la séquence présentée dans SEQ ID NO : 1.

5. Procédé selon l'une quelconque des revendications précédentes, dans lequel :

   i) la cassette d'expression de rapporteur comprend entre 1 et 5, entre 1 et 10, entre 1 et 15, entre 1 et 20, entre 5 et 10, entre 5 et 15, entre 5 et 20, entre 10 et 15, entre 10 et 20, entre 10 et 18 ou entre 12 et 16 sites de liaison ; et/ou
   ii) certains ou tous les sites de liaison sont situés dans la séquence de codage de protéine de la cassette d'expression de rapporteur.

6. Procédé selon l'une quelconque des revendications précédentes, dans lequel la protéine de liaison à l'ADN est un facteur de transcription ou un fragment de liaison à l'ADN de celui-ci, facultativement dans lequel la protéine de liaison à l'ADN est un facteur de transcription eucaryote ou un fragment de liaison à l'ADN de celui-ci.

7. Procédé selon l'une quelconque des revendications précédentes, dans lequel la protéine de liaison à l'ADN est un facteur de transcription de glissière à leucine basique(bZIP), un facteur de transcription d'hélice-boucle basique (bHLH), un facteur de transcription de fglissière à leucine bHLH (bHLH-ZIP), ou un fragment de liaison à l'ADN de ceux-ci, et facultativement dans lequel :

   a) le au moins un site de liaison est un élément de réponse TPA (TRE) ayant la séquence nucléotidique

**TGACTCA (SEQ ID NO** : **5**) ou **TGAGTCA (SEQ ID NO: 6)** ;
b) le au moins un site de liaison est un élément de réponse Ebox ayant la séquence nucléotidique **CACGTG(SEQ ID NO : 7**) ou **CACTG(SEQ ID NO: 8)** ;
c) le au moins un site de liaison est un site de liaison CCAAT ayant la séquence nucléotidique **ATTGCGCAAT(SEQ ID NO : 9)** ;
d) le au moins un site de liaison est un élément de réponse cAMP(CRE) ayant la séquence nucléotidique **TGACGTCA (SEQ ID NO** : **10)** ;
e) le au moins un site de liaison est un élément de reconnaissance Maf (MARE) ayant la séquence nucléotidique **TGCTGAG/cTCAGCA (SEQ ID NO : 32**) ou **TGCTGAGc/cGTCAGCA (SEQ ID NO:** 33) ; ou
f) le au moins un site de liaison est un site de liaison PAP/CREB-2/PAR ayant la séquence nucléotidique **TTACGTAA(SEQ ID NO : 34).**

**8.** Procédé selon l'une quelconque des revendications précédentes, dans lequel :

i) la cellule est une cellule bactérienne, facultativement une cellule d'*Escherichia coli* ; ou
ii) la cellule est une cellule eucaryote, facultativement une cellule de mammifère.

**9.** Procédé selon la revendication 8 ii), dans lequel la cellule eucaryote a été isolée d'un patient humain et dans lequel la protéine de liaison à l'ADN est produite naturellement par la cellule, et facultativement dans lequel la protéine de liaison à l'ADN est suspectée d'être ou connue pour être dérégulée dans la cellule.

**10.** Procédé selon l'une quelconque des revendications 1 à 8, dans lequel le procédé comprend l'administration d'une cassette d'expression de protéine de liaison à l'ADN qui code pour la protéine de liaison à l'ADN afin de fournir la cellule comprenant la protéine de liaison à l'ADN.

**11.** Procédé selon l'une quelconque des revendications précédentes, dans lequel le composé de test est un composé de test peptidique.

**12.** Procédé selon la revendication 11, dans lequel le composé de test peptidique est exprimé de manière intracellulaire à partir d'une cassette d'expression de composé de test, facultativement dans lequel le procédé comprend la fourniture de la cassette d'expression de composé de test à la cellule.

**13.** Procédé selon la revendication 11 ou la revendication 12, dans lequel le procédé comprend l'administration d'un agent de réticulation dans la cellule afin d'introduire une réticulation entre deux résidus d'acides aminés dans une hélice alpha du composé de test peptidique pour produire un composé peptidique contraint en hélice.

**14.** Procédé selon l'une quelconque des revendications 1 à 11, dans lequel le procédé comprend l'administration du composé de test de manière extracellulaire afin de fournir la cellule comprenant le composé de test, facultativement dans lequel le composé de test est un composé de test peptidique, dans lequel le composé de test peptidique comprend un peptide contraint en hélice, et dans lequel le peptide contraint en hélice comprend une réticulation entre deux résidus d'acides aminés.

**15.** Procédé acellulaire de criblage d'un antagoniste d'une protéine de liaison à l'ADN, le procédé comprenant les étapes consistant à :

i) mettre en contact un composé de test avec une protéine de liaison à l'ADN et une cassette d'expression de rapporteur qui code pour un produit d'expression de rapporteur,
dans lequel la cassette d'expression de rapporteur comprend au moins un site de liaison pour la protéine de liaison à l'ADN de telle sorte que la liaison de la protéine de liaison à l'ADN au site de liaison inhibe l'expression du produit d'expression de rapporteur ; et
ii) déterminer l'expression du produit d'expression de rapporteur ;
dans lequel une augmentation de l'expression du produit d'expression de rapporteur en présence du composé de test indique que le composé de test est capable d'inhiber l'activité de liaison à l'ADN de la protéine de liaison à l'ADN,
dans lequel le procédé est effectué à l'extérieur d'une cellule dans un système in vitro qui comprend les composants nécessaires à l'expression du produit d'expression de rapporteur, et
dans lequel certains ou tous les sites de liaison sont situés dans la séquence transcrite de la cassette d'expression de rapporteur.

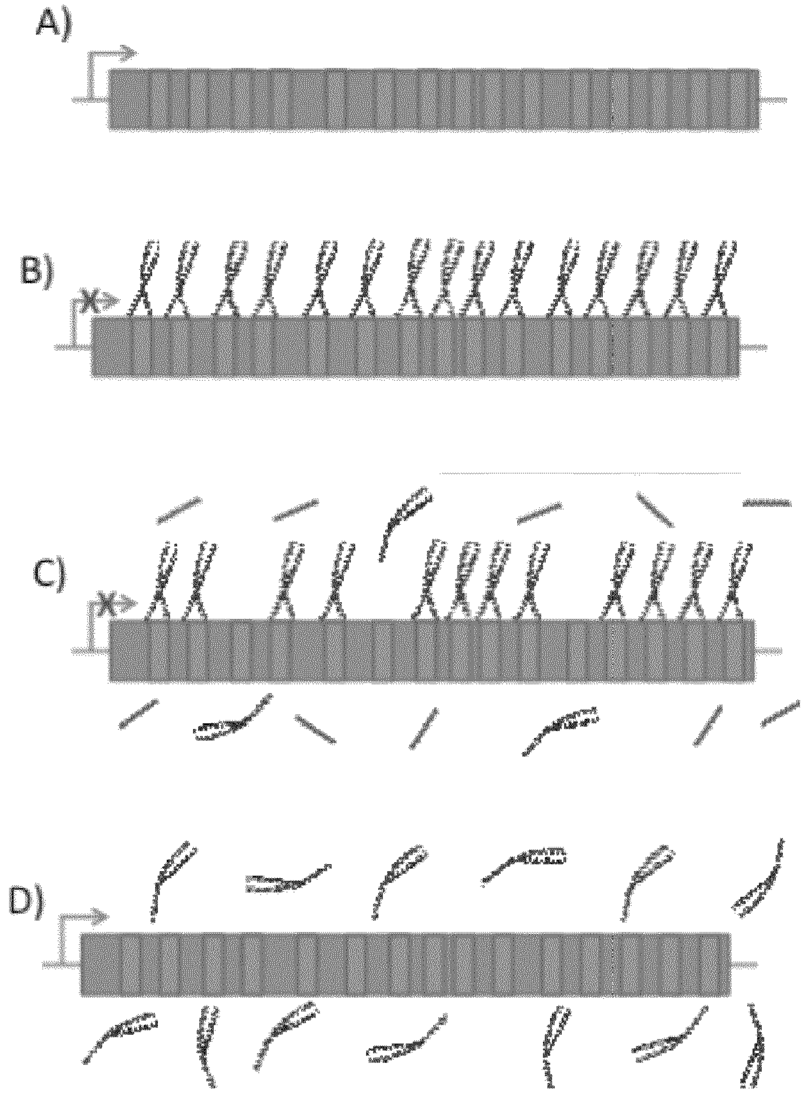


*Figure 1*

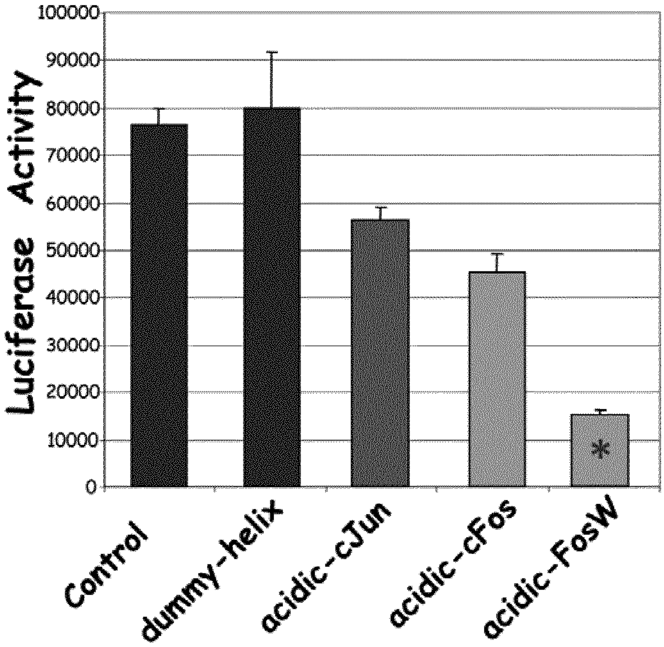
Luciferase Activity
100000
90000
80000
70000
60000
50000
40000
30000
20000
10000
0
*
Control
dummy-helix
acidic-cJun
acidic-cFos
acidic-FosW

*Figure 2*

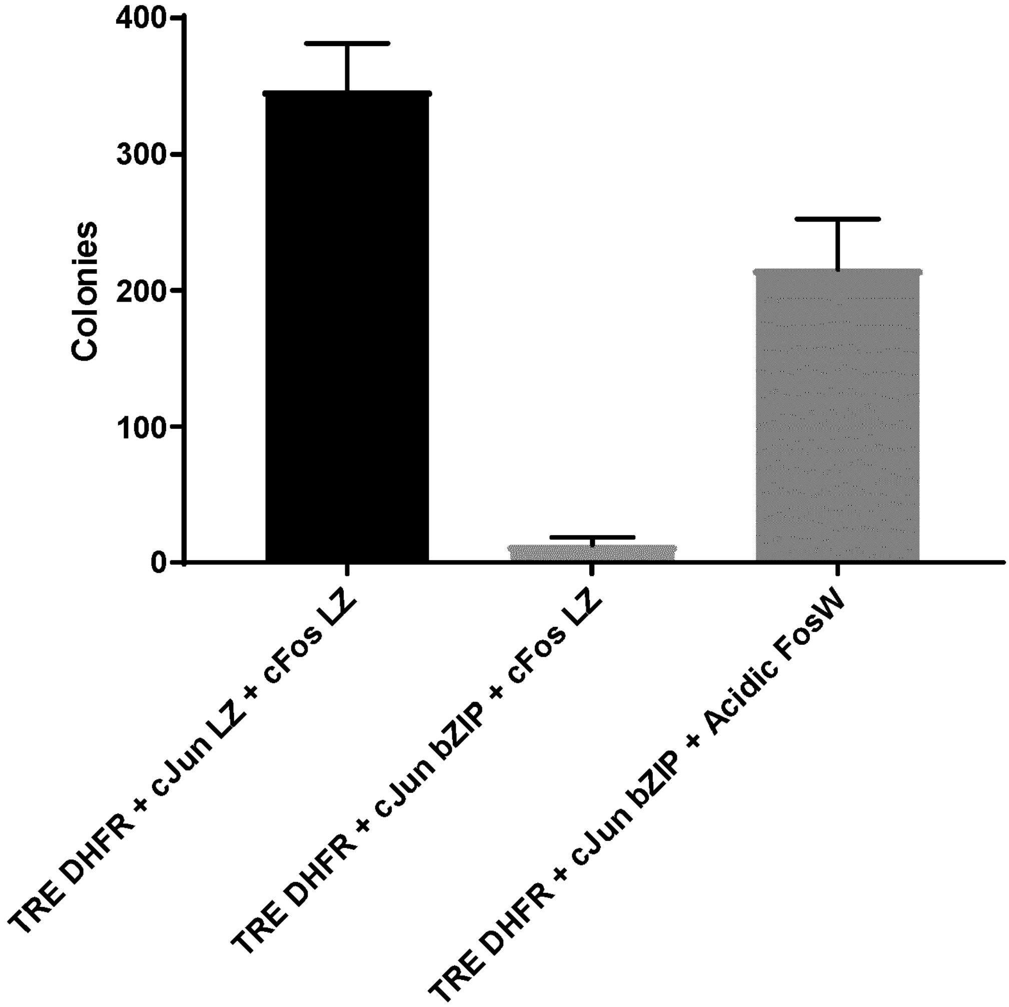
400
300
200
100
0
Colonies
TRE DHFR + cJun LZ + cFos LZ
TRE DHFR + cJun bZIP + cFos LZ
TRE DHFR + cJun bZIP + Acidic FosW

*Figure 3*

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2018075486 A1
- FR 2861742
- WO 9913069 A1
- WO 0159450 A2
- WO 9957535 A2
- WO 2008110784 A1


### Non-patent literature cited in the description


- **STANLEY SABRINA Y R.** An Investigation of Bacteriophage Anti-CRISPR and Anti-CRISPR Associated Proteins. Thesis University of Toronto, 01 November 2018
- **SAMBROOK et al.** Molecular Cloning: A Laboratory Manual. Cold Spring Harbor Press, 1989
- **CABEZAS, E. ; SATTERTHWAIT, A. C.** *J. Am. Chem. Soc.,* 1999, vol. 121, 3862
- **CODY, V. et al.** New insights into DHFR interactions: Analysis of Pneumocystis carinii and mouse DHFR complexes with NADPH and two highly potent 5-(omega-carboxy(alkyloxy) trimethoprim derivatives reveals conformational correlations with activity and novel parallel ring stacking interactions. *Proteins,* 2006, vol. 65 (4), 959-969
- **DE ARAUJO A.D. et al.** Comparative α-helicity of cyclic pentapeptides in water. *Angew Chem Int Ed Engl.,* 2014, vol. 53 (27), 6965-9
- **FUJIMOTO, K. et al.** Development of a series of cross-linking agents that effectively stabilize alpha-helical structures in various short peptides. *Chemistry,* 2008, vol. 14 (3), 857-63
- **GRITZ L ; DAVIES J. ; 1983.** Plasmid-encoded hygromycin B resistance: the sequence of hygromycin B phosphotransferase gene and its expression in Escherichia coli and Saccharomyces cerevisiae. *Gene,* vol. 25 (2-3), 179-88
- **HANEY, C.M. et al.** Promoting peptide α-helix formation with dynamic covalent oxime side-chain cross-links. *Chem Commun (Camb),* 2011, vol. 47 (39), 10915-7
- **HOLLAND-NELL, K. ; MELDAL, M.** Maintaining biological activity by using triazoles as disulfide bond mimetics. *Angew Chem Int Ed Engl.,* vol. 50 (22), 5204-6
- **HEINIS, C. et al.** Phage-encoded combinatorial chemical libraries based on bicyclic peptides. *Nat Chem Biol.,* 2009, vol. 5 (7), 502-507
- **JO, H. et al.** Development of α-helical calpain probes by mimicking a natural protein-protein interaction. *J Am Chem Soc.,* 2012, vol. 134 (42), 17704-17713
- **JOCHIM, A.L. ; ARORA, P.S.** Systematic analysis of helical protein interfaces reveals targets for synthetic inhibitors. ACS. *Chem. Biol.,* 2010, vol. 5, 919-923
- **KAUFMAN et al.** Selection and amplification of heterologous genes encoding adenosine deaminase in mammalian cells. *Proc Natl Acad Sci USA.,* 1986, vol. 83 (10), 3136-3140
- **LEDUC, A.M. et al.** Helix-stabilized cyclic peptides as selective inhibitors of steroid receptor-coactivator interactions. *Proc Natl Acad Sci USA.,* 2003, vol. 100 (20), 11273-8
- **MA. Y. et al.** Split focal adhesion kinase for probing protein-protein interactions. *Biochemical Engineering Journal.,* 2014, vol. 90, 272-278
- **MASON, J. et al.** Semirational design of Jun-Fos coiled coils with increased affinity: Universal implications for leucine zipper prediction and design. *Proc Natl Acad Sci USA.,* 2006, vol. 103 (24), 8989-94
- **MCCORMICK, F.** KRAS as a Therapeutic Target. *Clin Cancer Res.,* 2015, vol. 21 (8), 1797-1801
- **MERN, D.S. et al.** Inhibition of Id proteins by a peptide aptamer induces cell-cycle arrest and apoptosis in ovarian cancer cells. *Br J Cancer.,* 2010, vol. 103 (8), 1237-1244
- **MIRANDA, E. et al.** Deciphering interactions used by the influenza virus NS1 protein to silence the host antiviral sensor protein RIG-I using a bacterial reverse two-hybrid system. *Mol. BioSyst.,* 2011, vol. 7, 1042-1045
- **MUPPIDI, A. et al.** Achieving cell penetration with distance-matching cysteine cross-linkers: a facile route to cell-permeable peptide dual inhibitors of Mdm2/Mdmx. *Chem Commun (Camb).,* 2011, vol. 47 (33), 9396-8
- **NEWMAN ; KEATING.** Comprehensive identification of human bZIP interactions with coiled-coil arrays. *Science,* 2003, vol. 300 (5628), 2097-101

- **OLIVE, M. et al.** A Dominant Negative to Activation Protein-1 (AP1) That Abolishes DNA Binding and Inhibits Oncogenesis. *J Biol Chem.,* 1997, vol. 272 (30), 18586-94
- **PARK, J.H.** Bacterial beta-lactamase fragmentation complementation strategy can be used as a method for identifying interacting protein pairs. *Journal of Microbiology and Biotechnology.,* 2007, vol. 17 (10), 1607-15
- **PELAY-GIMENO, M. et al.** Structure-Based Design of Inhibitors of Protein-Protein Interactions: Mimicking Peptide Binding Epitopes. *Angew Chem Int Ed Engl.,* 2015, vol. 54 (31), 8896-927
- **PELLETIER J.N. ; CAMPBELL-VALOIS F.X. ; MICHNICK S.W.** Oligomerization domain-directed reassembly of active dihydrofolate reductase from rationally designed fragments. *Proc Natl Acad Sci U S A.,* 1998, vol. 95 (21), 12141-6
- **SOUCEK, L. et al.** Inhibition of Myc family proteins eradicates KRas-driven lung cancer in mice. *Genes. Dev.,* 2013, vol. 27 (5), 504-13
- **RAJ, M. et al.** Plucking the high hanging fruit: A systematic approach for targeting protein-protein interactions. *Bioorg. Med. Chem.,* 2013, vol. 21, 4051-4057
- **REMY, I. et al.** Detection of protein-protein interactions using a simple survival protein-fragment complementation assay based on the enzyme dihydrofolate reductase. *Nat Protoc.,* 2007, vol. 2 (9), 2120-5
- **ROBERTSON ; SPRING.** Using Peptidomimetics and Constrained Peptides as Valuable Tools for Inhibiting Protein-Protein Interactions. *Molecules,* 2018, vol. 23 (4), 959
- **RODRIGUEZ-MARTINEZ et al.** Combinatorial bZIP dimers display complex DNA-binding specificity landscapes. *Elife,* 2017, vol. 6, e19272
- **RUAN, F. et al.** Metal ion-enhanced helicity in synthetic peptides containing unnatural, metal-ligating residues. *J. Am. Chem. Soc.,* 1990, vol. 112 (25), 9403-9404
- **VAQUERIZAS, J.M. et al.** A census of human transcription factors: function, expression and evolution. *Nat Rev Genet.,* 2009, vol. 10 (4), 252-63
- **VINSON, C. et al.** Classification of human B-ZIP proteins based on dimerization properties. *Mol Cell Biol.,* 2002, vol. 22 (18), 6321-35
- **WALENSKY, L.D. et al.** Activation of apoptosis in vivo by a hydrocarbon-stapled BH3 helix. *Science,* 2004, vol. 305 (5689), 1466-70
- **WANG, D. et al.** Enhanced metabolic stability and protein-binding properties of artificial alpha helices derived from a hydrogen-bond surrogate: application to Bcl-xL. *Angew Chem Int Ed Engl.,* 2005, vol. 44 (40), 6525-9
- **WEHR, M.C. ; ROSSNER, M.J.** Split protein biosensor assays in molecular pharmacological studies. *Drug Discov Today.,* 2016, vol. 21 (3), 415-29
- **WEHR, M.C. et al.** Monitoring regulated protein-protein interactions using split TEV. *Nat Methods.,* 2006, vol. 3 (12), 985-93
- **WOOLLEY, G.A.** Photocontrolling peptide alpha helices. *Acc Chem Res,* 2005, vol. 38 (6), 486-93
- **YOUNG,K.H.** Yeast two-hybrid: so many interactions, (in) so little time... *Biol Reprod.,* 1998, vol. 58 (2), 302-11
- **SAMBROOK, J. ; RUSSEL, D.W.** Molecular Cloning, A Laboratory Manual. Cold Spring Harbor Laboratory Press, 2001